(19) Europäisches Patentamt / European Patent Office / Office européen des brevets

(11) EP 4 548 971 A1

(12) **EUROPEAN PATENT APPLICATION**

(43) Date of publication:
**07.05.2025 Bulletin 2025/19**

(21) Application number: **23208053.1**

(22) Date of filing: **06.11.2023**

(51) International Patent Classification (IPC):
***A61P 1/18*** *(2006.01)* ***A61P 9/04*** *(2006.01)*
***A61P 9/06*** *(2006.01)* ***A61P 25/00*** *(2006.01)*
***C07C 213/08*** *(2006.01)* ***C07C 217/20*** *(2006.01)*
***C07C 319/20*** *(2006.01)* ***C07C 323/25*** *(2006.01)*
***C07D 281/10*** *(2006.01)*

(52) Cooperative Patent Classification (CPC):
**C07D 281/10; A61P 1/18; A61P 9/04; A61P 9/06; A61P 25/00; C07C 213/08; C07C 217/20; C07C 319/20; C07C 323/25**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Georg-August-Universität Göttingen Stiftung Öffentlichen Rechts, Universitätsmedizin 37075 Göttingen (DE)**
- **Max-Planck-Gesellschaft zur Förderung der Wissenschaften e.V. 80539 München (DE)**

(72) Inventors:
- **Lehnart, Stephan 37073 Göttingen (DE)**
- **Wegener, Jörg 37085 Göttingen (DE)**
- **Mitronova, Gyuzel 37077 Göttingen (DE)**
- **Belov, Vladimir 37083 Göttingen (DE)**

(74) Representative: **Sonnenhauser, Thomas Martin Wuesthoff & Wuesthoff Patentanwälte und Rechtsanwalt PartG mbB Schweigerstraße 2 81541 München (DE)**

(54) **1,4-BENZOTHIAZEPINES AND RELATED COMPOUNDS WITH CYCLOPROPANOL GROUPS AS MULTI-TARGETED DRUGS**

(57) The present invention relates to multifunctional agents as defined in the claims based on *N*-substituted 1,4-benzothiazepines with cyclopropanol groups and their "open" analogues (such as the 3-[(4-methoxyphenyl)oxy]- and 3-[(4-methoxyphenyl)thio]propane-1-amine cyclopropanol derivatives; see formulae below) as well as the rearrangement products obtained from such cyclopropanol compounds, i.e. ethyl ketones, and the oxidation products from such cyclopropanol compounds, i.e. oxiranes.

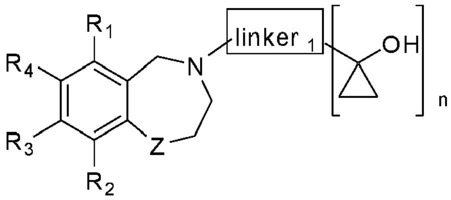

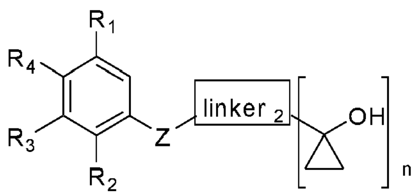

Such compounds can be used by preventing $Ca^{2+}$ leak through ryanodine receptor 2 (RyR2) and enhance cardiac sarco-endoplasmic reticulum (SR) $Ca^{2+}$ load by activation of $Ca^{2+}$-dependent ATPase 2a (SERCA2a) for example for the treatment of cardiac or neuronal diseases.

EP 4 548 971 A1

## Description

The present invention relates to multifunctional agents based on *N*-substituted 1,4-benzothiazepines with cyclopropanol groups and their "open" analogues (such as the 3-[(4-methoxyphenyl)oxy]- and 3-[(4-methoxyphenyl) thio]propane-1-amine cyclopropanol derivatives; see formulae below) as well as the rearrangement products obtained from such cyclopropanol compounds, i.e. ethyl ketones, and the oxidation products from such cyclopropanol compounds, i.e. oxiranes.

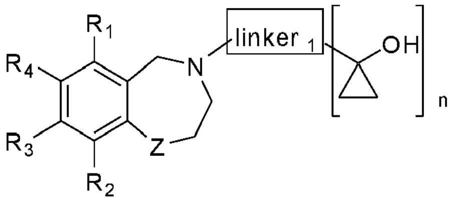
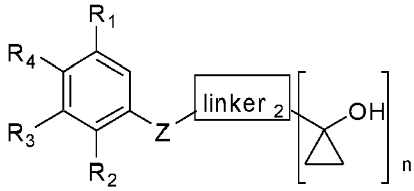

Such compounds can be used by preventing $Ca^{2+}$ leak through ryanodine receptor 2 (RyR2) and enhance cardiac sarco-endoplasmic reticulum (SR) $Ca^{2+}$ load by activation of $Ca^{2+}$-dependent ATPase 2a (SERCA2a) for example for the treatment of cardiac or neuronal diseases. For a comprehensive review of diseases associated with genetic and acquired ryanodine receptor diseases it is referred to A.R. Marks, Targeting ryanodine receptors to treat human diseases. J Clin Invest 2023, 133, e162891.

## Technical background

Ryanodine receptor type 2 (RyR2) and sarco/endoplasmic (SER) reticulum $Ca^{2+}$-ATPase 2a (SERCA2a) are the key intracellular players in intracellular cardiac $Ca^{2+}$ cycling. RyR2 is a $Ca^{2+}$ release channel and its main function is to initiate $Ca^{2+}$ release from the SER into the cytoplasm, which coordinates the contraction of the heart muscle by a process termed $Ca^{2+}$-induced $Ca^{2+}$ release [D. M. Bers, Cardiac excitation-contraction coupling. Nature 2002, 415, 198 - 205; S. O. Marx, A. R. Marks, Dysfunctional ryanodine receptors in the heart: new insights into complex cardiovascular diseases. J. Mol. Cell. Cardiol. 2013, 58, 225 - 231].

The SER $Ca^{2+}$-ATPase 2a (SERCA2a) is an energy-dependent $Ca^{2+}$-transport protein, and the major subtype of the cardiomyocyte SERCA transmembrane P-type ATPase, whose primary function is to pump $Ca^{2+}$ ions back into the SER $Ca^{2+}$ store after they are released by RyR2 $Ca^{2+}$ release channels during cardiac muscle contraction. The $Ca^{2+}$ ions are taken up into the SER mediating cardiac relaxation, until they are released in the next excitation-contraction cycle by RyR2 channel opening, which initiates the following heartbeat. In healthy conditions, SER $Ca^{2+}$ uptake as well as $Ca^{2+}$ release are strictly balanced to maintain physiological intracellular calcium concentrations $[Ca^{2+}]_i$ each in systole and diastole. However, abnormal regulation of the RyR2 channel both through significantly increased phosphorylation and oxidation combined with a reduced activity of the SERCA2a pump lead to i) impaired cardiac contractility contributing to heart failure and ii) cardiac rhythm disorders [S. O. Marx, A. R. Marks, Dysfunctional ryanodine receptors in the heart: new insights into complex cardiovascular diseases. J. Mol. Cell. Cardiol. 2013, 58, 225 - 231; D. M. Bers, Cardiac sarcoplasmic reticulum calcium leak: basis and roles in cardiac dysfunction. Annu. Rev. Physiol. 2014, 76, 107-127].

RyR2 channels in the healthy heart remain mostly closed during the relaxation phase of the cardiac cycle [D. M. Bers, Cardiac sarcoplasmic reticulum calcium leak: basis and roles in cardiac dysfunction. Annu. Rev. Physiol. 2014, 76, 107-127]. Under heart failure conditions, however, a significantly increased RyR2 opening activity promoted by its hyper-phosphorylation by protein kinase A (PKA) or calcium/calmodulin-dependent kinase II (CAMKII), as well as increased oxidation results in increased diastolic $Ca^{2+}$ leak from the SER [S. O. Marx, A. R. Marks, Dysfunctional ryanodine receptors in the heart: new insights into complex cardiovascular diseases. J. Mol. Cell. Cardiol. 2013, 58, 225 - 231; H. Uchinoumi, Y. Yang, T. Oda, N. Li, K. M. Alsina, J. L. Puglisi, Y. Chen-Izu, R. L. Cornea, X. H. T. Wehrens, D. M. Bers, CaMKII-dependent phosphorylation of RyR2 promotes targetable pathological RyR2 conformational shift. J. Mol. Cell. Cardiol. 2016, 98, 62 - 72]. Increased diastolic RyR2 $Ca^{2+}$ leak leads to $Ca^{2+}$ depletion of the SER $Ca^{2+}$ store, i) reducing cardiac contractility and ii) increasing sodium-calcium exchanger (NCX) 3 $Na^+$ in/1 $Ca^{2+}$ out electrogenic currents, promoting cardiomyocyte depolarization and a substrate for cardiac arrhythmias [X. H. T. Wehrens, S. E. Lehnart, A. R. Marks, Ryanodine Receptor-Targeted Anti-Arrhythmic Therapy. Ann. N. Y. Acad. Sci. 2005, 1047, 366 - 375]. Decreased SERCA2a activity, which may occur, for example, due to increased oxidative stress in heart failure leads to impaired $Ca^{2+}$ replenishment of the SER in cardiomyocytes, resulting in slowed diastolic relaxation and a decreased systolic $Ca^{2+}$ transient amplitude [J. Balderas-Villalobos, T. Molina-Muñoz, P. Mailloux-Salinas, G. Bravo, K. Carvajal, N. L. Gómez-Viquez, Oxidative stress in cardiomyocytes contributes to decreased SERCA2a activity in rats with metabolic syndrome. Am. J. Physiol. Heart Circ. 2013, 305, H1344 - H1353]. Hence, both SERCA2a and RyR2 dysfunctions can frequently occur in combination in heart

failure. These defects synergistically enhance the susceptibility for diastolic $Ca^{2+}$ overload and/or electrical re-activation of the cardiomyocyte and thus represent a central trigger mechanism for arrhythmias and contractile dysfunction [S. E. Lehnart, X. H. T. Wehrens, S. Reiken, S. Warrier, A. E. Belevych, R. D. Harvey, W. Richter, S. L. C. Jin, M. Conti, A. R. Marks, Phosphodiesterase 4D Deficiency in the Ryanodine-Receptor Complex Promotes Heart Failure and Arrhythmias. Cell 2005, 123, 25 - 35].

The enhancement of SER $Ca^{2+}$ load can be achieved by SERCA2a protein overexpression or by modification of signaling cascades that regulate SERCA2a at multiple levels, for example, via displacement or attenuation of the inhibitory effect of the small endogenous protein phospholamban (PLN) [W. J. Park, J. G. Oh, SERCA2a: a prime target for modulation of cardiac contractility during heart failure. BMB Rep. 2013, 46, 237 - 243; M. Jessup, B. Greenberg, D. Mancini, T. Cappola, D. F. Pauly, B. Jaski, A. Yaroshinsky, K. M. Zsebo, H. Dittrich, R. J. Hajjar, Calcium Upregulation by Percutaneous Administration of Gene Therapy in Cardiac Disease (CUPID). Circulation 2011, 124, 304 - 313; M. Kaneko, H. Yamamoto, H. Sakai, Y. Kamada, T. Tanaka, S. Fujiwara, S. Yamamoto, H. Takahagi, H. Igawa, S. Kasai, M. Noda, M. Inui, T. Nishimoto, A pyridone derivative activates SERCA2a by attenuating the inhibitory effect of phospholamban. Eur. J. Pharmacol. 2017, 814, 1 - 8]. The quinoline CDN1163 activates SERCA2 presumably through its ability to bind directly to SERCA2 and disrupt its structure [R. L. Cornea, S. J. Gruber, E. L. Lockamy, J. M. Muretta, D. Jin, J. Chen, R. Dahl, T. Bartfai, K. M. Zsebo, G. D. Gillispie, D. D. Thomas, High-Throughput FRET Assay Yields Allosteric SERCA Activators. J. Biomol. Screen. 2012, 18, 97 - 107]. There are reports about selective SERCA2a activators, derivatives of istaroxime metabolite, with unknown mechanism of action and on *N*-[(2-methoxyphenyl)methyl]-4,5-dimethyl-*N*-propyl-2-thiophenecarboxamide, which increases endoplasmic reticulum $Ca^{2+}$ load by enhancing SERCA2a-mediated $Ca^{2+}$ transport [A. Luraghi, M. Ferrandi, P. Barassi, M. Arici, S.-C. Hsu, E. Torre, C. Ronchi, A. Romerio, G.-J. Chang, P. Ferrari, G. Bianchi, A. Zaza, M. Rocchetti, F. Peri, Highly Selective SERCA2a Activators: Preclinical Development of a Congeneric Group of First-in-Class Drug Leads against Heart Failure. J. Med. Chem. 2022, 65, 7324 - 7333; R. Nikolaienko, E. Bovo, S. L. Yuen, L. M. Treinen, K. Berg, C. C. Aldrich, D. D. Thomas, R. L. Cornea, A. V. Zima, New N-aryl-N-alkyl-thiophene-2-carboxamide compound enhances intracellular Ca2+ dynamics by increasing SERCA2a Ca2+ pumping. Biophysical Journal 2023, 122, 386 - 396].

WO 2008/144483 mentions compounds for modulating ryanodine receptors RyRs.

Thus, conceptually there is a need to develop and identify novel small chemical agents that improve SERCA2a function and simultaneously allosterically inhibit RyR2 channel-mediated $Ca^{2+}$ leak from the SER, providing a novel causal multi-targeted approach to the treatment of relevant disorders, especially chronic cardiac disorders including arrhythmias and heart failure.

## Summary of invention

Accordingly, the present invention fulfils this need and provides chemically modified 1,4-benzothiazepines and their structural analogues, which, in addition to inhibition of RyR2 $Ca^{2+}$ leak, also enhance the activity of the SERCA2 $Ca^{2+}$ pump. The developed novel 1,4-benzothiazepines, as well as 3-[(4-methoxyphenyl)oxy]- and 3-[(4-methoxyphenylthio)] propane-1-amine derivatives contain a cyclopropanol fragment as a pharmacophore. Cyclopropanols and their derivatives have a variety of biological activities including enzyme inhibition, antibacterial and anticancer properties [R. Ueoka, M. Bortfeld-Miller, B. I. Morinaka, J. A. Vorholt, J. Piel, Toblerols: Cyclopropanol-Containing Polyketide Modulators of Antibiosis in Methylobacteria. Angew. Chem. Int. Ed. 2018, 57, 977 - 981; C. J. Suckling, The Cyclopropyl Group in Studies of Enzyme Mechanism and Inhibition. Angew. Chem. Int. Ed. 1988, 27, 537 - 552; A. Esposito, P. P. Piras, D. Ramazzotti, M. Taddei, First Stereocontrolled Synthesis of (S)-Cleonin and Related Cyclopropyl-Substituted Amino Acids. Org. Lett. 2001, 3, 3273 - 3275; X. Cai, F. He, J. Liang, B. Qu, W. Tao, J. Xu, L. Zhang, Y. Zhang, Ligand-drug conjugate of exatecan analogue, preparation method therefore and application thereof, WO2020063676A1, April 2, 2020**]**. However, cyclopropanol-containing compounds have never been featured as modulators of RyRs and/ or activators of SERCA. The rearrangement products obtained from cyclopropanol compounds, ethyl ketones (obtained in the presence of acids or strong bases [O. G. Kulinkovich, The Chemistry of Cyclopropanols. Chem. Rev. 2003, 103, 2597 - 2632; O. G. Kulinkovich, D. A. Astashko, V. I. Tyvorskii, N. A. Ilyina, Synthesis of $\alpha,\beta$-Epoxy Ketones from Alkyl- and Arylsubstituted Cyclopropanols. Synthesis 2001, 2001, 1453 - 1455]) have shown positive results confirming RyR2 modulation and SERCA2a activation. Based on the findings of the present inventors, the present inventors believe that the oxidation and rearrangement products of the specific cyclopropanols as described herein will have dual effect like the cyclopropanols compounds described herein for which this activity has been demonstrated herein.

Increased RyR2 $Ca^{2+}$ leak due to missense mutations is not only pathological in the heart but also in other organs. For example, RyR2 $Ca^{2+}$ leak in pancreatic beta-cells is associated with glucose intolerance [G. Santulli, G. Pagano, C. Sardu, W. Xie, S. Reiken, S. L. D'Ascia, M. Cannone, N. Marziliano, B. Trimarco, T. A. Guise, A. Lacampagne, A. R. Marks, Calcium release channel RyR2 regulates insulin release and glucose homeostasis. J Clin Invest. 2015, 125, 1968 - 1978]. Also, RyR2 $Ca^{2+}$ leak in the brain may participate in heart failure-induced cognitive dysfunction [H. Dridi, Y. Liu, S. Reiken, X. Liu, E. K. Argyrousi, Q. Yuan, M. C. Miotto, L. Sittenfeld, A. Meddar, R. K. Soni, O. Arancio, A.

Lacampagne, A. R. Marks, Heart failure-induced cognitive dysfunction is mediated by intracellular Ca2+ leak through ryanodine receptor type 2. Nat Neurosci. 2023, 26, 1365 - 1378.], cerebellar tremor pathophysiology [R. T. Martuscello, M. L. Chen, S. Reiken, L. R. Sittenfeld, D. S. Ruff, C. L. Ni, C. C. Lin, M. K. Pan, E. D. Louis, A. R. Marks, S. H. Kuo, P. L. Faust, Defective cerebellar ryanodine receptor type 1 and endoplasmic reticulum calcium 'leak' in tremor pathophysiology. Acta Neuropathol. 2023, 146, 301 - 318], cognitive dysfunction in Alzheimer's disease and posttraumatic disorder and Huntington's disease [A. R. Marks, Targeting ryanodine receptors to treat human diseases. J. Clin. Invest. 2023, 133, e162891] are associated with leaky RyR channels. Since the skeletal and cardiac RyR, i.e. RyR1 and RyR2, respectively show strong structural homologies in conserved functional domains, the present inventors expect that the compounds of the invention also affect skeletal RyR1 activities. Consequently, present inventors expect a beneficial effect on RyR1 mediated skeletal muscle diseases since RyR1 $Ca^{2+}$ release channel leak contributes in inherited myopathies including muscular dystrophies such as Duchenne to muscle weakness [A. M. Bellinger, S. Reiken, M. Dura, P. W. Murphy, S. X. Deng, D. W. Landry, D. Nieman, S. E. Lehnart, M. Samaru, A. LaCampagne, A. R. Marks, Remodeling of ryanodine receptor complex causes "leaky" channels: a molecular mechanism for decreased exercise capacity. Proc. Natl. Acad. Sci. USA. 2008,105, 2198 - 2202; A. R. Marks, Targeting ryanodine receptors to treat human diseases. J. Clin. Invest. 2023, 133, e162891), to age-related loss of muscle function and to cancer-associated muscle weakness (A. R. Marks, Targeting ryanodine receptors to treat human diseases. J. Clin. Invest. 2023, 133, e162891].

Since all cell types, generally electrically excitable cells, expressing RyR $Ca^{2+}$ release channels also express SERCA-type $Ca^{2+}$ pumps, the multi-targeted drug compounds described herein can be expected to target multiple organs and diseases e.g. RyR2 genetic disorders affecting both the heart and brain through stress-induced arrhythmias and generalized seizures, respectively [S. E. Lehnart, M. Mongillo, A. Bellinger, N. Lindegger, B. X. Chen, W. Hsueh, S. Reiken, A. Wronska, L. J. Drew, C. W. Ward, W. J. Lederer, R. S. Kass, G. Morley, A. R. Marks, Leaky Ca2+ release channel/ryanodine receptor 2 causes seizures and sudden cardiac death in mice. J. Clin. Investig. 2008, 118, 2230 - 2245], and pancreas function [G. Santulli, G. Pagano, C. Sardu, W. Xie, S. Reiken, S. L. D'Ascia, M. Cannone, N. Marziliano, B. Trimarco, T. A. Guise, A. Lacampagne, A. R. Marks, Calcium release channel RyR2 regulates insulin release and glucose homeostasis. J Clin Invest. 2015, 125, 1968 - 1978]. In analogy, heart failure and heart failure-induced cognitive dysfunction represent systemic diseases, where at least two organ systems are affected [H. Dridi, Y. Liu, S. Reiken, X. Liu, E. K. Argyrousi, Q. Yuan, M. C. Miotto, L. Sittenfeld, A. Meddar, R. K. Soni, O. Arancio, A. Lacampagne, A. R. Marks, Heart failure-induced cognitive dysfunction is mediated by intracellular Ca2+ leak through ryanodine receptor type 2. Nat Neurosci. 2023, 26, 1365 - 1378] and could be potentially treated with the drug compounds. Taken together, application of the multi-targeted drug compounds can be expected to be of benefit for many electrically excitable organ systems. Such compounds may be useful in the treatment of cardiac and skeletal muscle, brain and pancreas disorders, such as cardiac arrhythmia, e.g. atrial fibrillation [S. E. Lehnart, M. Mongillo, A. Bellinger, N. Lindegger, B. X. Chen, W. Hsueh, S. Reiken, A. Wronska, L. J. Drew, C. W. Ward, W. J. Lederer, R. S. Kass, G. Morley, A. R. Marks, Leaky Ca2+ release channel/ryanodine receptor 2 causes seizures and sudden cardiac death in mice. J. Clin. Investig. 2008, 118, 2230 - 2245], heart failure with increased adrenergic stress [M. Yano, S. Kobayashi, M. Kohno, M. Doi, T. Tokuhisa, S. Okuda, M. Suetsugu, T. Hisaoka, M. Obayashi, T. Ohkusa, M. Kohno, M. Matsuzaki, FKBP12.6-mediated stabilization of calcium-release channel (ryanodine receptor) as a novel therapeutic strategy against heart failure. Circulation 2003,107, 477 - 484], sudden cardiac death in the genetic RyR2 disorder stress-induced Catecholaminergic Polymorphic Ventricular Tachycardia patients [M. J. Wleklinski, P. J. Kannankeril, B. C. Knollmann, Molecular and tissue mechanisms of catecholaminergic polymorphic ventricular tachycardia. J. Physiol.-London 2020, 598, 2817 - 2834], and age-related cardiac dysfunction [M. Ruiz-Meana, M. Minguet, D. Bou-Teen, E. Miro-Casas, C. Castans, J. Castellano, E. Bonzon-Kulichenko, A. Igual, R. Rodriguez-Lecoq, J. Vazquez, D. Garcia-Dorado, Ryanodine Receptor Glycation Favors Mitochondrial Damage in the Senescent Heart. Circulation 2019, 139, 949 - 964] by improving maintenance of diastolic $Ca^{2+}$ levels.

The present invention is therefore directed to a compound represented by formula (1) or (2):

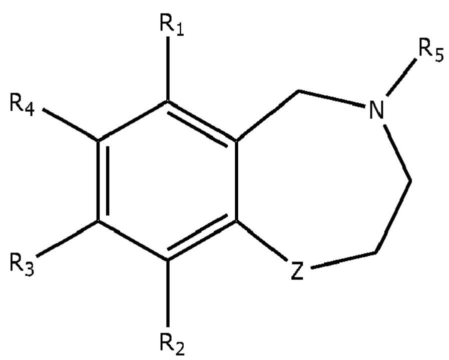

(1)

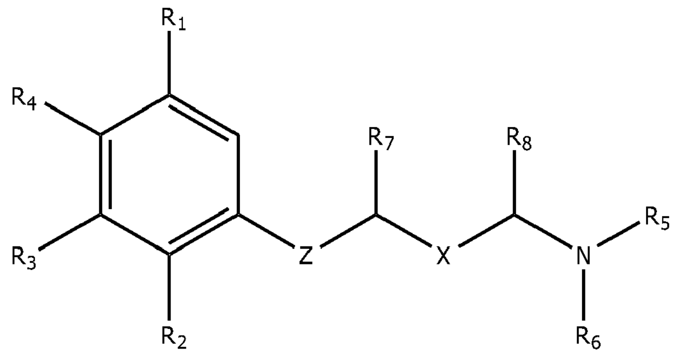

(2)

; wherein Z is O, or S;

$R_1$, $R_2$, $R_3$, and $R_4$ are independently selected from the group consisting of: H, OH, methoxy, ethoxy, methoxyalkyl, ethoxyalkyl, $CF_3$, and $CH_2CF_3$;

$R_5$ and optionally $R_6$ are independently selected from the groups represented by any of formulae (3) to (11):

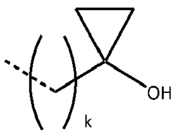 (3) 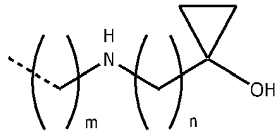 (4) 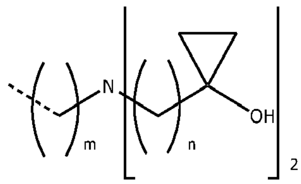 (5)

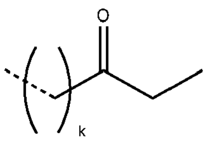 (6) 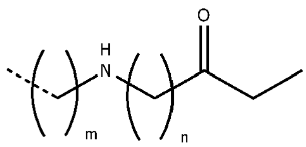 (7) 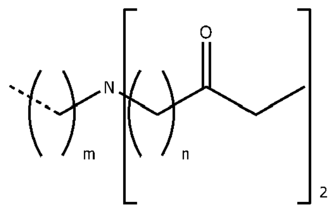 (8)

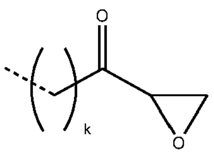 (9) 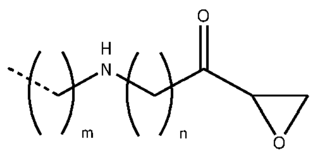 (10) 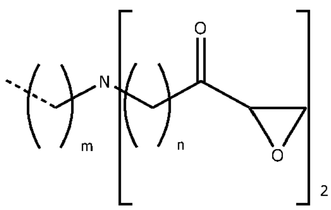 (11)

, wherein $R_6$ can be represented by H if it is not represented by any of formulae (3) to (11);

wherein the dashed line represents the bond to the nitrogen atom of formula (1) or (2); and wherein

$$2 \leq k \leq 16;$$

$$2 \leq m \leq 8;$$

$$2 \leq n \leq 8;$$

and $R_7$ and $R_8$ are independently selected from the group consisting of: H, substituted or non-substituted $C_{1-8}$-alkyl, and $C_{1-12}$-alkylcyclopropanol;

wherein X represents a single bond or is $-(CR_9R_{10})-$, wherein $R_9$ and $R_{10}$ are independently selected from the group consisting of: H, substituted or non-substituted $C_{1-8}$-alkyl, and $C_{1-12}$-alkylcyclopropanol.

The present invention is further directed to a compound as defined herein for use as a medicament.

The present invention is further directed to a compound as defined herein for use in the treatment of cardiac and skeletal muscle, brain and pancreas disorders, such as for the heart each cardiac arrhythmia including atrial fibrillation, heart failure with increased adrenergic stress, sudden cardiac death in the genetic RyR2 disorder stress-induced Catecholaminergic Polymorphic Ventricular Tachycardia patients, and age-related cardiac dysfunction by improving maintenance of resting cell $Ca^{2+}$ levels.

The present invention is further directed to a method of synthesizing a compound represented by formula (1):

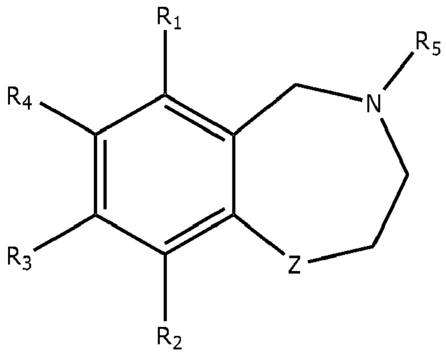

(1)

, or

a compound represented by formula (2):

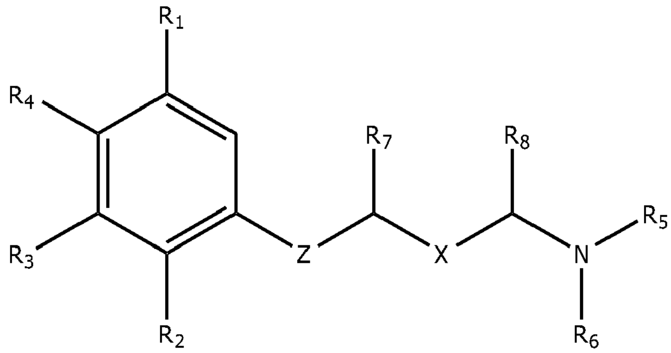

(2)

, wherein Z, X, $R_1$, $R_2$, $R_3$, $R_4$, $R_7$, and $R_8$ are as defined herein,
wherein $R_5$ and optionally $R_6$ are independently selected from the group represented by any of formulae (3) to (5):

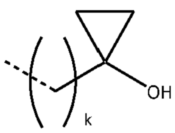

(3)

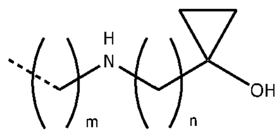

(4)

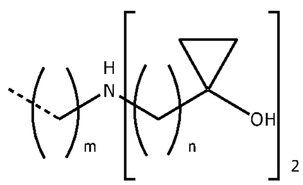

(5)

, wherein $R_6$ can be represented by H if it is not represented by any of formulae (3) to (5),
wherein k, m, n are as defined herein;
wherein the method comprises the steps of:

a) providing

a compound represented by formula (Ia) to synthesize the compound represented by formula (1) wherein $R_5$ is represented by formula (3), (4), or (5):

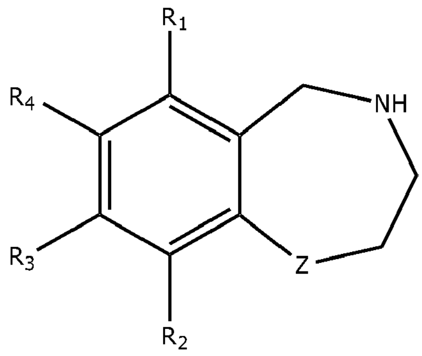

(Ia)

, or

a compound represented by formula (Ia’) to synthesize the compound represented by formula (1) wherein $R_5$ is independently selected from the group represented by any of formulae (4) or (5):

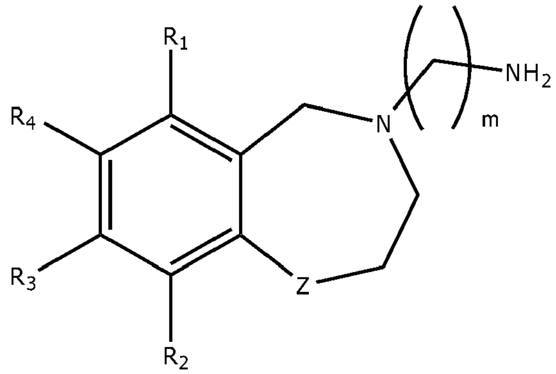

(Ia')

, or

a compound represented by formula (Ib) to synthesize the compound represented by formula (2):

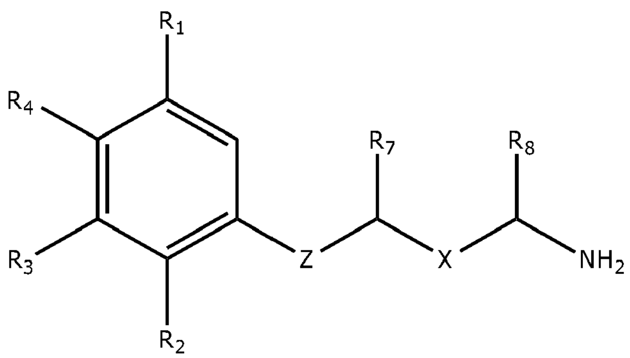

(Ib)

, wherein Z, X, $R_1$, $R_2$, $R_3$, $R_4$, $R_7$, and $R_8$ and m are as defined herein;

b) reacting the compound provided by step a) using

b1) reductive alkylation with a suitable reducing agent and a compound represented by formula (II), (III) or (IV) if the compound represented by formula (Ia) or if the compound represented by (Ib) is provided by step a):

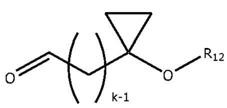 (II) 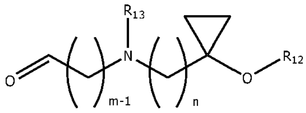 (III) 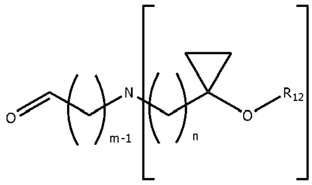 (IV)

, or

a compound represented by formula (II') if the compound represented by formula (Ia') is provided by step a):

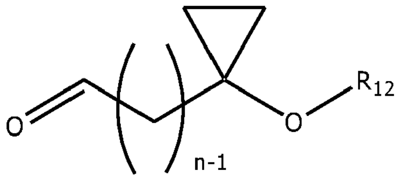 (II')

or

b2) direct alkylation with a suitable strong base and

a compound represented by formulae (V), (VI) or (VII) if the compound represented by formula (Ia) or if the compound represented by (Ib) is provided by step a):

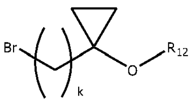 (V) 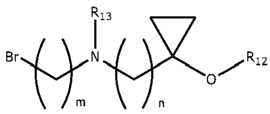 (VI) 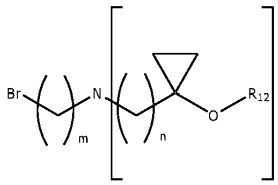 (VII)

, or

a compound represented by formula (V') if the compound represented by formula (Ia') is provided by step a):

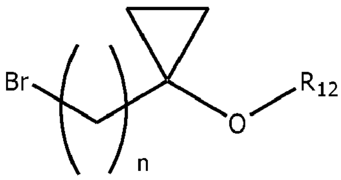 (V')

, wherein k, m, n are as defined herein,

wherein $R_{12}$ is a silyl ether or allyl ether protecting group,

wherein $R_{13}$ is an amine protecting group, preferably selected from the group consisting of: tert-butyloxycarbonyl, 9-fluorenylmethyloxycarbonyl, allyloxycarbonyl;

c) reacting the reaction product of step b) in a deprotection reaction with a suitable deprotection agent to form the cyclopropanol groups represented by any of formulae (3) to (5).

The present invention is further directed to a method of synthesizing a compound represented by formula (1):

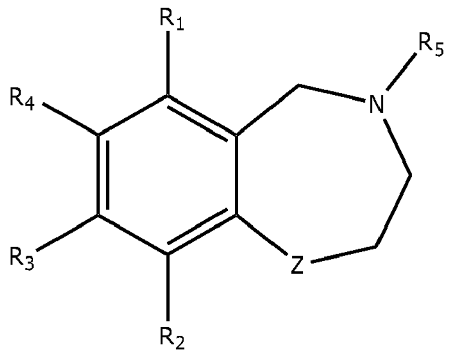

(1)

, or

a compound represented by formula (2):

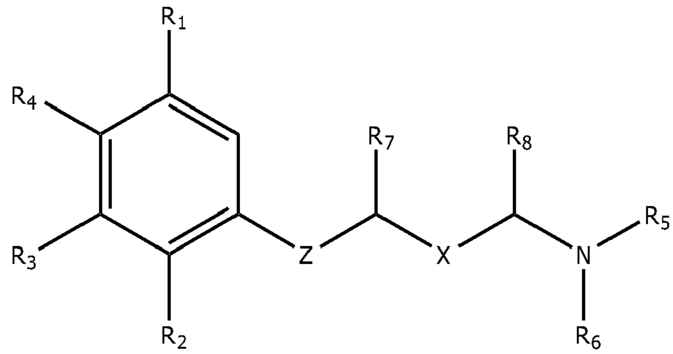

(2)

, wherein Z, X, $R_1$, $R_2$, $R_3$, $R_4$, $R_7$, and $R_8$ are as defined herein,
wherein $R_5$ and optionally $R_6$ are independently selected from the group represented by any of formulae (6) to (8):

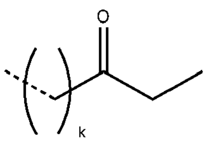

(6)

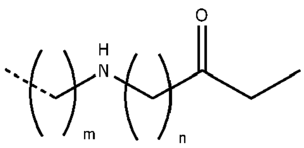

(7)

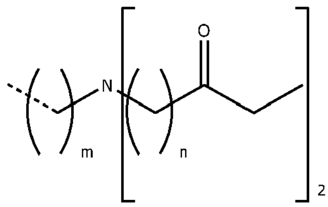

(8)

, wherein $R_6$ can be represented by H if it is not represented by any of formulae (6) to (8),
wherein k, m, and n are as defined herein;
wherein the method comprises the steps of:

a) providing

a compound represented by formula (1),
or
a compound represented by formula (2),
each prepared by the method according to any of claims 8 to 11;

b) reacting the compound provided in step a) under acidic conditions in a rearrangement reaction to form the groups represented by any of formulae (6) to (8).

The present invention is further directed to a method of synthesizing

a compound represented by formula (1):

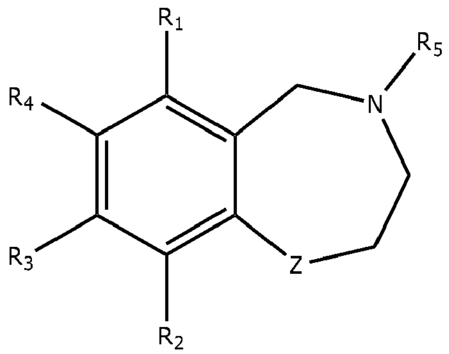

(1)

, or

a compound represented by formula (2):

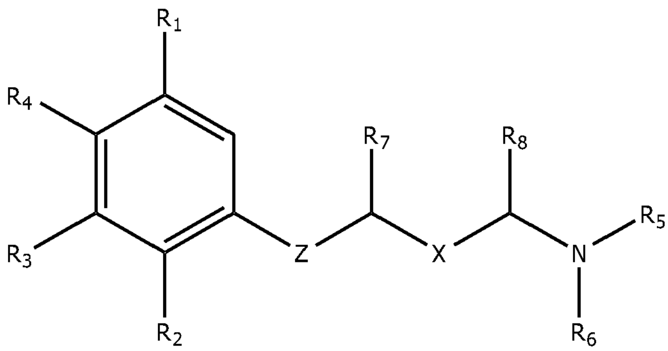

(2)

, wherein Z, X, $R_1$, $R_2$, $R_3$, $R_4$, $R_7$, and $R_8$ are as defined herein,
wherein $R_5$ and optionally $R_6$ are independently selected from the group represented by any of formulae (9) to (11):

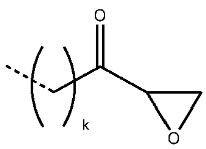 (9) 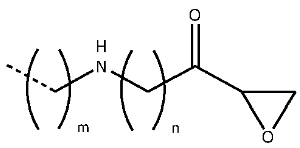 (10) 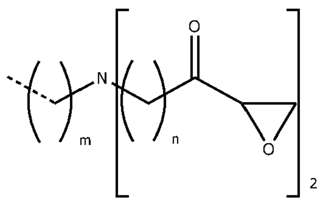 (11)

, wherein $R_6$ can be represented by H if it is not represented by any of formulae (9) to (11),
wherein k, m, n are as defined herein;
wherein the method comprises the steps of:

a) providing

a compound represented by formula (1),
or
a compound represented by formula (2),
each prepared by the method according to any of claims 8 to 11;

b) reacting the compound provided by step a) with a compound selected from the group consisting of $Mn(acac)_3$, Mn(II) abietate, and combinations thereof in an oxygen-containing atmosphere, followed by an addition of a base to form the groups represented by any of formulae (9), (10) or (11).

## Detailed description of the invention

The present invention refers to a compound represented by formula (1) or (2):

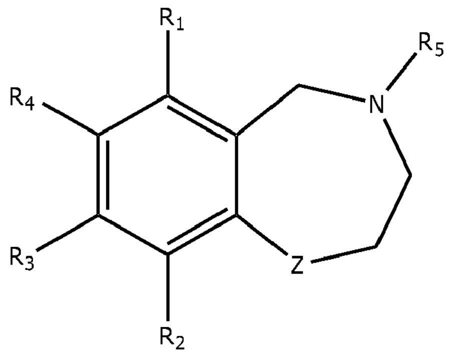

(1)

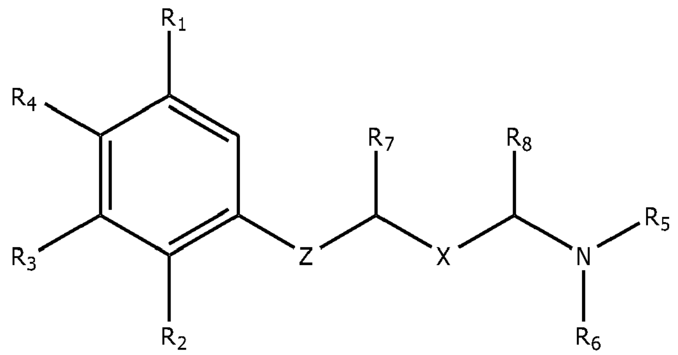

(2)

; wherein Z is O, or S;
$R_1$, $R_2$, $R_3$, and $R_4$ are independently selected from the group consisting of: H, OH, methoxy, ethoxy, methoxyalkyl, ethoxyalkyl, $CF_3$, and $CH_2CF_3$;
$R_5$ and optionally $R_6$ are independently selected from the group represented by any of formulae (3) to (11):

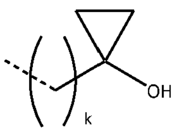

(3)

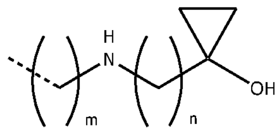

(4)

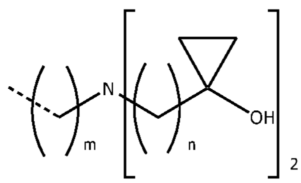

(5)

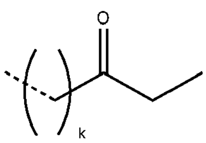

(6)

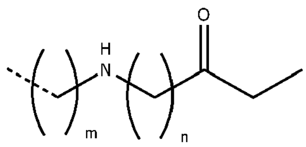

(7)

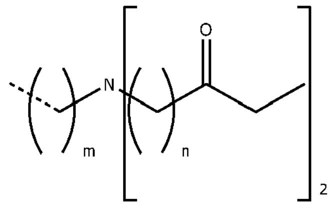

(8)

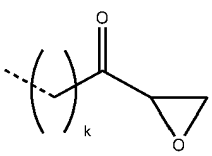

(9)

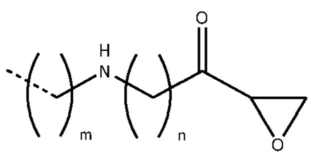

(10)

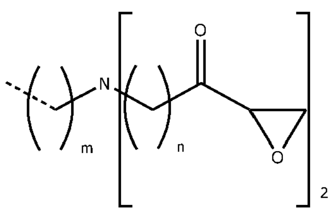

(11)

, wherein $R_6$ can be represented by H if it is not represented by any of formulae (3) to (11);
wherein the dashed line represents the bond to the nitrogen atom of formula (1) or (2); and wherein
$2 \leq k \leq 16$, preferably $4 \leq k \leq 8$, and for formula (2) only: preferably $k = 4$;
$2 \leq m \leq 8$, preferably $3 \leq m \leq 6$, more preferably $m = 3$ or $m = 4$;
$2 \leq n \leq 8$, preferably $4 \leq n \leq 6$, more preferably $n = 4$;

and $R_7$ and $R_8$ are independently selected from the group consisting of: H, substituted or non-substituted $C_{1-8}$-alkyl, and $C_{1-12}$-alkylcyclopropanol, preferably $R_7$ and $R_8$ are independently selected from the group consisting of: H, and $C_{1-12}$-alkylcyclopropanol, more preferably $R_7$ and $R_8$ are H;

wherein X represents a single bond or is $-(CR_9R_{10})-$, preferably X is $-(CR_9R_{10})-$, wherein $R_9$ and $R_{10}$ are independently selected from the group consisting of: H, substituted or non-substituted $C_{1-8}$-alkyl, and (non-substituted) $C_{1-12}$-alkylcyclopropanol, preferably $R_7$ and $R_8$ are independently selected from the group consisting of: H, and (non-substituted) $C_{1-12}$-alkylcyclopropanol, more preferably $R_7$ and $R_8$ are H.

The compounds represented by formula (1), are 1,4-benzothiazepines as well as 1,4-benzooxazepines. 1,4-benzoxazepines are reported to show 5-HT1A receptor agonists activity [K. Kamei, N. Maeda, R. Ogino, M. Koyama, M. Nakajima, T. Tasuoka, T. Ohno, T. Inoue, New 5-HT1A receptor agonists possessing 1,4-Benzoxazepine scaffold exhibit highly potent anti-ischemic effects. Bioorg. Med. Chem. Lett. 2001, 11, 595-598], acetylchloninesterase inhibitor activities [D. Szalóki Vargáné, L. Tóth, B. Buglyó, A. Kiss-Szikszai, A. Mándi, P. Mátyus, S. Antus, Y. Chen, D. Li, L. Tao, et al. [1,5]-Hydride Shift-Cyclization versus C(sp2)-H Functionalization in the Knoevenagel-Cyclization Domino Reactions of 1,4- and 1,5-Benzoxazepines. Molecules 2020, 25, 1265] and demonstrate anticancer profiles [A. K. Singh, V. Raj, A. Rai, A. K. Keshari, S. Saha Indole-fused benzooxazepines: a new structural class of anticancer agents. Future Sci OA. 2017;3(1):FSO168]. The differences between the heterocyclic benzothiazepine or benzoxazepine backbone was tested in experiments in which these compounds served as inhibitors of CBP/P300 bromodomains (CBP - cAMP responsive element binding protein, P300 - adenovirus E1A-associated 300 kDa protein, these are two evolutionary conserved and closely related histone acetyl transferases). The benzothiazepine compounds were found to be significantly less active than their benzoxazepine congeners [T. A. Popp, C. Tallant, C. Rogers, O. Fedorov, P. E. Brennan, S. Müller, S. Knapp, and F. Bracher. Development of Selective CBP/P300 Benzoxazepine Bromodomain Inhibitors. J. Med. Chem. 2016, 59, 8889 - 8912].

Formulae (4), (7), (10) and formulae (5), (8), (11) represent groups, which represent substituents $R_5$ and optionally $R_6$ of formula (1) or (2). Formulae (4), (7), (10) and formulae (5), (8), (11) bear an alkylaminoalkyl linker linking the cyclopropanol, the ethyl ketone or the oxirane functionality to the basic structures represented by formula (1) or (2), respectively. In that regard, formulae (4), (7), (10) represent mono-*N*-substituted products, whereas formulae (5), (8), (11) represent bi-N-substituted products. Such compounds can be prepared starting from respective primary amines in one single synthesis. This synthetic step can be advantageous for the production of drugs specifically tailored to individual needs, because these mono- and bi-products, according to the investigations of the inventors, have different biological properties towards RyR2 and SERCA2a (see Examples, particularly Table 1), and further, a second cyclopropanol group can directly influence the SERCA2a activity.

The length of the linker is defined by the indexes k (for the alkyl linker of formulae (3), (6), (9)) as well as m and n (for the alkylaminoalkyl linker). Varying the value for theses indexes can lead to drugs specifically tailored to individual needs. In case of the "open" analogues of the 1,4-benzothiazepines/1,4-benzooxazepines represented by formula (2), the linker with $m \geq 2$ is necessary for mimicking 1,4-benzotiazepine/1,4-benzooxazepine structures.

In one embodiment k is $\geq 4$ and $\leq 16$, particularly preferred is $k > 4$ and $\leq 16$ if the compounds are represented by formula (1), since these compounds have a shorter linker to the reactive group and higher "k"-values are beneficial.

In one embodiment $2 \leq k \leq 16$, preferably $4 \leq k \leq 8$, more preferably $k = 4$, if the compound represented by formula (2).

In case of a substitution with $R_7$ and $R_8$ being represented by a $C_{1-12}$-alkylcyclopropanol group, branched structures with more than two cyclopropanol groups may be resulting. Such structures are expected to show RyR2-SERCA2a dual activities.

The term "bond" or "single bond" refers to a chemical bond between two atoms, or two moieties when the atoms joined by the bond are considered to be part of a larger substructure.

The term "substituted" with respect to groups such as electron-donating or alkyl/alkene/alkyne groups as used herein refers to groups, wherein one or more hydrogen atoms are replaced by different atoms or groups such as halogens (e.g. Cl, Br, I, F), amines (primary, secondary, tertiary), sulphates, phosphates, ethers, esters, OH, SH, $CF_3$, CN, (unsubstituted) $C_{1-4}$ alkyl, $C_{1-4}$ heteroalkyl, $C_{6-14}$ aryl and $C_{4-12}$ heteroaryl groups etc.

The term "unsubstituted" with respect to groups such as electron-donating or alkyl/alkene/alkyne groups as used herein refers to groups, wherein no hydrogen is replaced by a different atom or group. That is, the term unsubstituted alkyls as used herein includes methyl, ethyl, propyl etc.

It is noted that not all hydrogen atoms are shown in the formulas throughout the application. Furthermore, any atom in the general formulas can be substituted by any of its isotopes. That is, any hydrogen atom can be substituted by deuterium D.

Furthermore, any carbon atom in the disclosed and/or claimed compounds can be substituted (partially or fully) by any of its isotopes, preferably $^{13}C$. Furthermore, any nitrogen atom in the disclosed and/or claimed compounds can be substituted (partially or fully) by $^{15}N$. Furthermore, any oxygen atom in the disclosed and/or claimed compounds can be

substituted (partially or fully) by $^{17}O$.

The term "alkyl" as used herein refers to straight chain/linear, or branched hydrocarbon substituents, preferably having 1 to 20, or 1 to 10 carbon atoms. Examples of alkyl groups include methyl, ethyl, propyl, butyl, isopropyl, sec-butyl, isobutyl, *tert*-butyl.

The term "aryl" refers to and includes both single-ring aromatic hydrocarbyl groups and polycyclic aromatic ring systems. The polycyclic rings may have two or more rings in which two carbons are common to two adjoining rings (the rings are "fused") wherein at least one of the rings is an aromatic hydrocarbyl group, e.g., the other rings can be cycloalkyls, cycloalkenyls, aryl, heterocycles, and/or heteroaryls. Preferred aryl groups are those containing six to thirty carbon atoms, preferably six to twenty carbon atoms, more preferably six to twelve carbon atoms. Especially preferred is an aryl group having six carbons, ten carbons or twelve carbons. Suitable aryl groups include phenyl, biphenyl, triphenyl, triphenylene, tetraphenylene, naphthalene, anthracene, phenalene, phenanthrene, fluorene, pyrene, chrysene, perylene, and azulene, preferably phenyl, biphenyl, triphenyl, triphenylene, fluorene, and naphthalene. Additionally, the aryl group is optionally substituted.

The term "methoxy", as used herein, refers to a group of formula -OMe or $-OCH_3$. The term "ethoxy", as used herein, refers to a group of formula -OEt or $-OCH_2CH_3$. As used herein, the term methoxyalkyl has the same definition as alkyl with the exception that one hydrogen atom in the hydrocarbon fragment is replaced by a methoxy group. As used herein, the term ethoxyalkyl has the same definition as alkyl with the exception that one hydrogen atom in the hydrocarbon fragment is replaced by a ethoxy group.

The term "aromatic group" as used herein refers to groups having aromaticity, namely having a conjugated ring of unsaturated bonds or ion pairs of electrons and satisfying the Hückel's rule which states that an aromatic system should have 4n + 2 n-electrons, where n is an integer $\geq 0$. Said aromatic group includes aryl and heteroaryl groups. The aromatic group may be substituted or unsubstituted. The aromatic group includes both single-ring aromatic hydrocarbyl groups and polycyclic aromatic ring systems. The polycyclic rings may have two or more rings in which two carbons are common to two adjoining rings (the rings are "fused") wherein at least one of the rings is an aromatic hydrocarbyl group, e.g., the other rings can be cycloalkyls, cycloalkenyls, aryl, heterocycles, and/or heteroaryls.

As used herein, the term "heteroalkyl" refers to an alkyl group as described herein in which one or more carbon atoms is replaced by a heteroatom. Suitable heteroatoms include oxygen, sulfur, nitrogen, phosphorus, and the like. Examples of heteroalkyl groups include, but are not limited to, alkoxy, amino, thioester, poly(ethylene glycol), and alkyl-substituted amino.

The term "heteroaryl" refers to and includes both single-ring aromatic groups and polycyclic aromatic ring systems that include at least one heteroatom. The heteroatoms include, but are not limited to O, S, N, P, B, Si, and Se. In many instances, O, S, or N are the preferred heteroatoms. Hetero-single ring aromatic systems are preferably single rings with 5 or 6 ring atoms, and the ring can have from one to six heteroatoms. The hetero-polycyclic ring systems can have two or more rings in which two atoms are common to two adjoining rings (the rings are "fused") wherein at least one of the rings is a heteroaryl, e.g., the other rings can be cycloalkyls, cycloalkenyls, aryl, heterocycles, and/or heteroaryls. The hetero-polycyclic aromatic ring systems can have from one to six heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and azaanalogs thereof. Additionally, the heteroaryl group is optionally substituted.

As used herein, the term "halogen" or "halo" refers to fluorine (fluoro), chlorine (chloro), bromine (bromo) or iodine (iodo), preferably fluorine, chlorine or bromine.

The term "linker", as used herein, refers to a chemical entity linking at least two other entities together. Typically, a linker uses covalent bonding. Typically, a linker is composed from a number of 1 to 10 atoms forming the backbone of the linker. The linker provides spacing between the two molecules or moieties such that they are able to function in their intended manner. For example, the linker is a spacer comprising at least one linear or branched structural unit comprising the formula $-(C(R'R''))_n-$ wherein R' and R" are, independently from each other, a hydrogen or a residue selected from the group consisting of, optionally substituted, alkyl, aryl, arylalkyl, and alkylaryl.

According to a preferred embodiment of the present invention, Z is S.

In one embodiment the compound is represented by formula (1) and Z is S.

In one embodiment the compound is represented by formula (2) and Z is O, or S.

According to one embodiment

a) Z is S in case the compound is represented by formula (1); and/or
b) Z is O, or S in case the compound is represented by formula (2).

According to one embodiment the compound is represented by formula (2) and Z is O.

According to one embodiment the compound is represented by formula (2) and Z is S.

According to a preferred embodiment of the present invention, $R_1$, $R_2$, and $R_3$ are H.

According to a preferred embodiment of the present invention, the compound is represented by formula (1) or (2):

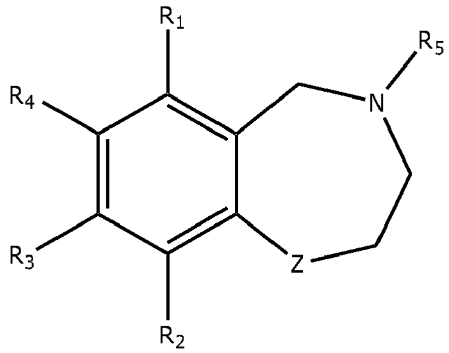

(1)

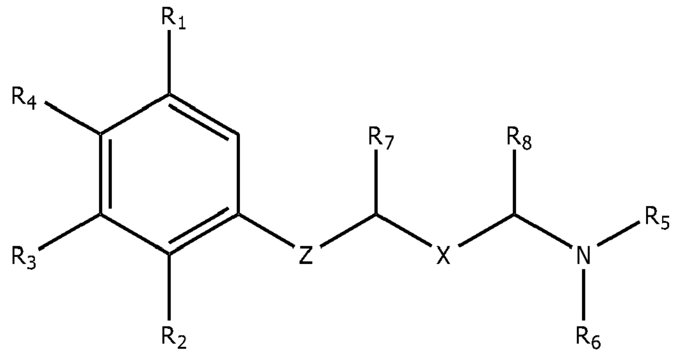

(2)

; wherein Z is O, or S;

$R_1$, $R_2$, and $R_3$ are independently selected from the group consisting of: H, OH, methoxy, ethoxy, methoxyalkyl, ethoxyalkyl, $CF_3$, and $CH_2CF_3$, preferably one of $R_1$, $R_2$, and $R_3$ is H, more preferably two of $R_1$, $R_2$, and $R_3$ are H, particularly preferably all of $R_1$, $R_2$, and $R_3$ are H;

$R_4$ is independently selected from the group consisting of: OH, methoxy, ethoxy, methoxyalkyl, ethoxyalkyl, $CF_3$, and $CH_2CF_3$;

$R_5$ and optionally $R_6$ are independently selected from the group represented by any of formulae (3) to (11):

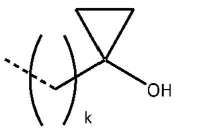

(3)

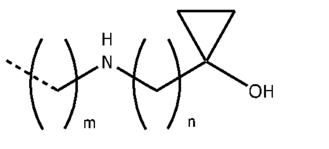

(4)

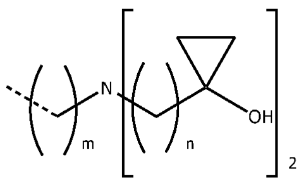

(5)

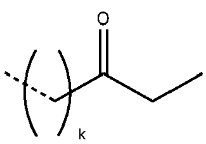

(6)

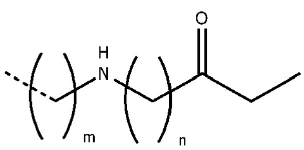

(7)

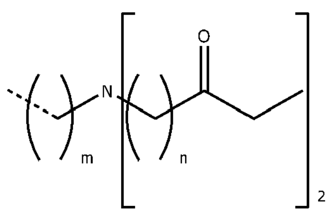

(8)

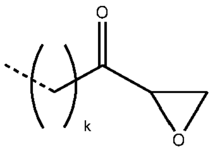 (9) 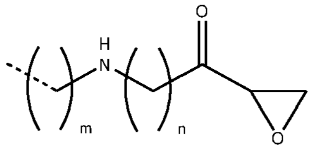 (10) 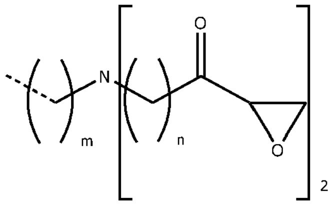 (11)

, wherein $R_6$ can be represented by H if it is not represented by any of formulae (3) to (11); wherein the dashed line represents the bond to the nitrogen atom of formula (1) or (2); and wherein

$$2 \leq k \leq 16;$$

$$2 \leq m \leq 8;$$

$$2 \leq n \leq 8;$$

and $R_7$ and $R_8$ are independently selected from the group consisting of: H, substituted or non-substituted $C_{1-8}$-alkyl, and $C_{1-12}$-alkylcyclopropanol;
wherein X represents a single bond or is $-(CR_9R_{10})-$, wherein $R_9$ and $R_{10}$ are independently selected from the group consisting of: H, substituted or non-substituted $C_{1-8}$-alkyl, and $C_{1-12}$-alkylcyclopropanol.

According to a preferred embodiment of the present invention, $R_4$ is $C_1$-$C_4$ alkoxy, such as methoxy.

It has been found that when $R_4$ is methoxy a better efficacy in hERG current experiments is shown than compounds in which $R_4$ is H [A. R. Marks, D. W. Landry, S. Deng, C. Z. Zhuang, S. E. Lehnart, Agents for preventing and treating disorders involving modulation of the RyR receptors. US7879840B2, February 1, 2011.]. Besides, changing from OMe to OH reduced the RyR2 stabilising activity of JTV-519 analogue (S.E. Lehnart, unpublished data).

In one embodiment $R_5$ and optionally $R_6$ is selected from the group represented by any of formulae (3) to (5).

In one embodiment $R_5$ is selected from the group represented by any of formulae (3) to (5) and $R_6$ is H.

In one embodiment the compound is represented by formula (1) and $R_5$ is selected from the group represented by any of formulae (3) to (5).

In one embodiment the compound is represented by formula (1) and $R_5$ is selected from the group represented by any of formulae (4), (5), (7), (8), (10) and (11). In one embodiment the compound is represented by formula (1) and $R_5$ is selected from the group represented by any of formulae (4) and (5).

In one embodiment the compound is represented by formula (2) and

$R_5$ is represented by formula (3),
$R_6$ is H,
Z is O, or S.

In one embodiment the compound is represented by formula (2) and

$R_5$ and $R_6$ are represented by formula (3),
Z is O, or S.

In one embodiment the compound is represented by formula (2), wherein $R_5$ and optionally $R_6$ is selected from the group represented by any of formulae (4), (5), (7), (8), (10) and (11), wherein $R_6$ can be represented by H if it is not represented by any of formulae (4), (5), (7), (8), (10) and (11). In one embodiment, $R_6$ is H and $R_5$ is selected from the group represented by any of formulae (4), (5), (7), (8), (10) and (11). In one embodiment, $R_6$ is H and $R_5$ is selected from the group represented by any of formulae (4) and (5).

In one embodiment $R_5$ and optionally $R_6$ are independently selected from the group represented by any of formulae (3), (4), (5), (9), (10), and (11);
further preferred $R_5$ and optionally $R_6$ are independently selected from the group represented by any of formulae (3), (4), and (5).

Even though $R_5$ and optionally $R_6$ are preferred to be represented by cyclopropanol and oxirane groups, particularly preferably cyclopropanol groups, it is expected that the ketone groups represented by formulae (6), (7), and (8)

will also act as multi-targeted drugs in accordance with the invention. Consequently, oral administration of these multi-targeted drugs would also be possible, since, for example, acidic pH values such as in the stomach would lead to ring opening of the cyclopropanols/oxiranes to the said ketones, which would then still be able to exert their effect.

The present invention also refers to a compound as defined herein for use as a medicament.

The RyR2 channel stabilizing activity of the compounds of the invention decrease the probability for single-channel opening and thus $Ca^{2+}$ flux through the channel pore. In electrically excitable cells RyR2 channels exists in groups which also known as clusters e.g. in cardiomyocytes. In this context the compounds of the invention are expected to inhibit diastolic $Ca^{2+}$ leak particularly in the context of a pathologic increase of leak e.g. in RyR2 channels containing mutations that cause catecholaminergic ventricular arrhythmias in patients [S. E. Lehnart, M. Mongillo, A. Bellinger, N. Lindegger, B. X. Chen, W. Hsueh, S. Reiken, A. Wronska, L. J. Drew, C. W. Ward, W. J. Lederer, R. S. Kass, G. Morley, A. R. Marks, Leaky Ca2+ release channel/ryanodine receptor 2 causes seizures and sudden cardiac death in mice. J. Clin. Investig. 2008, 118, 2230 - 2245.]. On the other hand, the simultaneous SERCA2a $Ca^{2+}$ pump activating property of the compounds of the invention will enhance SER $Ca^{2+}$ uptake while decreasing diastolic intracellular $Ca^{2+}$ levels. The latter might be particularly advantageous in the context of increased RyR2 $Ca^{2+}$ leak combined with decreased SER $Ca^{2+}$ uptake e.g. in cardiomyocytes from heart failure patients or animal models [P. A. Gorski, A. Lee, P. Lee, J. G. Oh, P. Vangheluwe, K. Ishikawa, R. Hajjar, C. Kho, Identification and Characterization of p300-Mediated Lysine Residues in Cardiac SERCA2a. Int. J. Mol. Sci. 2023, 24, 3502].

The term "medicament" is understood as meaning a substance or substance composition that is intended for the curing or prevention of pathologies in humans, is suitable for influencing physiological functions, or facilitates a medical diagnosis.

The present invention also refers to a compound as defined herein for use in the treatment of cardiac and skeletal muscle, brain and pancreas disorders, such as for the heart each cardiac arrhythmia including atrial fibrillation, heart failure with increased adrenergic stress, sudden cardiac death in the genetic RyR2 disorder stress-induced Catecholaminergic Polymorphic Ventricular Tachycardia patients, and age-related cardiac dysfunction by improving maintenance of resting cell $Ca^{2+}$ levels.

According to a preferred embodiment of the present invention, improving maintenance of diastolic resting cell $Ca^{2+}$ levels comprises

a) preventing pathologically increased either acutely, preferably stress-induced acutely, or chronic $Ca^{2+}$ leak through inhibiting ryanodine receptor type 2 (RyR2) channel activity,
and/or
b) enhancing cardiac sarco-endoplasmic reticulum $Ca^{2+}$ load by stimulating $Ca^{2+}$ uptake via SER $Ca^{2+}$-ATPase (SERCA), preferably in cardiomyocytes the SER $Ca^{2+}$-ATPase 2a isoform SERCA2a.

Further SERCA isoforms, e.g. SERCA1a in skeletal muscle, to treat muscle fatigue and/or muscular dystrophies are likely targets of the compounds as well, although untested in this context at present.

The synergistic effect of inhibiting pathologically increased RyR2 channel activity while stimulating SERCA2a activity in the heart/brain/pancreas will be more controlled (ideally physiologic) SER-dependent organelle intracellular $Ca^{2+}$ cycling while disease-causing events e.g. intracellular $Ca^{2+}$ waves triggering e.g. cardiac arrhythmias and cardiac contractile dysfunction are prevented.

In principle, all claimed compounds with RyR2 inhibitory and SERCA2a activatory activity would act with the same quality, however, their efficacy relative to the RyR2 or SERCA2a target or both are expected to differ to some extent. This could eventually, based on quantitative readouts of model systems and disease models, lead to personalized concepts of treatments.

The cardiac sarco-endoplasmic reticulum $Ca^{2+}$ load enhancing by stimulating $Ca^{2+}$ uptake via sarco/endoplasmic reticulum $Ca^{2+}$-ATPase (SERCA), preferably sarco/endoplasmic reticulum $Ca^{2+}$-ATPase 2a (SERCA2a). This means that the $Ca^{2+}$ pump activity is enhanced such that SER $Ca^{2+}$ uptake by the $Ca^{2+}$ storage organelle is increased while diastolic $Ca^{2+}$ levels in cardiomyocytes are decreased. On the other hand, an increased SER $Ca^{2+}$ load has been associated with increased cardiomyocyte and heart contractility (positive inotropy), a mechanism which might benefit patients with heart failure with reduced ejection fraction (HFrEF).

The present invention also refers to a method of synthesizing

a compound represented by formula (1):

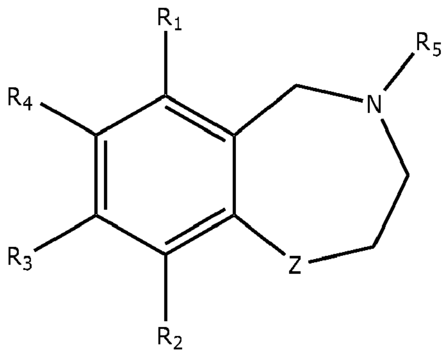

(1)

, or

a compound represented by formula (2):

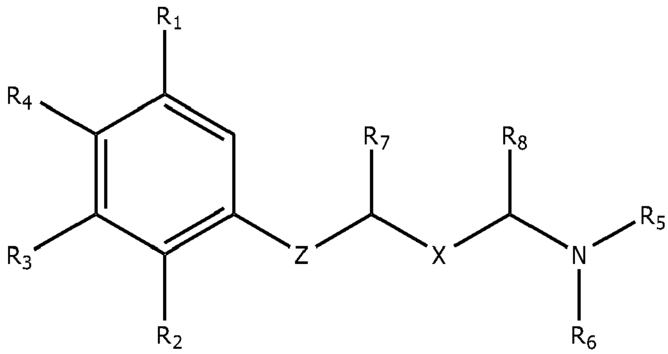

(2)

, wherein Z, X, $R_1$, $R_2$, $R_3$, $R_4$, $R_7$, and $R_8$ are as defined herein,

wherein $R_5$ and optionally $R_6$ are independently selected from the group represented by any of formulae (3) to (5):

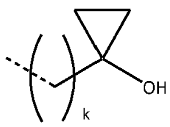

(3)

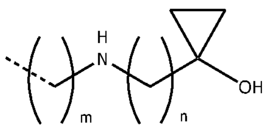

(4)

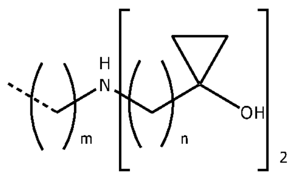

(5)

, wherein $R_6$ can be represented by H if it is not represented by any of formulae (3) to (5),

wherein k, m, n are as defined herein;

wherein the method comprises the steps of:

a) providing

a compound represented by formula (Ia) to synthesize the compound represented by formula (1) wherein $R_5$ is represented by formula (3), (4), or (5), preferably wherein $R_5$ is represented by formula (3), more preferably wherein $R_5$ is represented by formula (3) and $k \geq 5$:

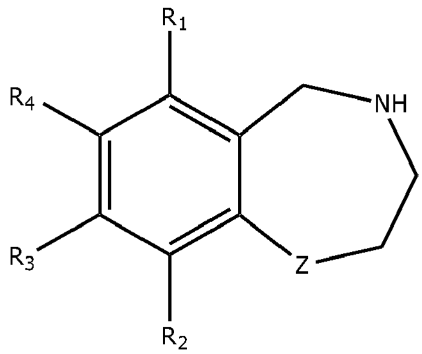

(Ia)

, or

a compound represented by formula (Ia') to synthesize the compound represented by formula (1) wherein $R_5$ is independently selected from the group represented by any of formulae (4) or (5):

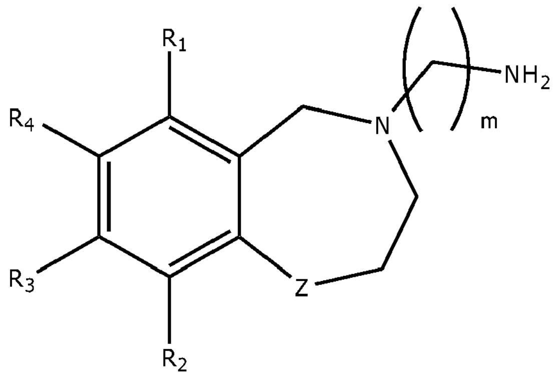

(Ia')

, or

a compound represented by formula (Ib) to synthesize the compound represented by formula (2):

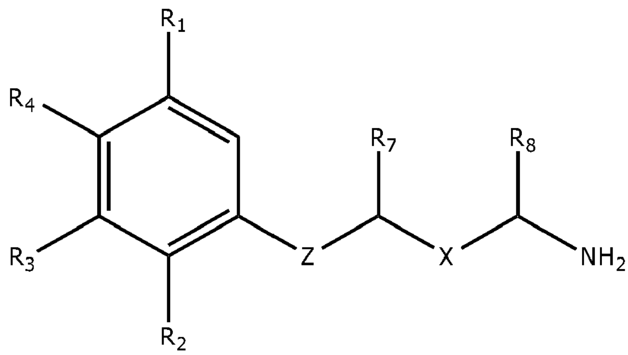

(Ib)

, wherein Z, X, $R_1$, $R_2$, $R_3$, $R_4$, $R_7$, and $R_8$ and m are as defined herein;

b) reacting the compound provided by step a) using

b1) reductive alkylation with a suitable reducing agent and

a compound represented by formula (II), (III) or (IV) if the compound represented by formula (Ia) or if the compound represented by (Ib) is provided by step a):

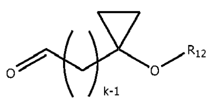 (II) 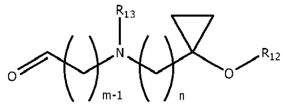 (III) 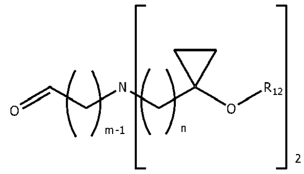 (IV)

, or

a compound represented by formula (II') if the compound represented by formula (Ia') is provided by step a):

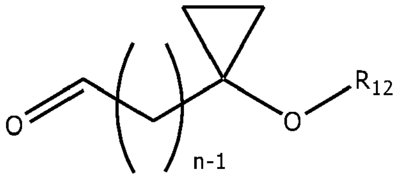 (II')

or

b2) direct alkylation with a suitable strong base and

a compound represented by formulae (V), (VI) or (VII) if the compound represented by formula (Ia) or if the compound represented by (Ib) is provided by step a):

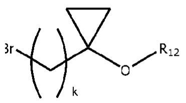 (V) 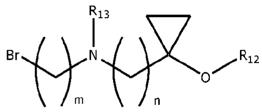 (VI) 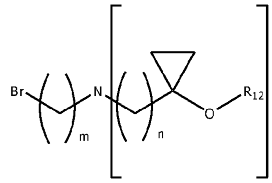 (VII)

, or

a compound represented by formula (V') if the compound represented by formula (Ia') is provided by step a):

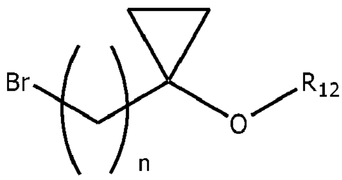 (V')

, wherein k, m, n are as defined herein,
wherein $R_{12}$ is a silyl ether or allyl ether protecting group,
wherein $R_{13}$ is an amine protecting group, preferably selected from the group consisting of: tert-butyloxycarbonyl, 9-fluorenylmethyloxycarbonyl, allyloxycarbonyl;

c) reacting the reaction product of step b) in a deprotection reaction with a suitable deprotection agent to form the cyclopropanol groups represented by any of formulae (3) to (5).

The compounds used in step b), i.e. the aldehydes represented by formula (II), (III) or (IV) and the bromides represented by formulae (V), (VI) or (VII), can be prepared using the Kulinkovich cyclopropanation followed by tert-butyldimethylsilyl (TBDMS) protection of the hydroxyl group [O. G. Kulinkovich, The Chemistry of Cyclopropanols. Chem. Rev. 2003, 103, 2597 - 2632; O. G. Kulinkovich, D. A. Astashko, V. I. Tyvorskii, N. A. Ilyina, Synthesis of $\alpha,\beta$-Epoxy Ketones from Alkyl- and Arylsubstituted Cyclopropanols. Synthesis 2001, 2001, 1453 - 1455].

The term "reductive alkylation" refers to a reaction in which a carbonyl group and an amino group are converted to a higher substituted amine by an intermediate imine in the presence of a reducing agent.

The term "reducing agent" refers to compound that donates an electron to another species. Common reducing

agents include metals potassium, calcium, barium, sodium and magnesium, and also compounds that contain the H- ion, those being NaH, LiH, $LiAlH_4$, $NaBH_4$ and CaHz. Borohydrides are preferred reducing agents.

The term "direct alkylation" as employed herein refers to a process in which an alkyl group is added to a compound. More specifically, it refers to a chemical reaction between an alkyl halide, preferably a bromide, and an amine. The reaction product is a higher substituted amine.

The term "protecting group" as used herein represents a group intended to protect a hydroxy or an amino group from participating in one or more undesirable reactions during chemical synthesis. More specifically, it refers to a moiety covalently attached to a functional group (particularly the amino group or the hydroxyl group) that can be selectively attached to the functional group and selectively removed without affecting the integrity of the molecule the protecting group is attached to.

The term "deprotection reaction" as used herein and hereafter refers to a reaction, wherein a protecting group is removed from a compound.

The term "deprotection agent" in the context of the present specification relates to an agent which is able to cleave a certain protecting group. The skilled person is able to select the deprotection agent according to the protecting group. The conditions under which the protecting group is cleavable constitute the deprotection agent, e.g. if the protecting group is cleavable under acidic conditions, then the deprotection agent is an acid.

In one embodiment, the method the compound represented by formula (II), (III) or (IV) is provided with at least a 2-fold molar excess respect to the compound provided by step a).

In one embodiment the method the compound represented by formula (V), (VI) or (VII) is provided with at least a 2-fold molar excess respect to the compound provided by step a).

In one embodiment the method of synthesizing

a compound represented by formula (1):

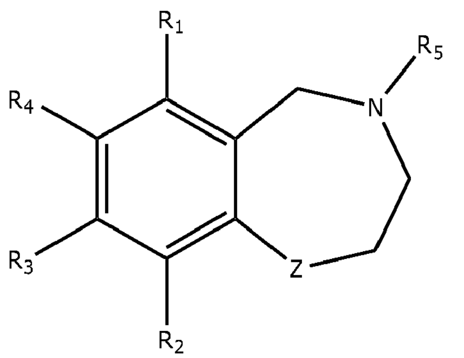

(1)

, or

a compound represented by formula (2):

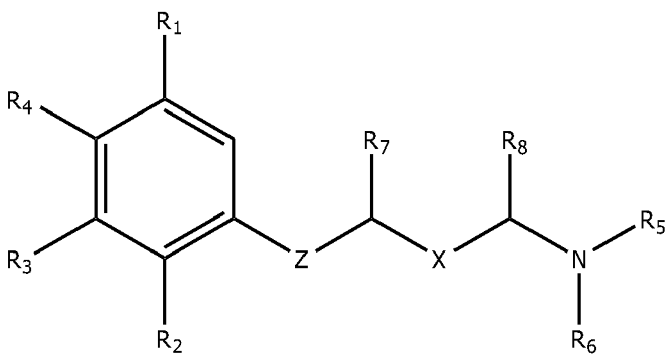

(2)

, wherein Z, X, $R_1$, $R_2$, $R_3$, $R_4$, $R_7$, and $R_8$, $R_9$ are as defined herein,

wherein $R_5$ and optionally $R_6$ are independently selected from the group represented by any of formulae (3) to (5):

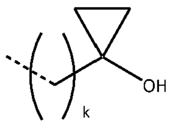 (3) 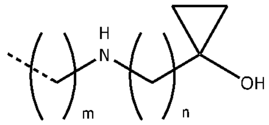 (4) 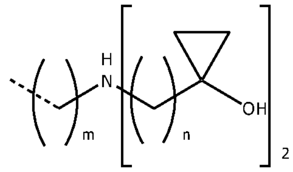 (5)

, wherein $R_6$ can be represented by H if it is not represented by any of formulae (3) to (5), wherein k, m, n are as defined herein;
comprises the steps of:

a) providing

a compound represented by formula (Ia) to synthesize the compound represented by formula (1):

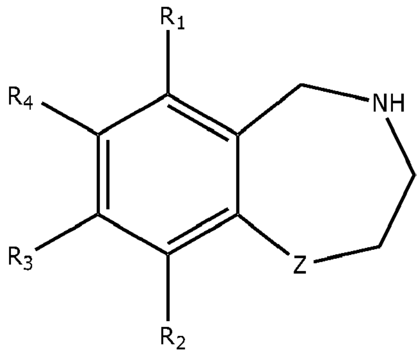

(Ia)

, or
a compound represented by formula (Ib) to synthesize the compound represented by formula (2):

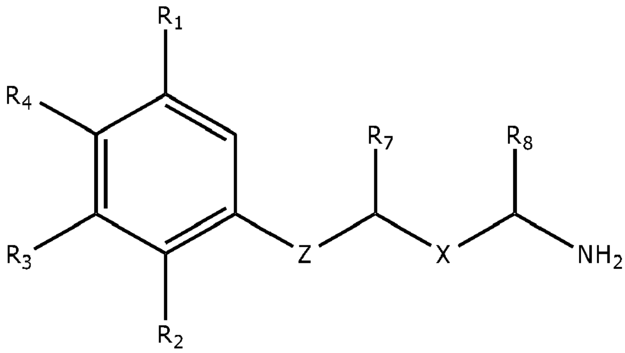

(Ib)

, wherein Z, X, $R_1$, $R_2$, $R_3$, $R_4$, $R_7$, and $R_8$ are as defined herein;

b) reacting the compound provided by step a) using

b1) reductive alkylation with a suitable reducing agent and a compound represented by formula (II), (III) or (IV):

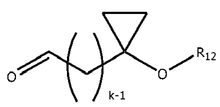 (II) 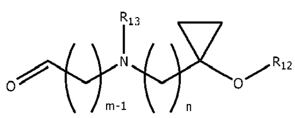 (III) 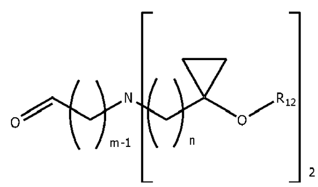 (IV)

or
b2) direct alkylation with a suitable strong base and a compound represented by formulae (V), (VI) or

(VII):

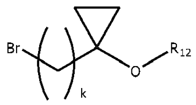 (V) 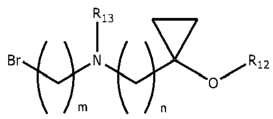 (VI) 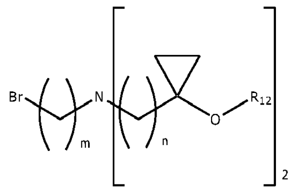 (VII)

, wherein k, m, n are as defined herein,
wherein $R_{12}$ is a silyl ether or allyl ether protecting group,
wherein $R_{13}$ is an amine protecting group, preferably selected from the group consisting of: tert-butyloxycarbonyl, 9-fluorenylmethyloxycarbonyl, allyloxycarbonyl;

c) reacting the reaction product of step b) in a deprotection reaction with a suitable deprotection agent to form the cyclopropanol groups represented by any of formulae (3), (4) or (5).

According to a preferred embodiment of the present invention, the reducing agent comprises borohydrides, preferably wherein the reducing agent comprises a borohydride selected from the group consisting of: sodium cyanoborohydride, triacetoxyborohydride, 2-picolynyl borane, and combinations thereof, more preferably wherein the reducing agent comprises sodium triacetoxyborohydride.

According to a preferred embodiment of the present invention, the deprotection agent for

a) the silyl ether protecting group comprises a fluoride containing compound, preferably wherein the fluoride containing compound is selected from the group consisting of: HF, KF, n-$Bu_4NF$, and combinations thereof;
b) the allyl ether protecting group comprises a Pd (0) reagent in combination with a reducing agent.

According to a particularly preferred embodiment of the present invention, the deprotection agent for the allyl ether protecting group comprises palladium on carbon and/or tetrakis(triphenylphosphine)palladium(0) ($Pd(PPh_3)_4$) in the presence of formic acid-organic base pare.

According to a preferred embodiment of the present invention, the silyl ether protecting group is selected from the group consisting of: trimethylsilyl, triethylsilyl, tert-butyldimethylsilyl, tert-butyldiphenylsilyl, triisopropylsilyl; preferably wherein the silyl ether protecting group is tert-butyldimethylsilyl.

The present invention also refers to the use of a compound represented by formula (Ia):

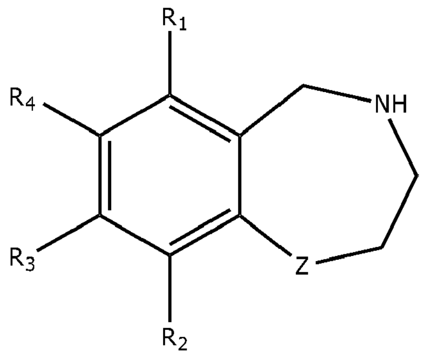

(Ia)

to synthesize a compound represented by formula (1):

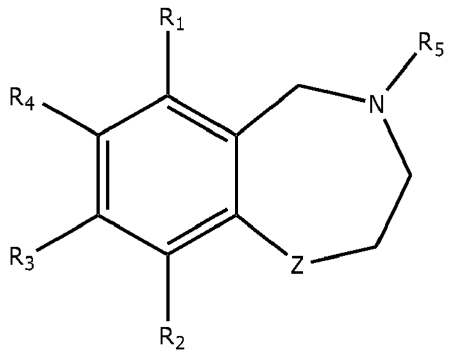

(1)

; wherein Z, $R_1$, $R_2$, $R_3$, $R_4$, are as defined herein,
wherein $R_5$ is represented by formula (3), (4), or (5), preferably wherein $R_5$ is represented by formula (3), more preferably wherein $R_5$ is represented by formula (3) and $k \geq 5$:

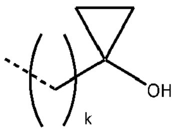 (3) 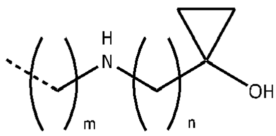 (4) 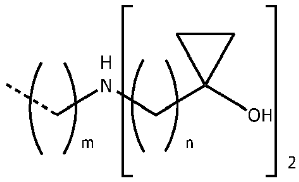 (5)

wherein k, m, and n is as defined herein.

The present invention also refers to the use of a compound represented by formula (Ia'):

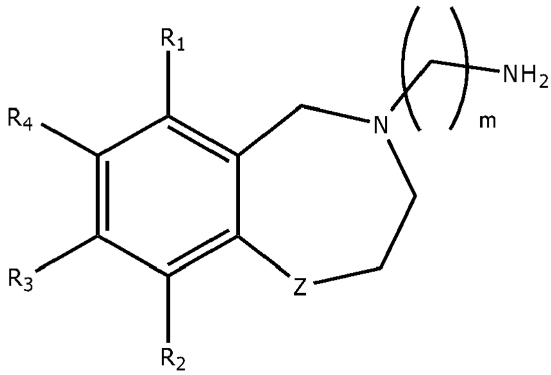

(Ia')

to synthesize a compound represented by formula (1):

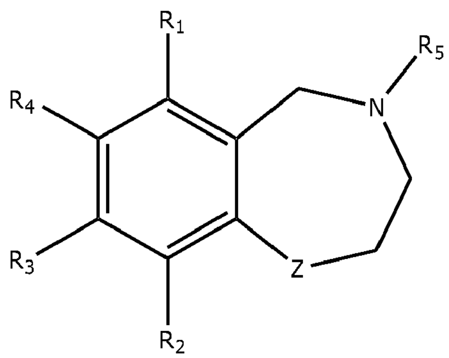

(1)

; wherein Z, $R_1$, $R_2$, $R_3$, $R_4$, are as defined herein,
wherein $R_5$ is independently selected from the group represented by any of formulae (4) or (5):

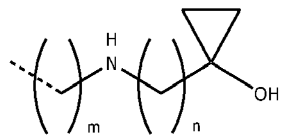

(4)

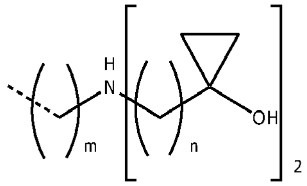

(5)

wherein m and n are as defined herein.

The present invention also refers to the use of a compound represented by formula (Ib):

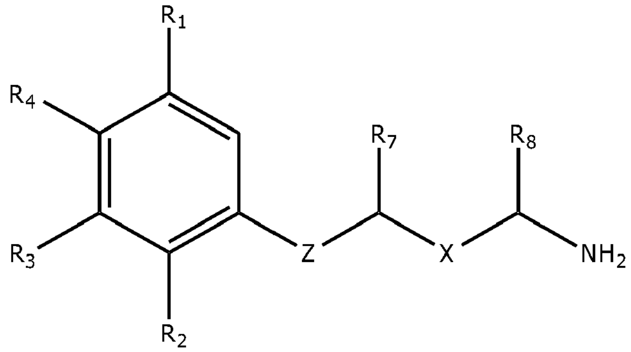

(Ib)

to synthesize a compound represented by formula (2):

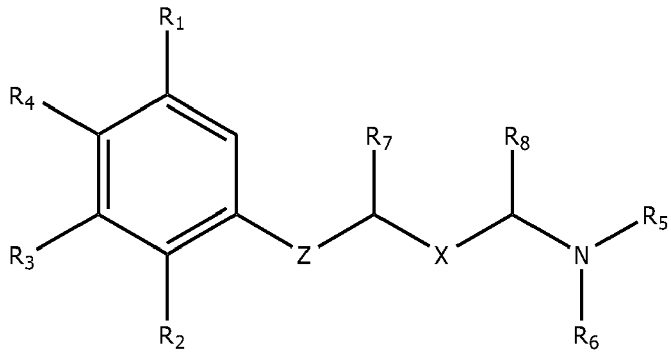

(2)

, wherein Z, X, $R_1$, $R_2$, $R_3$, $R_4$, $R_7$, and $R_8$ are as defined herein,
wherein $R_5$ and optionally $R_6$ are independently selected from the group represented by any of formulae (3) to (5):

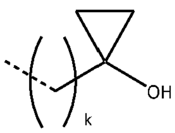

(3)

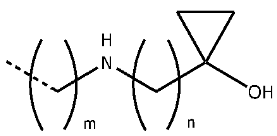

(4)

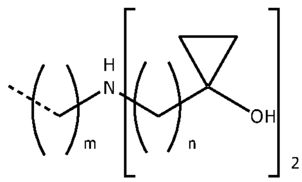

(5)

, wherein $R_6$ can be represented by H if it is not represented by any of formulae (3) to (5),
wherein k, m, n are as defined herein.

The present invention also refers to the use of a compound represented by formula (Ia):

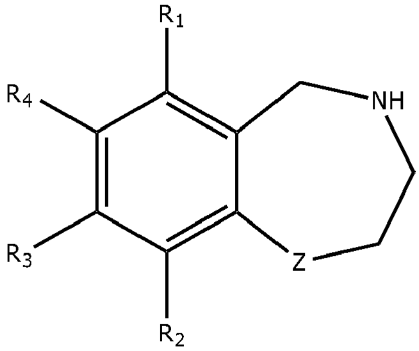

(Ia)

to synthesize a compound represented by formula (1):

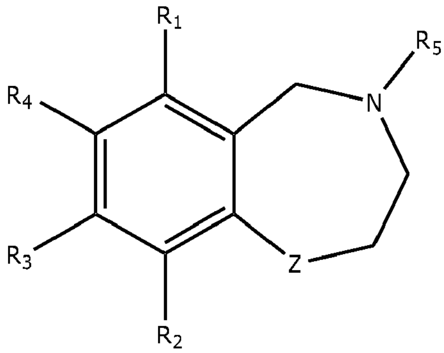

(1)

; wherein Z, $R_1$, $R_2$, $R_3$, $R_4$, are as defined herein,
wherein $R_5$ is independently selected from the group represented by any of formulae (3) to (5):

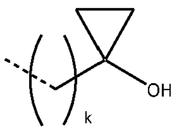

(3)

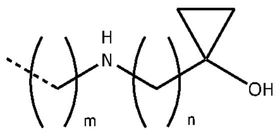

(4)

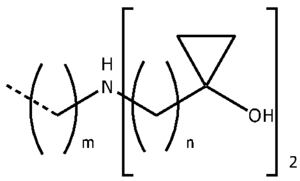

(5)

wherein k, m, n are as defined herein.

The present invention also refers to a method of synthesizing a compound represented by formula (1):

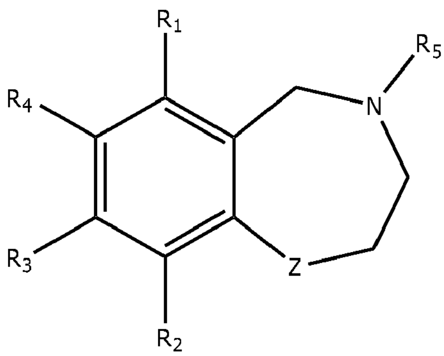

(1)

, or
a compound represented by formula (2):

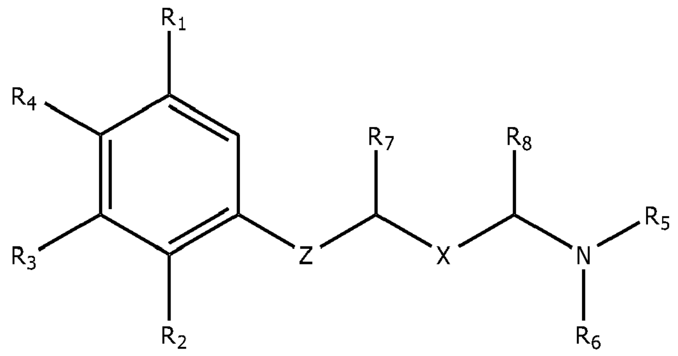

(2)

, wherein Z, X, $R_1$, $R_2$, $R_3$, $R_4$, $R_7$, and $R_8$ are as defined herein,
wherein $R_5$ and optionally $R_6$ are independently selected from the group represented by any of formulae (6) to (8):

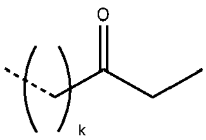 (6) 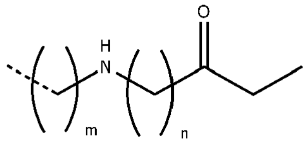 (7) 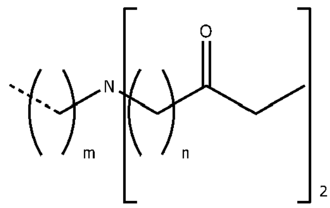 (8)

, wherein $R_6$ can be represented by H if it is not represented by any of formulae (6) to (8),
wherein k, m, and n are as defined herein;
wherein the method comprises the steps of:

a) providing

a compound represented by formula (1),
or
a compound represented by formula (2),
each prepared by the method according to any of claims 8 to 11;

b) reacting the compound provided in step a) under acidic conditions in a rearrangement reaction to form the groups represented by any of formulae (6) to (8).

The term "acidic conditions" as used herein, means a pH less than 2.0, and more specifically, conditions that enable a cyclopropanol ring opening rearrangement reaction, e.g., a pH of less than 2.0, less than 1.5, less than 1.0.

The term "rearrangement reaction" as used herein is the reaction where the carbon skeleton of a molecule is rearranged to give a structural isomer of the original molecule. This includes a cyclopropanol ring opening.

According to a preferred embodiment of the present invention, step b) is performed at a pH < 2.

In one embodiment step b) is performed using HF to create the acidic conditions in the rearrangement reaction.

In one embodiment step b) is performed using 0.1 % trifluoroacetic acid to create the acidic conditions in the rearrangement reaction.

The present invention also refers to the use of a compound represented by formula (1):

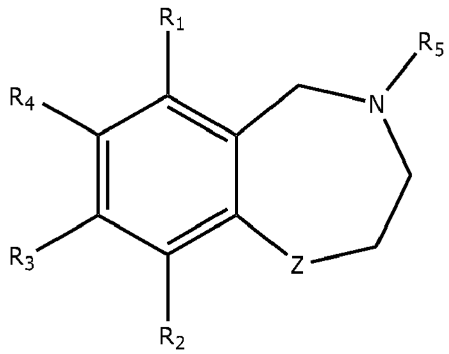

(1)

; wherein Z, $R_1$, $R_2$, $R_3$, $R_4$, are as defined herein,
wherein $R_5$ is independently selected from the group represented by any of formulae (3) to (5):

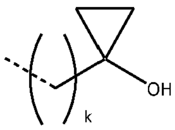

(3)

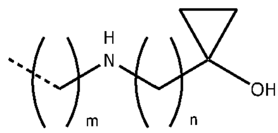

(4)

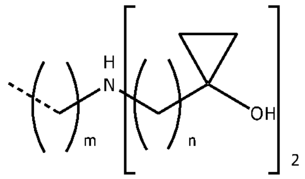

(5)

wherein k, m, n are as defined herein;
to synthesize a compound represented by formula (1'):

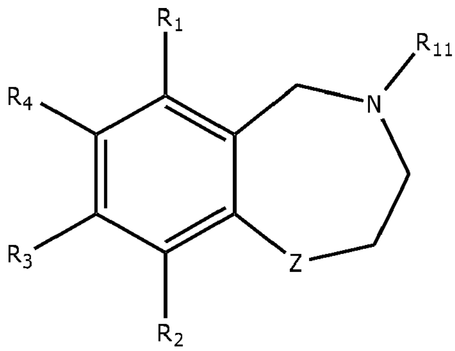

(1')

; wherein Z, $R_1$, $R_2$, $R_3$, $R_4$, are as defined herein,
wherein $R_{11}$ is independently selected from the group represented by any of formulae (6) to (8):

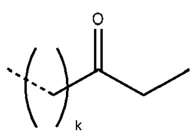

(6)

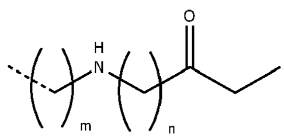

(7)

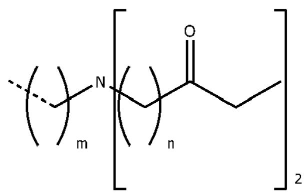

(8)

wherein k, m, n are as defined herein.

The present invention also refers to the use of a compound represented by formula (2):

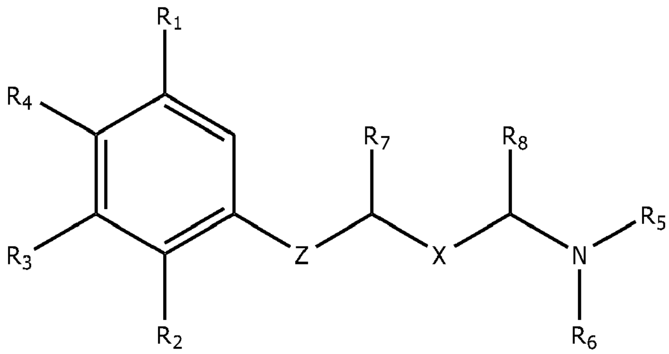

(2)

, wherein Z, X, $R_1$, $R_2$, $R_3$, $R_4$, $R_7$, and $R_8$ are as defined herein,
wherein $R_5$ and optionally $R_6$ are is independently selected from the group represented by any of formulae (3) to (5):

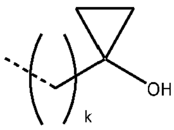 (3) 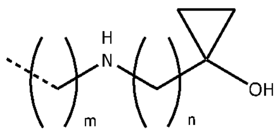 (4) 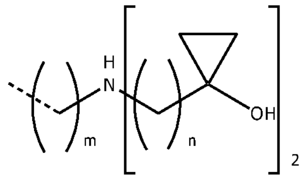 (5)

, wherein $R_6$ can be represented by H if it is not represented by any of formulae (3) to (5),
wherein k, m, n are as defined herein;
to synthesize a compound represented by formula (2'):

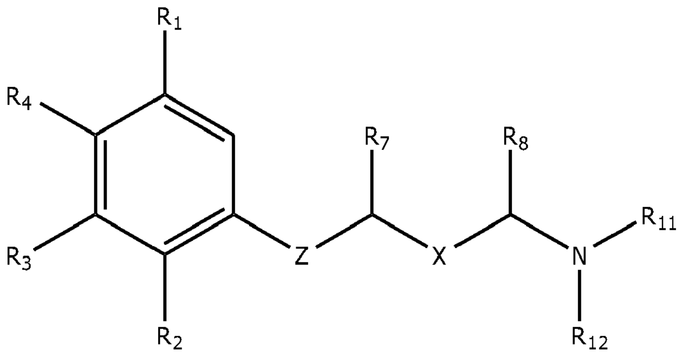

(2')

, wherein Z, X, $R_1$, $R_2$, $R_3$, $R_4$, $R_7$, and $R_8$ are as defined herein,
wherein $R_{11}$ and optionally $R_{12}$ are independently selected from the group represented by any of formulae (6) to (8):

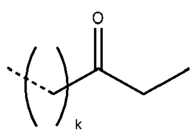 (6) 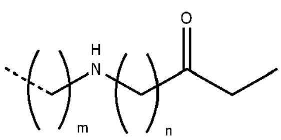 (7) 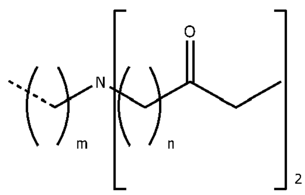 (8)

, wherein $R_{12}$ can be represented by H if it is not represented by any of formulae (6) to (8);
wherein k, m, n are as defined herein.

The present invention also refers to a method of synthesizing

a compound represented by formula (1):

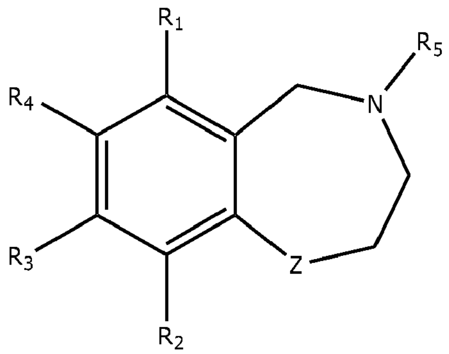

(1)

, or

a compound represented by formula (2):

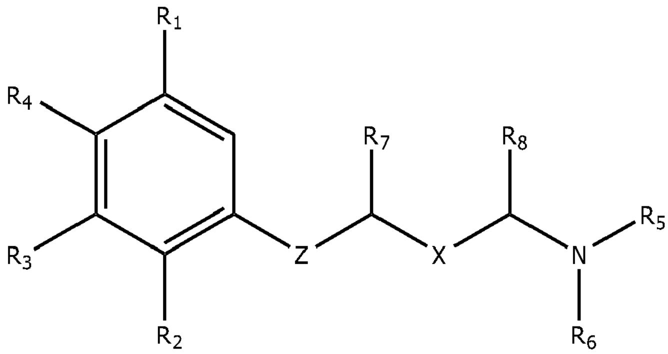

(2)

, wherein Z, X, $R_1$, $R_2$, $R_3$, $R_4$, $R_7$, and $R_8$ are as defined herein,

wherein $R_5$ and optionally $R_6$ are independently selected from the group represented by any of formulae (9) to (11):

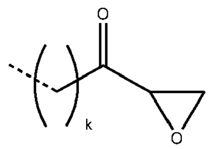 (9) 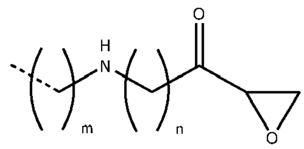 (10) 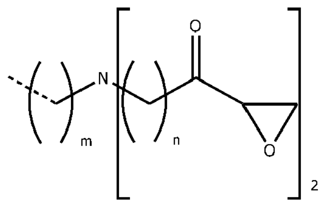 (11)

, wherein $R_6$ can be represented by H if it is not represented by any of formulae (9) to (11),

wherein k, m, n are as defined herein;

wherein the method comprises the steps of:

a) providing

a compound represented by formula (1),

or

a compound represented by formula (2),

each prepared by the method according to any of claims 8 to 11;

b) reacting the compound provided by step a) with a compound selected from the group consisting of $Mn(acac)_3$, Mn(II) abietate, and combinations thereof in an oxygen-containing atmosphere, followed by an addition of a base to form the groups represented by any of formulae (9), (10) or (11).

According to a preferred embodiment of the present invention, the base is selected from the group consisting of: 1,8-diazabicyclo[5.4.0]undec-7-ene, KOH, and combinations thereof, preferably wherein the base is 1,8-diazabicyclo [5.4.0]undec-7-ene.

The term "oxygen-containing atmosphere" refers to an atmosphere having an oxygen content of at least 1% (v/v). Preferably, the oxygen-containing atmosphere includes oxygen in an amount ranging from 1% (v/v) to 20.95% (v/v). An example of the oxygen-containing atmosphere is an air atmosphere.

The present invention also refers to the use of a compound represented by formula (1):

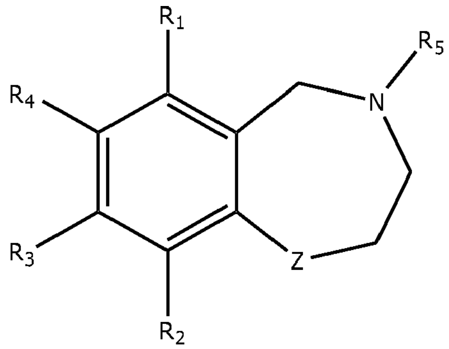

(1)

; wherein Z, $R_1$, $R_2$, $R_3$, $R_4$, are as defined herein,
wherein $R_5$ is independently selected from the group represented by any of formulae (3) to (5):

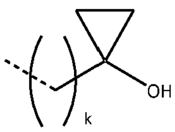

(3)

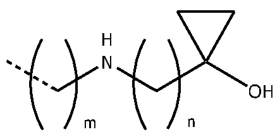

(4)

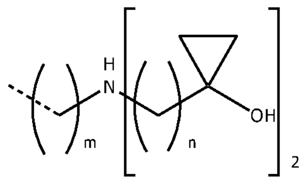

(5)

wherein k, m, n are as defined herein;
to synthesize a compound represented by formula (1"):

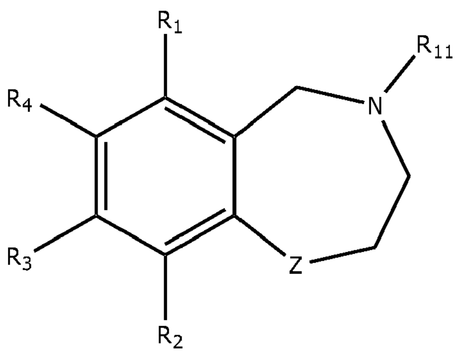

(1")

; wherein Z, $R_1$, $R_2$, $R_3$, $R_4$, are as defined herein,
wherein $R_{11}$ is independently selected from the group represented by any of formulae (9) to (11):

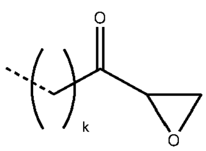

(9)

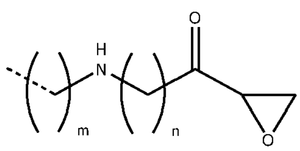

(10)

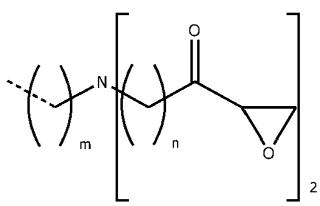

(11)

wherein k, m, n are as defined herein.

The present invention also refers to the use of a compound represented by formula (2):

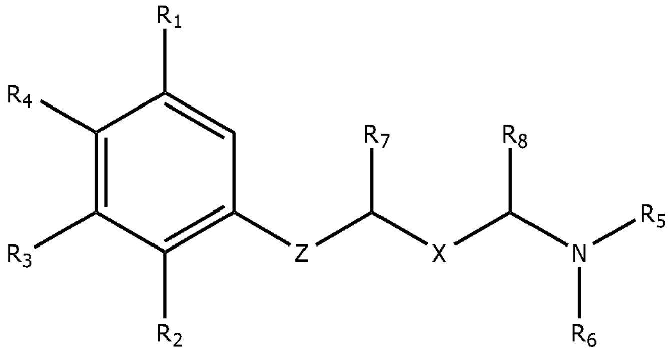

(2)

, wherein Z, X, $R_1$, $R_2$, $R_3$, $R_4$, $R_7$, and $R_8$ are as defined herein, wherein $R_5$ and optionally $R_6$ are is independently selected from the group represented by any of formulae (3) to (5):

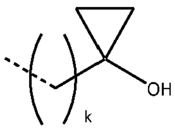 (3) 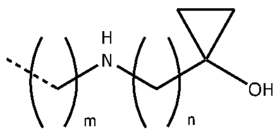 (4) 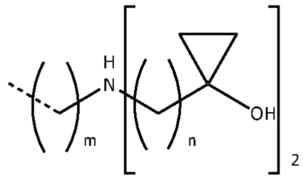 (5)

, wherein $R_6$ can be represented by H if it is not represented by any of formulae (3) to (5), wherein k, m, n are as defined herein;
to synthesize a compound represented by formula (2"):

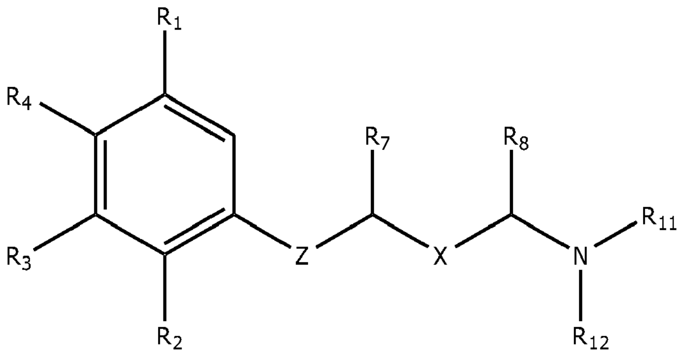

(2")

, wherein Z, X, $R_1$, $R_2$, $R_3$, $R_4$, $R_7$, and $R_8$ are as defined herein, wherein $R_{11}$ and optionally $R_{12}$ are independently selected from the group represented by any of formulae (9) to (11):

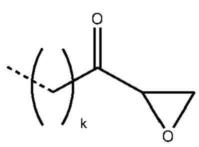 (9) 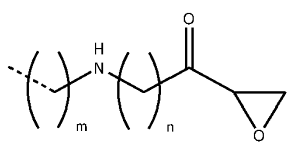 (10) 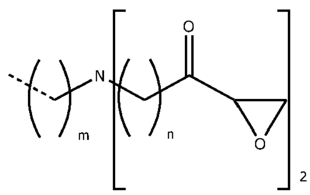 (11)

, wherein $R_{12}$ can be represented by H if it is not represented by any of formulae (9) to (11); wherein k, m, n are as defined herein.

## Description of the Figures

Figure 1: Synthesis scheme showing the synthesis of multifunctional compounds with cyclopropanol residues based on 1,4 -benzothiazepines.
The respective reaction conditions (a) to (i) are as follows: (a) Jones reagent, aceton; (b) $H_2SO_4$, EtOH; (c) EtMgBr, Ti(O*i*Pr)$_4$, EtzO; (d) *t*-BuMe$_2$SiOSO$_2$CF$_3$, 2,6-lutidine, DCM; (e) Hz, Pd/C, THF; (f) Dess-Martin reagent, DCM; (g) $K_2CO_3$, 1,4-dioxane; (h) $N_2H_4$*$H_2O$, EtOH; (i) Na($CH_3COO)_3$BH, 1,2-dichloroethane; (j) NaH, DMF; (k) aq. HF (55%), MeCN or 1 M n-Bu$_4$NF in THF or KF, Et$_3$N*HCl, MeOH. m,n = 2-8.

Figure 2: Synthesis scheme showing the synthesis of multifunctional compounds with cyclopropanol residues based on the "open" analogs 1,4 -benzothiazepines (i.e. 3-[(4-methoxyphenyl)oxy]-and 3-[(4-methoxyphenylthio)]propane-1-amine derivatives).
The respective reaction conditions (i) to (k) are as follows: (i) Na($CH_3COO)_3$BH, 1,2-dichloroethane; (j) NaH, DMF; (k) 1 M n-Bu$_4$NF in THF or KF, Et$_3$N*HCl, MeOH. m, n = 2-8; (I) 3-bromopropan-1-amine hydrobromide, $K_2CO_3$, EtOH.

Figure 3: Representation of the multifunctional compounds that have been synthesized and evaluated.

Figure 4: Representation of the structures of S36, S107 and ARM210 as well as JTV-519 and *N*-[(2-methoxyphenyl) methyl]-4,5-dimethyl-*N*-propyl-2-thiophenecarboxamide (compound at the bottom of Fig. 4).

Figure 5: R-CEPIA1er assay fluorescence measurements with HEK-293 cells expressing WT RyR2. The effect of compound addition (25 μM, 1 mM S107) on $[Ca^{2+}]_{ER}$ was normalized to the control (Cnt, 0.1 v/v% DMSO). Values represent the mean ± S.D., *** $P < 0.0005$, **** $P < 0.0001$ vs. DMSO by unpaired *t* test; n = 6 - 28.

Figure 6: Time-lapse fluorescence measurements using TECAN Spark 20M plate reader with HEK293 cells expressing WT RyR2 and R-CEPIA1er indicator. (a) R-CEPIA1*er* assay, control (0.1 v/v% DMSO) or 100 μM of compound 14a was injected at 90 seconds (arrow). Fluorescence ratio $F/F_0$, where $F_0$ - average fluorescence signal for the first 90 seconds, *F*- average fluorescence signal for the last 100 s, was taken for the calculations. (b-d) Dose-response effects of compounds 13 and 14 on $[Ca^{2+}]_{ER}$ in HEK293-RyR2-R-CEPIA1*er* cells. Data represent as mean ± SD, n = 8 - 32 independent measurements.

Figure 7: Dantrolene (Dan), S36, and GM1869 modulate differentially Ca2+ spark activity and Ca2+ uptake in saponin-permeabilized ventricular cardiomyocytes (sp-vCM) from WT and RyR2R2474S/+ mice. (A, B, C) Reduction of Ca2+ spark frequency (in % of control) by Dan (A, 10μM), S36 (B, 10 μM), and GM1869 (C, 10 μM) in cardiomyocytes from WT and RyR2R2474S/+ mice. Numbers correspond to the number of cells/number of mice. Please note that the data shown for WT correspond to the respective data pairs from Fig. 3 (Ca2+ spark activity). Data sets were analyzed by unpaired Student's t-test. n.s. non-significant with P=0.59 for Dan, **, P<0.01 with P=0.003 for S36, and *, P<0.05 with P=0.019 for GM1869; (D, E, F) Relative changes in the decay time tau of Ca2+ transients from spontaneous Ca2+ waves (in control/drug) by Dan (D, 10μM), S36 (E, 10 μM), and GM1869 (F, 10 μM) in cardiomyocytes from WT and RyR2R2474S/+ mice. Data points for Dan (D) were significantly above 1 for both mice strains (paired Student's t-test with P= 0.0008 for WT and P =0.03 for RyR2R2474S/+ mice). Data points for S36 (E) were not significantly different from 1 in cardiomyocytes from WT and RyR2R2474S/+ mice (paired Student's t-test with P= 0.94 for WT and P =0.31 for RyR2R2474S/+ mice). Data points for GM1869 (F) were significantly below 1 in cardiomyocytes from WT and RyR2R2474S/+ mice (paired Student's t-test with P= 0.004 for WT and P =0.02 for RyR2R2474S/+ mice). Numbers correspond to the number of cells/number of mice. Please note that the data shown for WT correspond to the respective data pairs from Figure 4 (Ca2+ decay times). Data sets between mouse strains were analyzed by unpaired Student's t-test. n.s. non-significant with P=0.72 for Dan, with P=0.28 for S36, and P=0.77 for GM1869.

Figure 8: S36 and GM1869 modulate differentially electrically induced Ca2+ transients in intact human iPSC-derived cardiomyocytes. (A, B) Overlay of representative Ca2+ transients in control conditions and in the presence of S36 (A, 0.1μM) and GM1869 (B, 0.1μM) in iPSC-derived cardiomyocytes. Insets show the overlay of the Ca2+ transients from start to peak (A) and from 70% of the maximum to baseline (B). (C, E) Changes in rise times of Ca2+ transients in the presence of S36 (C) and GM1869 (E). Rise times represent the tau values calculated by a mono-exponential fit of the Ca2+ transients from start to the peak of the signal. Ca2+ transients were recorded in control conditions and every 2 min (S36) or 4 min (GM1869) after drug application. **, P = 0.0065 for S36; ****, P = 0.000019 for GM1869, evaluated by paired Student's t-test. (D, F) Changes in decay times of Ca2+ transients in the presence of S36 (D) and GM1869 (F). Decay times represent the tau values calculated by a mono-exponential fit of the Ca2+ transients from half maximum to baseline of the signal. Ca2+ transients were recorded in control conditions and every 2 min (S36) or 4 min (GM1869) after drug application. Data sets were analyzed by paired Student's t-test. n.s., P = 0.14; **, P = 0.0043. (G-H) Concentration-response curves for S36 (G, H) and GM1869 (I, J) on rise times (G, I) and decay times (H, J) of Ca2+ transients, respectively, in RyR2-WT (red) and RyR2-R2474S+/- (blue) iPSC cardiomyocytes. Data from each experiment were normalized to the first transient recorded. Each cell was treated cumulatively with 3 increasing drug concentrations. Data are shown as mean ± SEM. Lines represent a non-linear fit of each data set using Graphpad Prism (with log(agonist) vs. response (three parameters)). IC50 values for the rise times were estimated to 150 pM (WT) and 40 pM (R2474S+/-) for S36 (G) and to 25 pM (WT) and 45 pM (R2474S+/-) for GM1869 (I). EC50 values for the decay times were estimated to 97 pM (WT) and 71 pM (R2474S+/-) for GM1869 (J) whereas no EC50 values were obtained for the data sets with S36. Instead, data sets with S36 were manually approximated by the dotted lines shown (H). Numbers correspond to the number of cells investigated.

Figure 9: Increase of caffeine-induced $Ca^{2+}$ release by test compounds. Effects of 10 μM solutions of CDN1163, ARM210, S107, **13** and **14** (25 μM for **13d)** on caffeine-induced $Ca^{2+}$ release. Values represent the mean ± S.D., ** *P* < 0.005, *** *P* < 0.001, **** *P* < 0.0001 vs. control (Cnt, 0.1 v/v% DMSO) by unpaired t test, n = 8 - 24.

Figure 10: Caffeine-induced $Ca^{2+}$ release assay. (a) HL-1 cells were incubated with FLIPR Calcium 6 $Ca^{2+}$ indicator and then treated with various concentrations of compound 14c. $Ca^{2+}$ influx was initiated by the addition of 10 mM caffeine. The differences between the caffeine induced peak minus basal fluorescence, $\Delta F_{Caff}$, were taken for the analysis. The inset shows the dose-response curve of 14c. (b) Dose-response curves of compounds 13, 14a, b. The response on caffeine addition was normalized to the control (0.1 v/v% DMSO). Data represent as mean ± S.D., n = 8 independent measurements.

Figure 11: S36 and S107 have shown no concentration dependent effect in caffeine-induced $Ca^{2+}$ release assay, n = 8.

Figure 12: Dose-response curves of CDN1163 and ARM210 in caffeine-induced $Ca^{2+}$ release assay. The data were normalized to the 0.1 v/v% DMSO. Values represent the mean ± S.D., n = 8.

Figure 13: NADH-coupled ATPase assay. (a) Mouse ventricular microsomes were incubated in the assay buffer with and without 100 μM 14a. After ATP injection the intrinsic fluorescence of NADH decreases due to ATP consumption. The reaction rate of NADH oxidation is greater in the mouse SR sample containing 14a. (b) Compound 14a evokes an enhancement in the kinetic rate of NADH - $NAD^+$ conversion in a dose-dependent manner. The effects of compound 14a are expressed as a percentage of the control effect. Data represented as mean ± S.D., n = 3 - 15 independent measurements. (c) Effect of 10 μM 13, 14 (3 μM for 14b) SERCA2a activity in mouse SR. The data were normalized to the control (Cnt, 0.1 v/v% DMSO). Values represent the mean ± S.D., * *P* < 0.05, ***P* < 0.005, ****P* < 0.001, *****P* < 0.0001 vs. control by unpaired t test; n = 9 - 24/ 3 - 5 mice.

Figure 14: SERCA2 activity measurements on microsomal membrane vesicles derived from HEK-293T cells. Effect of 10 μM **13, 14** (1 μM for **14b),** ARM210 and CDN1163 on SERCA2 activity. The rate of ATP consumption was normalised to the 0.1 v/v% DMSO. Values represent the mean ± S.D., **P*< 0.05 vs. control (0.1 % DMSO) by unpaired t test; n = 6 - 14.

Figure 15: Cell viability after 24-hour incubation with test compounds 13a to 14c. HL cells were treated with various concentrations of the tested compounds (24 h) and assayed using CytoTox-Glo™ Cytotoxicity kit. The effect from non-treated cells (incubation in DMEM for 24 hours) was taken as a 100% cell viability. Data represent as mean ± SD, n = 3 - 6 independent measurements.

## Examples

### 1. Synthesis of multifunctional agents for treatment of cardiovascular diseases

The synthesis of multifunctional compounds with cyclopropanol residues based on 1,4 -benzothiazepines include the following major steps (see Figure 1):

1) preparation of aldehyde **4** and bromide **8** including the Kulinkovich cyclopropanation followed by *tert*-butyldimethylsilyl (TBDMS) protection of the hydroxyl group [O. G. Kulinkovich, The Chemistry of Cyclopropanols. Chem. Rev. 2003, 103, 2597 - 2632; O. G. Kulinkovich, D. A. Astashko, V. I. Tyvorskii, N. A. Ilyina, Synthesis of α,β-Epoxy Ketones from Alkyl- and Arylsubstituted Cyclopropanols. Synthesis 2001, 2001, 1453 - 1455];
2) introduction of a linker (**C**);

3) alkylation of amine **10** with alkyl bromide **8** followed by cleavage of *tert*-butyldimethylsilyl group **(13, 14),** or
4) reductive alkylation of the amine **10** with aldehyde **6** cleavage of *tert*-butyldimethylsilyl group (**13, 14).**

The synthesis of "open" analogs of 1,4 -benzothiazepines (i.e. 3-[(4-methoxyphenyl)oxy]-and 3-[(4-methoxyphenylthio)]propane-1-amine derivatives; see Figure 2) can be performed similarly.
The multifunctional agents as depicted in Figure 3 have been synthesized and evaluated.
For the structures of S36, S107 and ARM210 as well as JTV-519 and *N*-[(2-methoxyphenyl)methyl]-4,5-dimethyl-*N*-propyl-2-thiophenecarboxamide see Figure 4.

1.1 General materials and methods

All reagents and solvents were purchased from commercial sources and used without further purification. Flash chromatography was performed using Biotage Isolera ONE flash purification system with a cartridge and solvent gradient indicated. NMR spectra were recorded at ambient temperature on Agilent 400-MR spectrometer (MPI NAT Göttingen) at 400.06 MHz ($^1$H) and 100.60 MHz ($^{13}$C) and the chemical shifts are reported in ppm. All $^1$H spectra are referenced to tetramethylsilane ($\delta$ =0 ppm) using the signals of the residual protons of $CHCl_3$ (7.26 ppm) in $CDCl_3$, $CHD_2CN$ (1.94 ppm) in $CD_3CN$, CHDzOD (3.31 ppm) in $CD_3OD$ or DMSO-$d_5$ (2.50 ppm) in DMSO-$d_6$. $^{13}$C spectra are referenced to tetramethylsilane ($\delta$ = 0 ppm) using the signals of the solvent: $CDCl_3$ (77.16 ppm), $CD_3CN$ (1.32 ppm), $CD_3OD$ (49.00 ppm) or DMSO-$d_6$ (39.52 ppm). Multiplicities of signals are described as follows: s = singlet, d = doublet, q = quartet, dq = doublet of quartets, m = multiplet or overlap of signals. Low resolution mass spectra (50 - 3500 m/z) with electro-spray ionization (ESI) were recorded on a Varian 500-MS spectrometer (Agilent) at MPI NAT Göttingen. High resolution mass spectra (ESI-HRMS) were recorded on a MICROTOF spectrometer (Bruker) equipped with ESI ion source (Apollo) and direct injector with LC autosampler Agilent RR 1200 in the Institute of Organic and Biomolecular Chemistry (Georg-August-Universität Göttingen).
Liquid chromatography: analytical HPLC was per-formed with Knauer AZURA liquid chromatography system. Analytical column: Knauer Eurosphere II 100-5, C18-H, 5 μm, 150×4.6 mm (unless otherwise stated); solvent A: $H_2O$ + 0.1% v/v TFA, solvent B: MeCN + 0.1% v/v TFA; temperature 25 °C. All final compounds are >95% pure by HPLC analysis (HPLC peak area >95%). The purity of the compounds was additionally confirmed by the LCMS method. UltiMate 3000 Standard (SD) HPLC Systems with ISQ EM Single Quadrupole Mass-Spectrometer were employed. Column: Phenomenex (2.6 μm, 3.0 × 75 mm). Flow rate: 0.5 mL/min. UV detection wavelength: 254 nm. solvent A: $H_2O$ + 0.1% formic acid, solvent B: MeCN + 0.1% for-mic acid; temperature 25 °C. Analytical TLC was per-formed on MERCK ready-to-use plates with silica gel 60 ($F_{254}$).

1.2 Compound S36

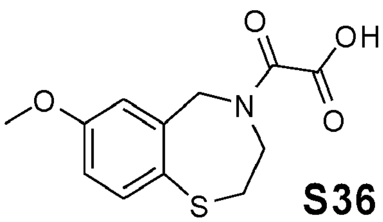

**S36**

Compound S36 was obtained in two steps from 7-methoxy-2,3,4,5-tetrahydrobenzo[1,4-*f*]thiazepine (BLD Pharm, Kaiserslautern, Germany) as described by Marks et al [A. R. Marks, D. W. Landry, S. Deng, C. Z. Zhuang, S. E. Lehnart, Agents for preventing and treating disorders involving modulation of the RyR receptors. US7879840B2, February 1, 2011.**].**

1.3 Compound S107

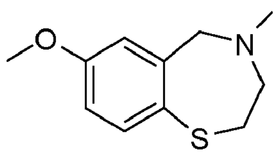

**S107**

Compound S107 was synthesized by methylation of 7-methoxy-2,3,4,5-tetrahydrobenzo[1,4-*f*]thiazepine with aqueous formaldehyde as described by Smith et al [C. D. Smith, A. Wang, K. Vembaiyan, J. Zhang, C. Xie, Q. Zhou, G. Wu, S. R. Chen, T. G. Back, Novel carvedilol analogues that suppress store-overload-induced Ca2+ release. J. Med. Chem. 2013, 56, 8626 - 8655].

1.4 Compound ARM210

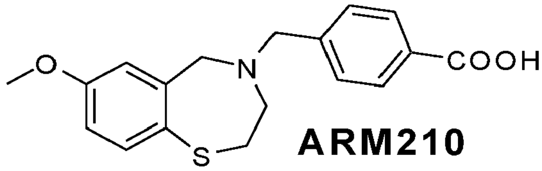

**ARM210**

ARM210 was obtained from 7-methoxy-2,3,4,5-tetrahydrobenzo[1,4-*f*]thiazepine (20 mg, 0.1 mmol) and 43 mg 4-(bromomethyl)benzoic acid (0.2 mmol) with 20 μl (0.26 mmol) pyridine in 3 mL DCM. The reaction mixture was stirred overnight at rt. After the solvent was evaporated, 2 mL water was added and the aqueous solution extracted with EtOAc (2×5 mL). The combined organic solutions were dried over $Na_2SO_4$. The filtrate was evaporated, and the product isolated by flash column chromatography using SNAP Ultra 10 g cartridge with SiOz (gradient: MeOH in DCM 8% - 100%). Yield - 9 mg (27%) of yellowish oil. $^1$H NMR (400 MHz, $CD_3OD$) $\delta$8.19 - 8.10 (m, 2H, $H_{Ar}$), 7.69 - 7.62 (m, 2H, $H_{Ar}$), 7.57 (d, *J* = 9.2 Hz, 1H, $H_{Ar}$), 7.04 - 6.96 (m, 2H, $H_{Ar}$), 4.73 (s, 2H, $CH_2$), 4.51 (s, 2H, $CH_2$), 3.82 (s, 3H, $CH_3$), 3.69 - 3.59 (m, 2H, $CH_2$), 3.16 - 3.06 (m, 1H, $CH_2$). ESI-MS, negative mode: *m*/*z*(rel. int., %) = 328 (100) [*M*-H]$^-$. HRMS *m*/*z* calcd for $C_{18}H_{18}NO_3S$ [M - H]$^-$, 328.1013; found, 328.1017.

1.5 Ethyl 5-(benzyloxy)pentanoate (**2**)

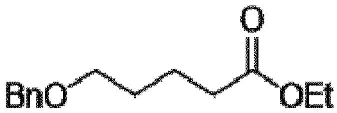

Ethyl 5-(benzyloxy)pentanoate (**2**) was obtained in two steps from 5-(benzyloxy)pentan-1-ol (BLD Pharm, 5g, 26 mmol) as described by Shi et al [Z.-F. Shi, W.-Y. Chai, P. An, X.-P. Cao, Novel Synthesis of Amphiphilic Dendrons by the Double-Stage Convergent Method. Org. Lett. 2009, 11, 4394-4397].

1.6 1-(4-(Benzyloxy)butyl)cyclopropan-1-ol **(3)**

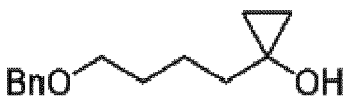

1-(4-(Benzyloxy)butyl)cyclopropan-1-ol (3) was prepared from compound **2** by the Kulinkovich reaction [O. G. Kulinkovich, D. A. Astashko, V. I. Tyvorskii, N. A. Ilyina, Synthesis of α,β-Epoxy Ketones from Alkyl- and Arylsubstituted Cyclopropanols. Synthesis 2001, 2001, 1453 - 1455]. To a mixture of Ti(O$^i$Pr)$_4$ (279 μl, 265 mg, 0.93 mmol) and ethyl 5-(benzyloxy)pentanoate (2.6 g, 11 mmol) in 3 mL EtzO a solution of EtMgBr in diethyl ether (3 M, 8 mL, 24 mmol)was added over 1 h at 0 °C. The reaction mixture was stirred for 5 h at 0 °C, hydrolyzed by addition of cold 10% $H_2SO_4$ solution in water (20 mL) and then extracted with EtOAc (3 × 10 mL). The combined organic solutions were washed with saturated aq.

NaHCOs, brine and dried over $Na_2SO_4$. The product was used as obtained without further purification. Yield - 2.4 g (99%) of yellowish oil. $^1$H NMR (400 MHz, $CDCl_3$) $\delta$7.34 (s, 5H, $H_{Ar}$), 4.51 (s, 2H, $OCH_2$), 3.50 (s, 2H, $OCH_2$), 2.49 - 2.37 (m, 2H, $CH_2$), 1.71 - 1.51 (m, 5H, OH, $CH_2$), 0.73 (s, 2H, $CH_2$), 0.43 (s, 2H, $CH_2$). $^{13}$C NMR (101 MHz, $CDCl_3$) $\delta$ 138.5, 128.4, 128.3, 127.7, 127.6, 127.5, 72.9, 70.4, 70.0, 42.0, 38.0, 29.6, 22.6, 13.5, 13.5. ESI-MS, positive mode: *m/z*(rel. int., %) = 221 (100) $[M + H]^+$.

1.7 (1-(4-(Benzyloxy)butyl)cyclopropoxy)(tert-butyl)dimethylsilane (**6**)

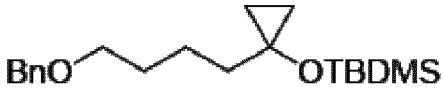

(1-(4-(Benzyloxy)butyl)cyclopropoxy)(*tert*-butyl)dimethylsilane (**6**) was obtained from compound **3** (1.2 g, 5.4 mmol), *tert*-butyldimethylsilyl trifluoromethanesulfonate (Sigma Aldrich, 2.2 g, 8.2 mmol) and 2,6-lutidine (Acros Organics, 1.4 g, 13.1 mmol) in 10 mL DCM. The reaction mixture was stirred at 0 °C for 2 h, poured onto ice (30 mL) and saturated aq. NaHCOs (30 mL) and extracted with DCM. The combined organic solutions were dried over $Na_2SO_4$. The filtrate was evaporated, and the product isolated by flash column chromatography using SNAP Ultra 25 g cartridge with SiOz (gradient: DCM in hexane 8% - 100%).Yield - 1.7 g (94%) of clear oil. The compound was used in the next reaction without further purification. $^1$H NMR (400 MHz, $CDCl_3$) $\delta$7.35 (s, 2H, $H_{Ar}$), 7.34 - 7.33 (m, 2H, $H_{Ar}$), 7.31 - 7.27 (m, 1H, $H_{Ar}$), 4.51 (s, 2H, $CH_2$), 3.50 -- 3.45 (m, 2H, $CH_2$), 1.70 - 1.34 (m, 6H, $CH_2$), 0.85 (d, *J* = 0.5 Hz, 9H, $CH_3$), 0.74 - 0.62 (m, 2H, $CH_2$), 0.45 - 0.32 (m, 2H, $CH_2$), 0.09 (s, 6H, $SiCH_3$). $^{13}$C NMR (101 MHz, $CDCl_3$) $\delta$138.8, 128.5, 127.7, 127.6, 73.0, 70.7, 56.9, 39.1, 30.0, 25.9, 22.8, 17.9, 13.2, -3.3. ESI-MS, positive mode: *m/z* (rel. int., %) = 335 (100) $[M + H]^+$.

1.8 4-(1-((*tert*-Butyldimethylsilyl)oxy)cyclopropyl)butan-1-ol **(5)**

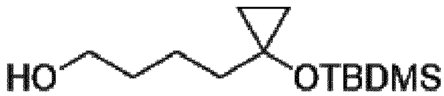

A 50 mL Schlenk-flask was evacuated and flushed with argon two times. Pd/C (Merck, 89 mg; oxidized form) and THF (4 mL) were added, and the mixture was stirred vigorously under hydrogen to activate the catalyst. A solution of **6** (200 mg, 0.60 mmol) in 2 mL of THF was then added. The reaction mixture was stirred for 30 min at room temperature under $H_2$. Hydrogen was replaced with argon, and the mixture filtered through Celite. The filter cake was washed with MeOH. The solvents were evaporated *in vacuo.* The title compound was isolated by flash chromatography using Biotage® HP-Sfär Silica HC Duo 20 $\mu$m, 25 g (gradient: DCM in hexane 8% - 100%). Yield - 90 mg (62%) of clear oil. $^1$H NMR (400 MHz, $CDCl_3$) $\delta$3.68 - 3.60 (m, 2H, $CH_2$), 1.66 - 1.38 (m, 2H, 6H, $CH_2$), 0.84 (s, 9H, $CH_3$), 0.71 - 0.64 (m, 2H, $CH_2$), 0.44 - 0.38 (m, 2H, $CH_2$), 0.08 (s, 6H, $CH_3$). $^{13}$C NMR (101 MHz, $CDCl_3$) $\delta$63.0, 56.7, 38.9, 32.8, 25.7, 22.2, 17.8, 13.0, -3.5. ESI-MS, positive mode: *m/z* (rel. int., %) = 245 (100) $[M + H]^+$. HRMS *mlz* calcd for $C_{13}H_{28}NaO_2Si$ $[M + Na]^+$, 267.1751; found, 267.1754.

1.9 4-(1-((*tert*-Butyldimethylsilyl)oxy)cyclopropyl)butanal (**4**)

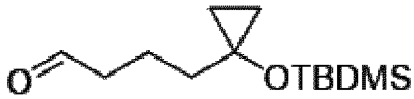

4-(1-((*tert*-Butyldimethylsilyl)o>cy)cyclopropyl)butanal (**4**) was prepared from alcohol **5** (512 mg, 2.1 mol) and Dess-Martin periodinane (TCI, 1.15 g, 2.7 mmol) in 5 mL DCM by the method of Dess & Martin [D. B. Dess, J. C. Martin, Readily accessible 12-I-5 oxidant for the conversion of primary and secondary alcohols to aldehydes and ketones. J. Org. Chem. 1983, 48, 4155 - 4156]. Yield - 467 mg (92%) of yellowish oil. $^1$H NMR (400 MHz, $CD_3CN$) $\delta$9.69 (s, 1H, CH), 2.46 - 2.38 (m, 2H, $CH_2$), 1.84 - 1.73 (m, 2H, $CH_2$), 1.56 - 1.47 (m, 2H, $CH_2$), 0.85 (s, 9H, $CH_3$), 0.67 (d, *J* = 5.1 Hz, 2H, $CH_2$), 0.47 - 0.37 (m, 2H, $CH_2$), 0.10 (s, 6H, $CH_3$). $^{13}$C NMR (101 MHz, $CD_3CN$) $\delta$ 204.0 (CO), 118.3, 67.7, 57.4, 44.1, 39.0, 26.1, 19.6,

13.5, -3.2. ESI-MS, positive mode: *m/z* (rel. int., %) = 242 (100) $[M]^+$. HRMS *m/z* calcd for $C_{18}H_{26}NaO_2Si$ $[M + Na]^+$, 265.1594; found, 265.1596.

1.10 (1-(4-Bromobutyl)cyclopropoxy)(*tert*-butyl)dimethylsilane **(8)**

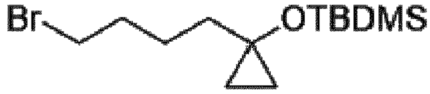

(1-(4-Bromobutyl)cyclopropoxy)(*tert*-butyl)dimethylsilane (**8**) was obtained from ethyl 5-bromopentanoate (BLD Pharm) using the same procedure as described by Elek et al [G. Z. Elek, K. Koppel, D. M. Zubrytski, N. Konrad, I. Järving, M. Lopp, D. G. Kananovich, Divergent Access to Histone Deacetylase Inhibitory Cyclopeptides via a Late-Stage Cyclopropane Ring Cleavage Strategy. Short Synthesis of Chlamydocin. Org. Lett. 2019, 21, 8473-8478].

1.11 2-(4-(7-Methoxy-2,3-dihydrobenzo [1,4- *f*]thiazepin-4(5*H*)-yl)butyl)isoindoline-1,3-dione **(9)**

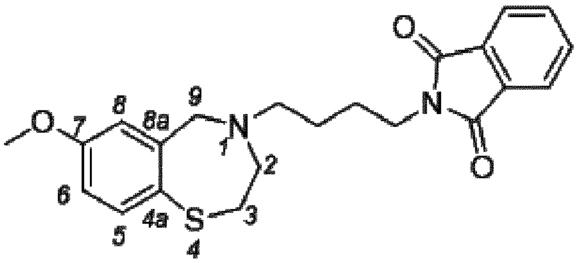

2-(4-Bromobutyl)isoindoline-1,3-dione (BLD Pharm, 700 mg, 2.5 mmol) and $K_2CO_3$ (Merk, 350 mg, 2.5 mmol) were added to a solution of 7-methoxy-2,3,4,5-tetrahydrobenzo[1,4-*f*]thiazepine (530 mg, 2.7 mmol) in anhydrous 1,4-dioxane (20 mL). Then the reaction mixture was refluxed overnight in the argon atmosphere. After the reaction mixture was filtered from the precipitate, 10 mL DCM and 10 mL water were added to the filtrate. The organic phase was separated, the aqueous layer was extracted with DCM (2×3 mL). The combined organic solutions were dried over $Na_2SO_4$. The filtrate was evaporated, and the product isolated by flash chromatography using Biotage® SNAP Ultra 25 g cartridge with SiOz (gradient: ethyl acetate in hexane 20% - 100%). Yield - 683 mg (69%) of yellowish oil. $^1H$ NMR (400 MHz, $CDCl_3$) $\delta$7.85 - 7.78 (m, 2H, $H_{Ar}$), 7.73 - 7.66 (m, 2H, $H_{Ar}$), 7.42 (d, *J* = 8.4 Hz, 1H, $H_{Ar}$-5), 6.78 (d, *J* = 2.8 Hz, 1H, $H_{Ar}$-8), 6.66 (dd, *J* = 8.4, 2.8 Hz, 1H, $H_{Ar}$-6), 4.09 (s, 2H, $NCH_2$), 3.77 (s, 3H, $OCH_3$), 3.68 (t, *J* = 7.1 Hz, 2H, $CH_2$), 3.33 - 3.26 (m, 2H, $CH_2$), 2.67 (dd, *J* = 6.6, 3.3 Hz, 2H, $CH_2$), 2.45 - 2.35 (m, 2H, $CH_2$), 1.73 - 1.63 (m, 2H, $CH_2$), 1.57 - 1.48 (m, 2H, $CH_2$). $^{13}C$ NMR (101 MHz, $CDCl_3$) $\delta$168.5, 159.2, 144.7, 134.1, 134.0, 133.7, 132.2, 127.8, 123.4, 123.3, 116.9, 112.4, 59.6, 58.3, 55.4, 51.4, 37.9, 30.2, 26.4, 24.7. ESI-MS, positive mode: m/z (rel. int., %) = 397 (100) $[M + H]^+$. HRMS *m/z* calcd for $C_{22}H_{25}N_2O_3S$ $[M + H]^+$, 397.1580; found, 397.1583.

1.12 4-(7-Methoxy-2,3-dihydrobenzo[1,4-*f*]thiazepin-4(5*H*)-yl)butan-1-amine (10)

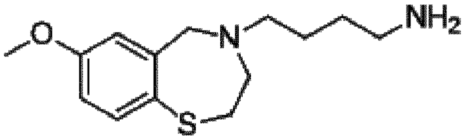

The mixture of compound **9** (230 mg, 0.58 mmol) and hydrazine hydrate (ABCR, 65 mg, 1.3 mmol) in 2 mL ethanol was stirred at 60 °C for 30 min. Then 3 mL of water was added, and the aqueous solution was extracted with DCM (2×3 mL). The combined organic solutions were dried over $Na_2SO_4$. The filtrate was evaporated, and the product isolated by preparative HPLC with gradient elution (B/A, 20/80 → 100/0). Yield - 144 mg (93%) of yellowish oil. $^1H$ NMR (400 MHz, $CDCl_3$) $\delta$7.42 (d, *J* = 8.4 Hz, 1H, $H_{Ar}$-5), 6.97 (d, *J* = 2.8 Hz, 1H, $H_{Ar}$-8), 6.72 (dd, *J* = 8.5, 2.8 Hz, 1H, $H_{Ar}$-6), 4.27 (s, 2H,

$NCH_2$), 3.79 (s, 3H, $OCH_3$), 3.48 - 3.40 (m, 2H, $CH_2$), 3.08 (t, *J* = 6.1 Hz, 2H, $CH_2$), 2.78 (dd, *J* = 6.6, 3.2 Hz, 2H, $CH_2$), 2.58 (t, *J* = 6.1 Hz, 2H, $CH_2$), 1.94 (p, *J* = 6.2 Hz, 2H, $CH_2$), 1.77 (q, *J* = 6.3 Hz, 2H, $CH_2$). $^{13}C$ NMR (101 MHz, $CDCl_3$) $\delta$ 159.2, 144.8, 133.7, 127.9, 117.1, 112.1, 77.5, 77.4, 77.2, 76.8, 59.6, 58.4, 55.5, 51.9, 42.1, 31.2, 30.2, 28.1, 25.0, 24.8. ESI-MS, positive mode: *m*/*z* (rel. int., %) = 267 (100) [M + H]$^+$. HRMS *m*/*z* calcd for $C_{14}H_{23}N_2OS$ [M + H]$^+$, 267.1526; found, 267.1527.

1.13 4-(1-((*tert*-butyldimethylsilyl)oxy)cyclopropyl)-*N*-(4-(7-methoxy-2,3-dihydrobenzo[1,4-*f*]thiazepin-4(5*H*-yl)butyl)butan-1-amine **(11a)**

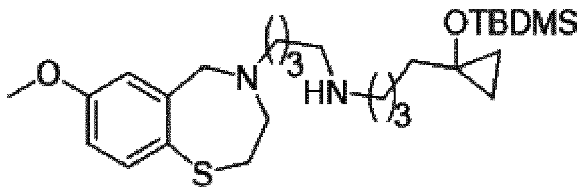

To the suspension of NaH (60% in mineral oil, 115 mg, 0.38 mmol) in 2 mL anhydrous DMF, compound **10** (100 mg, 0.38 mmol) was added and the reaction mixture was stirred at rt for 5 min. Then bromide **8** (115 mg, 0.38 mmol) was added, and the reaction mixture was stirred overnight at rt. After the solvent was removed *in vacuo,* 10 mL water and 10 mL DCM were added. The organic solution was separated, the aqueous solution extracted with DCM (2×5 mL). The combined organic solutions were dried over $Na_2SO_4$. The filtrate was evaporated, and the product isolated by flash column chromatography using Biotage® SNAP Ultra 10g cartridge (gradient: methanol in DCM 4% → 40%). Yield - 39 mg (21 %) of yellowish oil. HPLC: $t_R$ = 13.7 min (column: Interchim Uptisphere C18-HQ, particle size 10 μm, 250 × 4.6 mm; B/A: 10/90 - 100/0 in 20 min, flow 1.2 mL/min, 254 nm). $^1H$ NMR (400 MHz, $CDCl_3$) $\delta$7.45 (d, *J* = 8.5 Hz, 1H, $H_{Ar}$-5), 7.01 (d, *J* = 2.8 Hz, 1H, $H_{Ar}$-8), 6.73 (dd, *J* = 8.5, 2.8 Hz, 1H, $H_{Ar}$-6), 4.12 (s, 2H, $NCH_2$), 3.83 (s, 3H, $OCH_3$), 3.36 (d, *J* = 6.8 Hz, 2H, $CH_2$), 2.91 - 2.82 (m, 2H, $CH_2$), 2.79 - 2.75 (m, 2H, $CH_2$), 2.67 - 2.56 (m, 4H, $CH_2$), 2.18 - 2.08 (m, 2H, $CH_2$), 1.78 - 1.71 (m, 2H, $CH_2$), 1.68 - 1.57 (m, 2H, $CH_2$), 1.56 - 1.36 (m, 4H, $CH_2$), 0.83 (s, 9H, $CH_3$), 0.73 - 0.64 (m, 2H, $CH_2$), 0.45 - 0.33 (m, 2H, $CH_2$), 0.07 (s, 6H, $CH_3$). $^{13}C$ NMR (101 MHz, $CDCl_3$) $\delta$ 159.5, 142.0, 134.0, 127.7, 117.1, 113.3, 58.9, 58.3, 56.4, 52.7, 48.2, 47.7, 38.4, 30.4, 26.1, 25.7, 25.2, 23.5, 17.7, 13.1, -3.4. ESI-MS, positive mode: *m*/*z* (rel. int., %) = 493 (100) [M + H]$^+$. HRMS *m*/*z* calcd for $C_{27}H_{49}N_2O_2SSi$ [M + H]$^+$, 493.3275; found, 493.3281.

1.14 4-(1-((*tert*-Butyldimethylsilyl)oxy)cyclopropyl)-*N*-(4-(1-((*tert*-butyldimethylsilyl)oxy)cyclopropyl)butyl)-*N*-(4-(7-methoxy-2,3-dihydrobenzo [1,4-*f*]thiazepin-4(5*H*)-yl)butyl)butan-1-amine **(12a)**

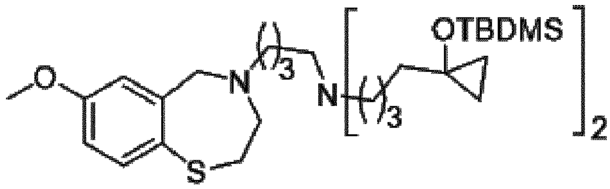

To compound **10** (19 mg, 0.07 mmol) in 0.4 mL 1,2-dichloroethane, aldehyde **4** (36 mg, 0.15 mmol) in 0.4 mL 1,2-dichloroethane and sodium triacetoxyborohydride (225 mg, 1.06 mmol) were added. The reaction mixture was stirred at rt for 1.5 h and concentrated *in vacuo.* The product was isolated by flash chromatography using Biotage® HP-Sfär Silica HC Duo 20 μm, 10 g; gradient 5% to 50% methanol in DCM. Yield - 13 mg (37 %) of yellowish oil. HPLC: $t_R$ = 17.8 min (column: Interchim Uptisphere C18-HQ, particle size 10 μm, 250 × 4.6 mm; B/A: 10/90 - 100/0 in 20 min, flow 1.2 mL/min, 254 nm). $^1H$ NMR (400 MHz, $CDCl_3$) $\delta$7.44 (d, *J* = 8.4 Hz, 1H, $H_{Ar}$-5), 6.79 (d, *J* = 2.8 Hz, 1H, $H_{Ar}$-8), 6.68 (dd, *J* = 8.4, 2.8 Hz, 1H, $H_{Ar}$-6), 4.09 (s, 2H, $NCH_2$), 3.79 (s, 3H, $OCH_3$), 3.32 - 3.26 (m, 2H, $CH_2$), 3.00 - 2.86 (m, 6H, $CH_2$), 2.69 -2.63 (m, 2H $CH_2$), 2.43 - 2.37 (m, 2H, $CH_2$), 1.82 - 1.70 (m, 6H $CH_2$), 1.58 - 1.45 (m, 10H, $CH_2$), 0.84 (s, 18H, $CH_3$), 0.71 - 0.62 (m, 4H, $CH_2$), 0.42 - 0.35 (m, 4H, $CH_2$), 0.08 (s, 12H, $CH_3$). $^{13}C$ NMR (101 MHz, $CDCl_3$) $\delta$159.2, 145.2, 133.7, 127.9, 117.0, 112.0, 59.8 ($CH_2$), 58.5 ($CH_2$), 57.0 ($CH_2$), 55.5 ($OCH_3$), 54.3, 39.2 ($CH_2$), 30.4 ($CH_2$), 25.9 ($CH_3$), 24.1, 17.9, 13.2 ($CH_2$), -3.3 ($CH_3$). ESI-MS, positive mode: *m*/*z* (rel. int., %) = 719 (100) [M + H]$^+$. HRMS *m*/*z* calcd for $C_{40}H_{75}N_2O_3SSi_2$ [M + H]$^+$, 719.5031; found, 719.5033.

1.15 1-(4-((4-(7-Methoxy-2,3-dihydrobenzo[1,4-*f*]-thiazepin-4(5*H*)-yl)butyl)amino)butyl)cyclopropan-1-ol (**13a**)

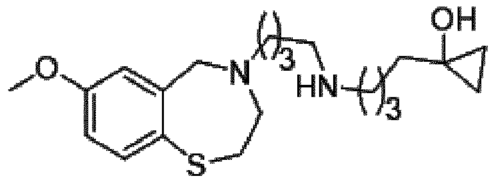

Caution: cyclopropanols can rearrange into the corresponding ethyl ketones in the presence of acids or strong bases [O. G. Kulinkovich, The Chemistry of Cyclopropanols. Chem. Rev. 2003, 103, 2597 - 2632.]! Hydrogen fluoride cleavage of TBDMS groups was performed at 0 °C in MeCN. A polyethylene container was charged with **11a** (35 mg, 71 µmol) and 1 mL MeCN and cooled down to 0 °C. Then 15 µl of aq. HF (55%) was added dropwise, and the reaction mixture stirred for 1 h at 0 °C. After the reaction mixture was washed with 5 % aq. $Na_2CO_3$, the organic layer was separated and the solvent removed in vacuo. The title compound was isolated by preparative HPLC using reversed phase cartridge Interchim PF-C18HC 12 g, particle size 30 µm and gradient elution (B/A, 10/90 → 80/20; B = MeCN + 0.1% TFA; A = $H_2O$ + 0.1% TFA). Yield - 19 mg (71 %) of clear oil. $^1$H NMR (400 MHz, DMSO-$d_6$) $\delta$ 7.51 (d, J = 8.5 Hz, 1H, $H_{Ar}$-5), 7.30 (d, J = 2.8 Hz, 1H, $H_{Ar}$-8), 6.97 (dd, J = 8.5, 2.8 Hz, 1H, $H_{Ar}$-6), 4.71 (br. s., 1H, NH), 4.57 (s, 2H, $NCH_2$), 3.78 (s, 3H, $OCH_3$), 3.71 - 3.56 (m, 2H, $CH_2$), 3.26 - 2.98 (s, 4H, $CH_2$), 2.95 - 2.85 (m, 4H, $CH_2$), 1.84 - 1.69 (s, 2H, $CH_2$), 1.69 - 1.54 (s, 4H, $CH_2$), 1.49 - 1.38 (s, 4H, $CH_2$), 0.52 (t, *J*= 5.5 Hz, 2H), 0.37 - 0.25 (m, 2H). $^{13}$C NMR (101 MHz, DMSO-$d_6$) $\delta$ 159.2, 158.1, 133.8, 127.8, 119.1, 114.9, 55.5, 53.5, 46.9, 46.0, 37.5, 25.5, 22.9, 22.6, 20.7, 12.7. ESI-MS, positive mode: m/z (rel. int., %) = 379 (100) [M + H]$^+$. HRMS m/z calcd for $C_{21}H_{35}N_2O_2S$ [M + H]$^+$, 379.2414; found, 379.2415.

1.16 1,1'-(((4-(7-Methoxy-2,3-dihydrobenzo[1,4-*f*]thiazepin-4(5*H*)-yl)butyl)azanediyl)bis(butane-4,1-diyl))bis(cyclopropan-1-ol) (**14a**)

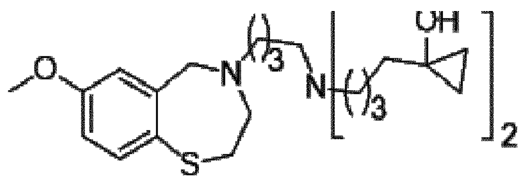

The title compound was obtained from **12a** (54 mg, 75 µmol) and 90 µl aq. HF (55%), similarly as described for compound **13a.** After the solvents were removed *in vacuo*, the title compound was isolated by preparative HPLC with gradient elution (B/A: 10/90 → 80/20). Yield - 20 mg (54%) of clear oil. $^1$H NMR (400 MHz, $CD_3OD$) $\delta$ 7.43 (d, *J* = 8.4 Hz, 1H, $H_{Ar}$-5), 6.95 (d, *J* = 2.8 Hz, 1H, $H_{Ar}$-8), 6.78 (d, *J* = 11.3 Hz, 1H, $H_{Ar}$-6), 4.15 (s, 2H, $NCH_2$), 3.79 (s, 3H, $OCH_3$), 3.36 - 3.33 (m, 2H, $NCH_2$), 3.12 - 3.01 (m, 2H, $CH_2$), 2.80 - 2.73 (m, 2H, $CH_2$), 2.54 (t, *J* = 7.0 Hz, 2H, $CH_2$), 1.80 - 1.67 (m, 6H, $CH_2$), 1.67 - 1.53 (s, 10H, $CH_2$), 0.70 - 0.63 (m, 4H, $CH_2$), 0.48 - 0.39 (m, 4H, $CH_2$). $^{13}$C NMR (101 MHz, $CD_3OD$) $\delta$ 160.9, 144.4, 134.7, 129.3, 118.5, 113.7, 60.2, 59.6, 55.9, 55.3, 54.1, 53.9, 53.0, 38.6, 31.0, 24.7, 24.7, 24.3, 22.6, 13.6. ESI-MS, positive mode: *m*/*z* (rel. int., %) = 491 (100) [M + H]$^+$. HRMS *m*/*z* calcd for $C_{28}H_{47}N_2O_3S$ [M + H]$^+$, 491.3302; found, 491.3289.

1.17 4-(1-((*tert*-Butyldimethylsilyl)oxy)cyclopropyl)-*N*-(3-(4-methoxyphenoxy)propyl)butan-1-amine (**11b**)

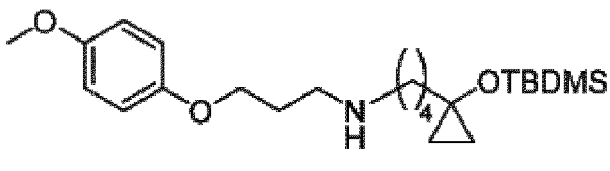

4-(1-((*tert*-Butyldimethylsilyl)oxy)cyclopropyl)-*N*-(3-(4-methoxyphenoxy)propyl)butan-1-amine (**11b**), was obtained from 3-(4-methoxyphenoxy)propan-1-amine (BLD Pharm, 180 mg, 1.00 mmol), 307 mg (1.0 mmol) (1-(4-bromobutyl)cyclopropoxy)(*tert*-butyl)dimethylsilane **8** and 120 mg NaH (60% in mineral oil) using the same procedure,

as described for compound **11a.** The title compound was isolated by flash column chromatography using Biotage® HP-Sfär Silica HC Duo cartridge 20 μm, 25 g (gradient: methanol in DCM 2% - 20%). Yield - 167 mg (41%) of yellowish solid. $^1$H NMR (400 MHz, $CDCl_3$) $\delta$ 6.85 - 6.77 (m, 4H, $H_{Ar}$), 3.98 (t, $J$ = 6.2 Hz, 2H, $NCH_2$), 3.75 (s, 3H, $OCH_3$), 2.80 (t, $J$ = 7.0 Hz, 2H, $NCH_2$), 2.67 - 2.58 (m, 2H), 2.01 - 1.91 (m, 2H), 1.57 - 1.44 (m, 6H, $CH_2$), 0.84 (s, 9H, $CH_3$), 0.70 - 0.62 (m, 2H, $CH_2$), 0.43 - 0.34 (m, 2H, $CH_2$), 0.07 (s, 6H, $CH_3$). $^{13}$C NMR (101 MHz, $cdcl_3$) $\delta$ 153.7, 153.1, 115.4, 114.6, 67.0, 56.8, 55.7, 55.7, 50.0, 46.9, 39.0, 30.0, 29.7, 25.7, 23.8, 17.8, 13.0, -3.4. ESI-MS, positive mode: *m/z* (rel. int., %) = 408 (100) [M + H]$^+$. HRMS *m/z* calcd for $C_{23}H_{42}NO_3Si$ [M + H]$^+$, 408.2928; found, 408.2931.

1.18 4-(1-((*tert*-Butyldimethylsilyl)oxy)cyclopropyl)-*N*-(4-(1-((*tert*-butyldimethylsilyl)oxy)cyclopropyl)butyl)-*N*-(3-(4-methoxyphenoxy)propyl)butan-1-amine (**12b**)

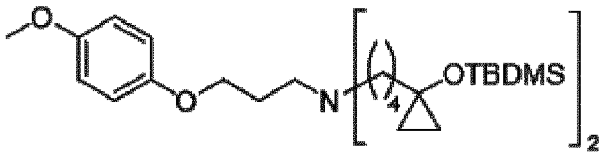

4-(1-((*tert*-Butyldimethylsilyl)oxy)cyclopropyl)-*N*-(4-(1-((tert-butyldimethylsilyl)oxy)cyclopropyl)butyl)-*N*-(3-(4-methoxyphenoxy)propyl)butan-1-amine (**12b**), was prepared from 3-(4-methoxyphenoxy)propan-1-amine (BLD Pharm, 30 mg, 0.17 mmol) and aldehyde **4** (100 mg, 0.41 mmol) in DCE according to the procedure described for compound **12a.** The title compound was isolated by flash column chromatography using Biotage® HP-Sfär Silica HC Duo 20 μm, 10 g (gradient: methanol in DCM 2% - 40%). Yield - 60 mg (55%) of yellowish oil. HPLC: $t_R$ = 5.1 min (B/A: 30/70 -100/0 in 15 min, flow 1.2 mL/min, 254 nm). $^1$H NMR (400 MHz, $CDCl_3$) $\delta$ 6.85 - 6.77 (m, 4H, $H_{Ar}$), 3.99 - 3.90 (m, 2H, $NCH_2$), 3.80 - 3.73 (m, 3H, $OCH_3$), 3.68 - 3.61 (m, 2H, $NCH_2$), 2.69 - 2.60 (m, 2H, $CH_2$), 2.50 - 2.42 (m, 2H, $CH_2$), 1.63 - 1.41 (m, 14H, $CH_2$), 0.88 - 0.79 (m, 18H, $CH_3$), 0.70 - 0.62 (m, 4H, $CH_2$), 0.45 - 0.35 (m, 4H, $CH_2$), 0.13 - 0.02 (m, 12H, $CH_3$). $^{13}$C NMR (101 MHz, $CDCl_3$) $\delta$ 153.7, 153.1, 115.4, 114.6, 63.0, 56.8, 55.7, 54.2, 50.6, 39.0, 38.9, 32.8, 25.7, 23.9, 22.2, 17.8, 13.0, -3.4. ESI-MS, positive mode: *m/z* (rel. int., %) = 634 (100) [M + H]$^+$. HRMS *m/z* calcd for $C_{36}H_{68}NO_4Si_2$ [M + H]$^+$, 634.4681; found, 634.4665.

1.19 1-(4-((3-(4-methoxyphenoxy)propyl)amino)butyl)cyclopropan-1-ol (**13b**)

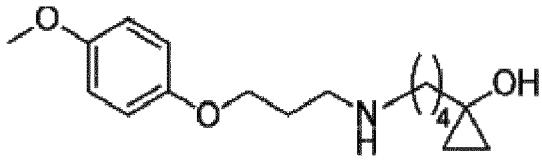

tert-Butyldimethylsilyl deprotection of **11b** (30 mg, 0.07 mmol) was performed in 140 μl 1 M TBAF in THF. The reaction mixture was stirred overnight at rt. The title compound was isolated by preparative HPLC with gradient elution (B/A: 20/80 → 100/0). Yield 5 mg (24 %) of clear oil. $^1$H NMR (400 MHz, $CDCl_3$) $\delta$ 6.82 (s, 4H, $H_{ar}$), 3.97 (t, $J$ = 6.1 Hz, 2H, $OCH_2$), 3.76 (s, 3H,$OCH_3$), 2.78 (t, $J$ = 7.0 Hz, 2H, $CH_2$), 2.70 - 2.62 (m, 2H, $CH_2$), 1.98 - 1.88 (m, 2H, $CH_2$), 1.69 - 1.52 (m, 7H, $CH_2$, OH), 0.99 (t, $J$ = 7.3 Hz, 1H, NH), 0.74 - 0.67 (m, 2H, $CH_2$), 0.43 - 0.36 (m, 2H, $CH_2$). $^{13}$C NMR (101 MHz, $CDCl_3$) $\delta$ 153.9 (C), 153.2 (C), 115.5 (CH), 114.8 (CH), 67.2 (CH), 55.9 (CH), 55.1 (C), 49.8 (CH), 47.1 (CH), 38.2 (CH), 29.8 (CH), 29.6 (CH), 23.6 (CH), 13.8 (CH). ESI-MS, positive mode: *m/z* (rel. int., %) = 294 (100) [M + H]$^+$. HRMS *m/z* calcd for $C_{17}H_{28}N_3O_3$ [M + H]$^+$, 294.2064; found, 294.2068.

1.20 1-(4-((3-(4-Methoxyphenoxy)propyl)amino)butyl)cyclopropan-1-ol (**14b**)

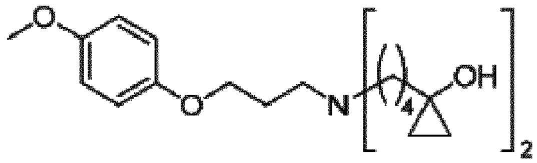

1-(4-((3-(4-Metho>cypheno>cy)propyl)amino)butyl)cyclopropan-1-ol **(14b),** was obtained by t-butyldimethylsilyl deprotection of **12b** (34 mg, 0.05 mmol) in DMF using 48 mg (0.82 mmol) KF together with 112 mg (0.82 mmol) $Et_3N \times HCl$. The reaction mixture was stirred overnight at 70°C. After the precipitate was filtered off, the DMF was evaporated *in vacuo*. The title compound was isolated by flash column chromatography using Biotage® HP-Sfär Silica HC Duo 20 μm, 10 g (gradient: methanol in DCM 2 % - 20 %). Yield - 13 mg (64 %). $^1H$ NMR (400 MHz, $CDCl_3$) $\delta$ 6.85 - 6.75 (m, 4H, $H_{ar}$), 4.01 (q, *J* = 5.2 Hz, 2H, $CH_2$), 3.75 (s, 3H, $CH_3$), 3.29 - 3.20 (m, 2H, $CH_2$), 3.15 - 3.02 (m, 4H, $CH_2$), 2.35 - 2.26 (m, 2H, $CH_2$), 1.99 - 1.86 (m, 4H, $CH_2$), 1.70 - 1.53 (m, 8H, $CH_2$), 0.77 - 0.70 (m, 4H, $CH_2$), 0.42 - 0.34 (m, 4H, $CH_2$). $^{13}C$ NMR (126 MHz, $CDCl_3$) $\delta$ 154.3, 152.4, 115.5, 114.9, 65.5, 55.8, 54.9, 53.2, 50.8, 37.3, 23.9, 23.6, 23.2, 13.7. ESI-MS, positive mode: *m/z* (rel. int., %) = 406 (100) $[M + H]^+$. HRMS *m/z* calcd for $C_{24}H_{40}NO_4$ $[M + H]^+$, 406.2952; found, 406.2957.

1.21 3-((4-Methoxyphenyl)thio)propan-1-amine (**15**)

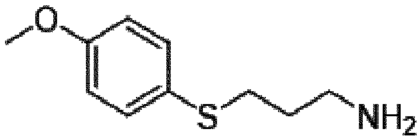

4-Methoxybenzenethiol (BLD Pharm, 280 mg, 2 mmol) was added to the solution of 3-bromopropan-1-amine hydrobromide (BLD Pharm, 432 mg, 2 mmol) in 6 mL ethanol containing potassium carbonate (560 mg, 4 mmol). Then the reaction mixture was heated to reflux. After 12h, it was poured into cold water (10 mL) and extracted with $CH_2Cl_2$ (4 × 5 mL). The extracts were combined, washed with water (3 × 5 mL) and dried over anhydrous $Na_2SO_4$. The solvent was evaporated *in vacuo* affording the title compound as a yellowish oil. Yield - 265 mg (67%). $^1H$ NMR (400 MHz, $CD_3OD$) $\delta$ 7.37 - 7.30 (m, 2H, $H_{Ar}$), 6.90 - 6.83 (m, 2H, $H_{Ar}$), 3.77 (s, 3H, $OCH_3$), 2.86 (t, *J* = 7.2 Hz, 2H, $CH_2$), 2.73 (t, *J* = 7.1 Hz, 2H, $CH_2$), 1.71 (p, *J* = 7.1 Hz, 2H, $CH_2$). $^{13}C$ NMR (101 MHz, $CD_3OD$) $\delta$ 160.5, 134.3, 127.7, 115.6, 55.8, 41.3, 33.9, 33.1. ESI-MS, positive mode: *m/z* (rel. int., %) = 198 (100) $[M + H]^+$.

1.22 4-(1-((*tert*-Butyldimethylsilyl)oxy)cyclopropyl)-*N*-(3-((4-methoxyphenyl)thio)propyl)butan-1-amine (**11c**), and 4-(1-((*tert*-butyldimethylsilyl)oxy)cyclopropyl)-N-(4-(1-((tert-butyldimethylsilyl)oxy)cyclopropyl)butyl)-*N*-(3-((4-methoxyphenyl)thio)propyl)butan-1-amine (**12c**)

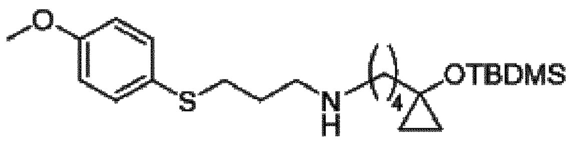

**11c**

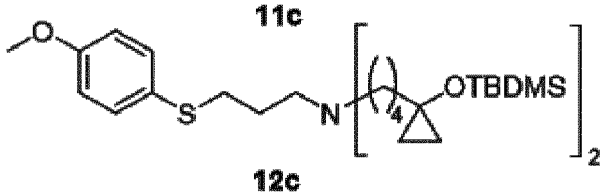

**12c**

4-(1-((*tert*-butyldimethylsilyl)oxy)cyclopropyl)-*N*-(3-((4-methoxyphenyl)thio)propyl)butan-1-amine (**11c**), *and* 4-(1-((*tert*-butyldimethylsilyl)oxy)cyclopropyl)-N-(4-(1-((tert-butyldimethylsilyl)oxy)cyclopropyl)butyl)-*N*-(3-((4-methoxyphenyl)thio)propyl)butan-1-amine (**12c**), were obtained from amine **15** (168 mg, 0.85 mmol) and bromide 8 (644 mg, 2.1 mmol) in anhydrous DMF according to the procedure as described for compound **11a.** The title compounds were isolated by flash column chromatography using Biotage® HP-Sfär Silica HC Duo 20 μm, 25 g (gradient: methanol in DCM 2% -

40%). Yield - 90 mg (21%) of *N*-mono- (**11c**) and 100 mg (18 %) of *N,N*-disubstituted compound **12c.** Compound **11c:** $^1$H NMR (400 MHz, $CDCl_3$) δ7.34 (d, *J* = 8.9 Hz, 2H, $H_{Ar}$), 6.83 (d, *J* = 8.9 Hz, 2H, $H_{Ar}$), 3.78 (s, 3H, $OCH_3$), 2.87 (t, *J* = 7.2 Hz, 2H, $CH_2$), 2.76 (t, *J* = 7.1 Hz, 2H, $CH_2$), 2.63 (t, *J* = 7.0 Hz, 2H, $CH_2$), 1.82 (p, J = 7.1 Hz, 2H, $CH_2$), 1.62 - 1.41 (m, 6H, $CH_2$), 0.84 (s, 9H), 0.70 - 0.62 (m, 2H, $CH_2$), 0.44 - 0.29 (m, 2H, $CH_2$), 0.07 (s, 6H, $CH_3$). $^{13}$C NMR (101 MHz, $CDCl_3$) δ 159.1, 133.4, 126.4, 114.7, 114.6, 56.9, 55.5, 49.8, 48.4, 39.2, 39.1, 33.8, 29.5, 29.1, 25.9, 23.8, 17.9, 13.2, 13.2, -3.3, -3.3. ESI-MS, positive mode: *m/z* (rel. int., %) = 424 (100) [M + H]$^+$. HRMS *m/z* calcd for $C_{23}H_{42}NO_2SSi$ [M + H]$^+$, 424.2700; found, 424.2698. Compound **12c:** $^1$H NMR (400 MHz, $CDCl_3$) δ7.32 (d, *J* = 8.9 Hz, 1H, $H_{ar}$), 6.82 (d, *J* = 8.9 Hz, 1H, $H_{ar}$), 3.78 (s, 3H, $OCH_3$), 2.84 (t, *J* = 7.1 Hz, 2H), 2.60 - 2.32 (m, 4H, $CH_2$), 1.86 - 1.61 (m, 4H, $CH_2$), 1.60 - 1.37 (m, 12H, $CH_2$), 0.84 (d, *J* = 1.2 Hz, 18H, $CH_3$), 0.70 - 0.63 (m, 4H, $CH_2$), 0.43 - 0.33 (m, 2H, $CH_2$), 0.07 (s, 12H, $CH_3$). $^{13}$C NMR (101 MHz, $CDCl_3$) δ 133.2, 114.7, 63.1, 56.9, 56.9, 55.4, 39.1, 39.0, 33.9, 32.9, 31.6, 26.2, 24.0, 22.3, 17.9, 13.2, 13.2, 8.4, -1.7, -3.3, -3.3. ESI-MS, positive mode: *m/z* (rel. int., %) = 650 (100) [M + H]$^+$. HRMS *m/z* calcd for $C_{36}H_{68}NO_3SSi_2$ [M + H]$^+$, 650.4453; found, 650.4434.

1.23 1-(4-((3-((4-Methoxyphenyl)thio)propyl)amino)butyl)cyclopropan-1-ol (**13c**)

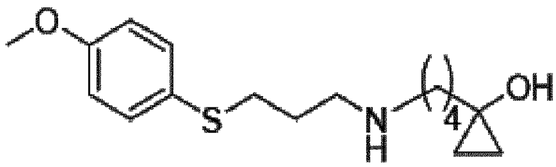

*tert*-Butyldimethylsilyl deprotection of **11c** (90 mg, 0.21 mmol) was performed in 350 μl 1 M TBAF in THF. The reaction mixture was stirred overnight at rt. The title compound was isolated by preparative HPLC with gradient elution (B/A: 10/90 → 100/0). Yield - 43 mg (67 %) of yellowish oil. $^1$H NMR (400 MHz, $CD_3OD$) δ 7.46 - 7.32 (m, 2H, $H_{ar}$), 6.95 - 6.85 (m, 2H, $H_{ar}$), 3.78 (s, 3H, $OCH_3$), 3.15 - 3.04 (m, 2H, $CH_2$), 3.05 - 2.93 (m, 2H, $CH_2$), 2.91 (t, *J* = 7.0 Hz, 2H), 1.94 (p, *J* = 7.1 Hz, 2H, $CH_2$), 1.80 - 1.65 (m, 2H, $CH_2$), 1.64 - 1.52 (m, 4H, $CH_2$), 0.72 - 0.57 (m, 2H, $CH_2$), 0.48 - 0.32 (m, 2H, $CH_2$). $^{13}$C NMR (101 MHz, $CD_3OD$) δ 160.8 (C), 134.7 (CH), 126.5 (C), 115.8 (CH), 55.8 ($CH_3$), 55.2 (C), 49.3 (CH), 49.1 (CH), 47.6 (CH), 38.6 (CH), 33.3 (CH), 27.0 (CH), 26.8 (CH), 24.1 (CH), 13.6 (CH). ESI-MS, positive mode: *m/z* (rel. int., %) = 310 (100) [M + H]$^+$. HRMS *m/z* calcd for $C_{17}H_{28}NO_2S$ [M + H]$^+$, 310.1835; found, 310.1839.

1.24 1,1'-(((3-((4-Methoxyphenyl)thio)propyl)azanediyl)bis(butane-4,1-diyl))bis(cyclopropan-1-ol) (**14c**)

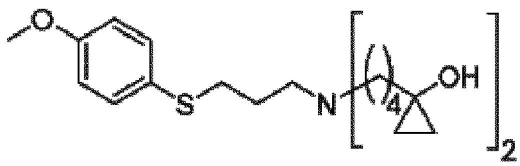

Desilylation of **12c** (107 mg, 0.15 mmol) was performed in 450 μl 1 M TBAF in THF. The reaction mixture was stirred overnight at rt. The title compound was isolated by flash column chromatography using Biotage® HP-Sfär Silica HC Duo 20 μm, 10 g (gradient: methanol in DCM 8% - 20%). Yield 33 mg (52%) of yellowish oil. $^1$H NMR (400 MHz, $CDCl_3$) δ 7.36 - 7.30 (m, 2H, $H_{Ar}$), 6.86 - 6.79 (m, 2H, $H_{Ar}$), 3.78 (s, 3H, $OCH_3$), 2.83 (t, *J* = 6.9 Hz, 2H, $CH_2$), 2.70 - 2.61 (m, 2H, $CH_2$), 2.53 (t, *J* = 6.7 Hz, 2H, $CH_2$), 1.76 (dd, *J* = 8.7, 6.3 Hz, 2H, $CH_2$), 1.59 - 1.40 (m, 12H, $CH_2$), 0.74 - 0.65 (m, 2H, $CH_2$), 0.41 - 0.33 (m, 2H, $CH_2$). $^{13}$C NMR (101 MHz, $CDCl_3$) δ 169.1, 159.0, 133.3, 126.2, 114.8, 55.5, 55.1, 53.7, 52.1, 37.9, 33.8, 25.8, 23.8, 13.7. ESI-MS, positive mode: *m/z* (rel. int., %) = 422 (100) [M + H]$^+$. HRMS *m/z* calcd for $C_{17}H_{28}NO_2S$ [M + H]$^+$, 422.2704; found, 422.2706.

1.25 4-(4-(1-((*tert*-Butyldimethylsilyl)oxy)cyclopropyl)butyl)-7-methoxy-2,3,4,5-tetrahydrobenzo[1,4-*f*]thiazepine (**16**)

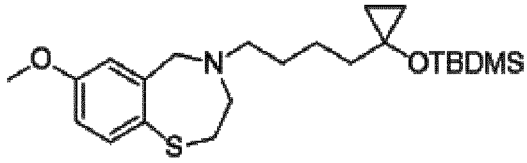

4-(4-(1-((*tert*-Butyldimethylsilyl)oxy)cyclopropyl)butyl)-7-methoxy-2,3,4,5-tetrahydrobenzo[1,4-*f*]thiazepine (**16**), was prepared from commercial 7-methoxy-2,3,4,5-tetrahydrobenzo[1,4-*f*]thiazepine (90 mg, 0.62 mmol) and aldehyde **4** (174 mg, 0.7 mmol) in DCE according to the procedure described for compound **12a.** The title compound was isolated by flash column chromatography using Biotage® HP-Sfär Silica HC Duo 20 μm, 10 g (gradient: methanol in DCM 2% - 40%). Yield - 111 mg (43%) of yellowish oil. $^1$H NMR (400 MHz, $CDCl_3$) $\delta$ 7.47 (d, *J* = 8.5 Hz, 1H, $H_{Ar}$), 6.87 (d, *J* = 2.8 Hz, 1H, $H_{Ar}$), 6.76 (dd, *J* = 8.5, 2.8 Hz, 1H, $H_{Ar}$), 4.30 (s, 2H, $CH_2$), 3.79 (s, 3H, $OCH_3$), 3.49 - 3.41 (m, 2H, $CH_2$), 2.85 - 2.79 (m, 2H, $CH_2$), 2.58 - 2.49 (m, 2H, $CH_2$), 1.67 (d, *J* = 9.6 Hz, 2H, $CH_2$), 1.47 (d, *J* = 3.7 Hz, 4H, $CH_2$), 0.82 (s, 9H, $CH_3$), 0.65 (d, *J* = 11.8 Hz, 2H, $CH_2$), 0.37 (d, *J* = 11.9 Hz, 2H, $CH_2$), 0.05 (s, 6H, $CH_3$). $^{13}$C NMR (101 MHz, $CDCl_3$) $\delta$ 159.6, 140.2, 134.0, 127.8, 117.9, 113.4, 58.7, 56.8, 55.5, 53.6, 51.8, 39.0, 28.8, 26.0, 25.8, 23.8, 17.9, 13.2, -3.3. ESI-MS, positive mode: *m*/*z* (rel. int., %) = 422 (100) [M + H]$^+$. HRMS *m*/*z* calcd for $C_{23}H_{40}NO_2SSi$ [M + H]$^+$, 422.2544; found, 422.2545.

1.26 1-(4-(7-methoxy-2,3-dihydrobenzo[1,4-*f*]thiazepin-4(5H)-yl)butyl)cyclopropan-1-ol (**13d**)

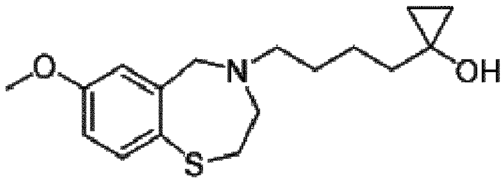

Cleavage of tert-butyldimethylsilyl ether **16** (148 mg, 0.36 mmol) was performed in 710 μl 1 M TBAF in THF, as described for compound **14c.** After the solvent was evaporated *in vacuo,* the title compound was isolated using flash column chromatography (Biotage® HP-Sfär Silica HC Duo 20 μm, 10 g, gradient: methanol in DCM 8 % - 10 %) followed by preparative HPLC with gradient elution (MeCN/$H_2O$: 10/90 → 100/0). Yield 22 mg (20 %) of clear oil. $^1$H NMR (400 MHz, $CDCl_3$) $\delta$7.45 (d, *J* = 8.4 Hz, 1H, $H_{Ar}$), 6.82 (d, *J* = 2.7 Hz, 1H, $H_{Ar}$), 6.71 (dd, *J* = 8.5, 2.8 Hz, 1H, $H_{Ar}$), 4.16 (s, 2H, $CH_2$), 3.80 (s, 3H, $CH_3$), 3.41 - 3.29 (m, 2H, $CH_2$), 2.79 - 2.66 (m, 2H, $CH_2$), 2.46 (t, *J* = 7.0 Hz, 2H, $CH_2$), 1.68 - 1.46 (m, 6H, $CH_2$), 0.78 - 0.65 (m, 2H, $CH_2$), 0.48 - 0.30 (m, 2H, $CH_2$). $^{13}$C NMR (101 MHz, $CDCl_3$) $\delta$ 159.2, 133.6, 127.7, 117.3, 112.3, 59.2, 57.8, 55.4, 55.3, 51.5, 37.9, 29.6, 26.6, 23.4, 13.7. ESI-MS, positive mode: *m*/*z* (rel. int., %) = 308 (100) [*M*+H]$^+$. HRMS *m*/*z* calcd for $C_{17}H_{26}NO_2S$, 308.1679 [M + H]$^+$; found, 308.1679.

**2. Evaluation of the functional potency of the multifunctional agents for treatment of cardiovascular diseases**

Cell culture

HEK-293 cells stably expressing R-CEPIA1*er* and WT RyR2 (generous gift from Takashi Murayama, Juntendo University School of Medicine, Tokyo) were cultured in Dulbecco's Modified Eagle medium (DMEM, Thermo Fisher Scientific, Darmstadt, Germany) supplemented with 10% fetal bovine serum (FBS, Thermo Fisher Scientific, Darmstadt, Germany), 0.9% Penicillin/Streptomycin (Merck KGaA, Darmstadt, Germany), 15ug/mL Blasticidine (Invivogen, Toulouse, France), 100 μg/mL Hygromycin (Merck KGaA, Darmstadt, Germany) and 400 μg/mL G418 (Thermo Fisher Scientific, Darmstadt, Germany) in a humidified 5% COz incubator at 37 °C. To induce the expression of WT RyR2 in the endoplasmic reticulum (ER), 2 μg/mL Doxycycline (Merck KGaA, Darmstadt, Germany) was added 24 h before experiments.

HL-1 cardiac cells (SCC065, Merck KGaA, Darmstadt, Germany) were cultivated in Claycomb's Medium (51800C, Merck KGaA, Darmstadt, Germany), supplemented with Glutamax, 10% fetal bovine serum, 0.9% Penicillin/-Streptomycin and 0.1 mM Noradrenaline (Merck KGaA, Darmstadt, Germany) on fibronectin/gelatin (Merck KGaA, Darmstadt, Germany) coated culture bottles or plates two days before plating.

Fluorescence Microscopy

Confocal and STED images were acquired on a Abberior STED 775 QUAD scanning microscope (Abberior

Instruments GmbH) equipped with 488 nm, 561 nm and 640 nm 40 MHz pulsed excitation lasers, a pulsed 775 nm STED 40 MHz laser, and UPlanSApo 100x/1.40 Oil objective. Pixel size was 30 - 40 nm for all STED images acquired on this setup.

Time-lapse $[Ca^{2+}]_{ER}$ assay

The fluorescent plate reader $[Ca^{2+}]_{ER}$ assay was performed according to the slightly modified protocol of Murayama & Kurebayashi [T. Murayama, N. Kurebayashi, Assays for Modulators of Ryanodine Receptor (RyR)/Ca(2+) Release Channel Activity for Drug Discovery for Skeletal Muscle and Heart Diseases. Curr. Protoc. Pharmacol. 2019, 87, e71.]. HEK-293 WT RyR2 R-CEPIA1*er* cells (50000 cells per well) were cultivated for 24 h in black-walled, clear-bottom 96-well micro-plates (Corning, Amsterdam, The Netherlands) covered with fibronectin (10 μg/mL, Roche, Mannheim, Germany) in a humidified incubator at 37 °C and 5% $CO_2$. After the cells were inducted with Doxycycline for 24 h, changes in ER calcium concentrations $[Ca^{2+}]_{ER}$ were measured on a multiwell plate reader TECAN Spark 20M at 37 °C. The 1000 × stock solutions of the test compounds were prepared in DMSO. Briefly, the culture medium was removed and 100 μl of Tyrode's solution containing 2 mM $CaCl_2$ was added to the cells. The time courses were recorded in each well. Fluorescence was evoked by 560 nm excitation wavelength and collected in a bottom-read mode at 610 nm. Data was recorded every 10 s, exposure - 20 flashes, excitation bandwidth - 20 nm, emission bandwidth - 20 nm. 100 μl of 0 - 100 μM solution of test compound in Tyrode's solution containing 2 mM CaCh was injected in each well (speed 100 μl/s) at the 90 s time point. $F/F_0$, the ratio of the averaged fluorescence intensities between the initial (before injection compound $F_0$) and the last ($F$) 100 s of the read out were taken for the analysis. The 0.1 v/v% DMSO and 2 mM CaCh Tyrode's solution was used as a control.

The dose response curves were calculated using the software package GraphPad Prism version 8.3.1. (GraphPad Software, Inc.). All data are presented as mean ± S.D. in 3 independent experiments. Y=Bottom + (Top-Bottom)/(1+10^((Log$EC_{50}$-X)*HillSlope)) was used where HillSlope describes the steepness of the curve, Top and Bottom are plateaus in the units of the Y axis.

Caffeine assay on HL-1 cells

HL-1 cardiac cells (50000 per well) were cultivated for 24 h in black-walled, clear-bottom 96-well micro-plates (Corning, Amsterdam, The Netherlands) covered with fibronectin/gelatin (Merck KGaA, Darmstadt, Germany) in a humidified incubator at 37 °C and 5% $CO_2$.

Changes in $Ca^{2+}$ concentration were measured on a multiwell plate reader TECAN Spark 20M using FLIPR 6 Calcium Assay Kit from Molecular Devices (Molecular Devices LLC, München, Germany) at 37 °C. The 1000 × stock solutions of the test compounds were prepared in DMSO. 100 μl solution of test compound in Tyrode's solution containing FLIPR Calcium 6 dye was incubated for 2 hours at 37°C and 5% COz in a humidified incubator. The time courses were recorded in each well. Fluorescence was evoked at 485 nm excitation wavelength and read out in a bottom-read mode at 525 nm. Data were recorded every 2 s, exposure - 20 flashes, excitation bandwidth - 10 nm, emission bandwidth - 15 nm. To initiate $Ca^{2+}$ influx, 25 μl of 50 mM caffeine solution in Tyrode's solution containing 2 mM CaCl2 was injected in each well (speed 100 μl/s) at 10 s time point, resulting in 10 mM final concentration in the well. The differences between the caffeine induced peak minus basal fluorescence, $\Delta F_{Caff}$, were taken for the analysis. The Tyrode's solution + 0.1 v/v% DMSO was used as a control. The dose response curves were calculated using the software package GraphPad Prism version 8.3.1. All data are presented as mean ± S.D. from 8 independent experiments. Y=Bottom + (Top-Bottom)/(1+10^((Log$EC_{50}$-X) *HillSlope)) was used where HillSlope describes the steepness of the curve, Top and Bottom are plateaus in the units of the Y axis.

Isolation of Microsomal Membrane Vesicles from HEK-293 cells

Isolation of microsomal membrane vesicles from HEK-293T cells (HEK-293 ER) was performed according to Stewart et al.[61] HEK-293T cells were harvested with Trypsin/EDTA, washed twice with ice-cold PBS containing protease inhibitor cocktail (cOmplete™, Roche, Mannheim, Germany) and centrifuged 800 × g for 5 min. The pellet was resuspended in ice-cold lysis buffer (1 mM EDTA, 10 mM HEPES, cOmplete, pH 7.4) and incubated on ice for 20 minutes. After the cells were homogenized with a tight fitting Dounce homogenizer (10-20 strokes), an equal volume of restoration buffer (500 mM sucrose, 10 mM HEPES, cOmplete, pH 7.2) was added and the cells were homogenized again (10-20 strokes). The homogenate was centrifuged at 10000 × g for 20 min at 4° C. The supernatant was collected and centrifuged again for another 2 hours (100000 × g at 4° C). Then the pellet was carefully resuspended in resuspension buffer (250 mM sucrose, 10 mM HEPES, cOmplete, pH 7.2) on ice by pipetting the solution up and down until fully dissolved. The obtained solution was diluted to a protein concentration of 15-20 μg/μl with the resuspension buffer, the aliquots were snap frozen in liquid nitrogen and stored at - 80 °C until usage.

Isolation of Microsomal Membrane Vesicles from mouse hearts

All animal procedures were performed in accordance with Directive 2010/63/EU of the European Parliament and the Council on the protection of animals used in research, as well as Animal Welfare Law of the Federal Republic of Germany (Tierschutzgesetz der Bundesrepublik Deutschland, TierSchG). Sacrificing wild-type rodents for subsequent preparation of tissue did not require specific authorization or notification (§7 Abs. 2 Satz 3 TierSchG). All mice were housed with a 12 hours light/dark cycle with free access to food and water. Adult C57BL/6N mice of both genders were sedated with isoflurane in a sealed container and quickly euthanized by cervical dislocation. Heart ventricles were taken out instantly, washed with ice cold PBS plus complete, cut into smaller pieces and snap frozen in liquid nitrogen straight away.

Thawed on ice lysis buffer (10mM EDTA, 10mM HEPES, cOmplete, pH 7.4) was added and the ventricles were homogenized with a Qiagen cellruptor for 20 s. On ice an equal volume of restoration buffer (500 mM sucrose, 10 mM HEPES, cOmplete, pH7.2) was added and briefly mixed. The homogenate was centrifuged at 10000 × g for 20 min at 4° C. The supernatant was collected and centrifuged again for another hour (100000 × g at 4°C). Then the pellet was carefully resuspended in resuspension buffer (250 mM sucrose, 10 mM HEPES, cOmplete, pH 7.2) on ice by pipetting the solution up and down until fully dissolved. The obtained solution was diluted to a protein concentration of 5 μg/μl with the resuspension buffer and the aliquots were snap frozen in liquid nitrogen and then stored at -80 °C until usage.

SERCA2a activity measurements

To determine whether new compounds could increase the SERCA2a activity, HEK-293 microsomes (40 - 150 μg of total protein) or mouse ventricular microsomes (1 - 20 μg of total protein) were diluted in 100 μl of the assay buffer used for measurement of SERCA activity (described below) and incubated at room temperature for 5 minutes prior to the experiments. The measurements of SERCA2a activity were made at 28 °C by using a NADH -coupled ATPase assay. Briefly, the assay buffer contained 60 mM MOPS, 120 mM KCl, 6 mM $MgCl_2$, 1 mM EGTA, 5 mM $NaN_3$, 0.5 mM phosphoenolpyruvate and 1.5 mM $CaCl_2$, pH 7.0. Before the reaction was started by the injection of 5 mL ATP (resulting in 2 mM final concentration in the well), 5 units/mL of both lactate dehydrogenase and pyruvate kinase, 0.5 mM NADH, and 1 μM $Ca^{2+}$ ionophore A-23187 (Sigma, C-7522) were added to the diluted sample with or without the tested compound. The 1.5 mM Ca/EGTA buffer has a free $Ca^{2+}$ concentration of about 200 μM. The NADH fluorescence decrease was recorded each 2 s for 10 min course on a multiwell plate reader TECAN Spark 20M in 96-well glass bottom plates (MatTek Corporation; Cat. No. PBK96G-1.5-5-F). Fluorescence was evoked by 380 nm excitation wavelength and collected in a bottom-read mode at 425 nm. Exposure - 30 flashes, excitation bandwidth - 10 nm, emission bandwidth - 10 nm. ATP was injected at 30 s. The amount of added protein, 7-13 μg/mL for mouse SR and 133-270 μg/mL for HEK ER, did not affect the slope of the fluorescence decrease in control samples (0.1% DMSO). The data were analyzed with computer software GraphPad Prism version 8.3.1 (GraphPad Software, Inc.). The difference in signal decrease with and without microsomes was taken as an indicator for the additional ATPase activity performed by SERCA2a. The obtained values for maximal SERCA2a activity were normalized for total protein concentration. Maximal SERCA2a activity values are reported as percentages of control (0.1% DMSO) values. Microsomal samples that were kept on ice until usage and set to 100%.

$Ca^{2+}$ Imaging and analysis of $Ca^{2+}$ sparks and $Ca^{2+}$ transients

Cardiomyocytes were imaged using an LSM 880 inverted microscope equipped with a 63x oil immersion objective (Zeiss, Germany). Fluo-4 was excited at 488nm with a krypton/argon laser. The fluorescent emission was collected through a long-pass filter (>515 nm). All images were acquired digitally in line-scan mode (1.85 or 2.5 ms per line-scan; pixel size 0.1 μm) with 6000 lines/image. The scan line was placed in parallel to the longitudinal axis of brick-shaped cardiomyocytes with clear cross striations that were solid attached to the cover slip. Images were recorded only from Fluo-4 loaded permeabilized cells that show spontaneous contractions and corresponding Ca2+ waves at frequencies below 4 waves/10s. These cells were attributed to have experienced a mild permeabilization procedure, i.e. the cell membrane was sufficiently permeabilized to allow the membrane-impermeable Ca2+ indicator to enter the cell but the intracellular membranes and contractile structures were not affected evidenced by Ca2+ release from intracellular stores and cell shortening, respectively. All measurements were performed under control conditions and after 2 min incubation with relaxing solution containing either vehicle (DMSO, 0.1%) or the indicated drug concentration. Drugs were added cumulatively if appropriate. Intact cardiomyocytes were loaded with Fluo-4 AM (5 μM, Thermo Fisher Scientific) for 45 min at room temperature and imaged as described above. Electrical field stimulation was performed as described.

Images were analyzed using ImageJ (http://rsb.info.nih.gov/nih-image) equipped with the SparkMaster plugin. The detection criteria for Ca2+ sparks were set at 3.8, i.e. the threshold for the detection of events was 3.8 times the standard deviation of the background noise divided by the mean. Ca2+ spark amplitude was normalized as F/F0 (with F0 being the initial fluorescence recorded under steady-state conditions and ΔF = F - F0), duration was expressed as full duration or taken from the full-duration half-maximum (FDHM), and width was expressed as full width or taken from full-width half-maximum (FWHM).

Ca2+ spark shape (i.e. FWHW and FDHM) is reported to be different between RyR+/+ and RyRR2474S/+ channel clusters, especially with the observation of ember sparks in cardiomyocytes from mice expressing the RyRR2474S/+ mutation. Spark shape is related to intracellular Ca2+ buffer capacity, i.e using EGTA at low concentration increases the width and length of calcium sparks compared to using EGTA at high concentration. Likewise, the use of the faster calcium chelator BAPTA reduces the width and length of Ca2+ sparks compared to EGTA. To compare the effects of the drugs tested more accurately, we used the fast calcium chelator BAPTA at 0.1 mM giving a free [Ca2+] of 80 nM. In this condition, the frequency of Ca2+ sparks and FWHM was not different in permeabilized cardiomyocytes from RyR2+/+ and RyRR2474S/+ mice, whereas FDHM was slightly increased in cardiomyocytes from RyRR2474S/+ mice. However, analysis of drug effects should not be hampered by the differences in FDHM.

Spontaneous Ca2+ waves were analyzed by calculating the wave speed and the Ca2+ wave decay reflecting Ca2+ uptake by SERCA2a activity. Ca2+ wave speed was obtained by a linear approximation of wave propagation (in μm/ms). Ca2+ decay was obtained in a 1-2μm part of the wave (10-20 pixels in width) before and after drug application to exclude the influence of wave propagation but to reduce the noise in the recording. Due to permeabilization procedure, activity of sodium-calcium exchanger (NCX) was not expected to contaminate our signals. The obtained Ca2+ decay was fitted by a mono-exponential function from 50% of the peak maximum to baseline resulting in the time constant tau as an indicator of decay velocity with $r^2 > 0.9$.

Statistical analysis

Normality and log normality tests were performed using the Shapiro-Wilk test. The significance between means were tested using unpaired Student's t-test and one-way ANOVA. The Dunnett test was performed to compare every mean to a control (0.1% DMSO) mean. Results are represented as mean ± S.D. A value of $P < 0.05$ was considered significant.

Western Blot Analysis

SERCA2a protein levels in microsomal membrane vesicles (HEK-293T cells and mouse hearts, 8-11-week-old mice, n = 10 males and 2 females, C57Bl/6N genotype) were determined by Western blot analysis. 20 μl of microsomes were mixed with 2 μl NuPAGE LDS Sample buffer (Thermo Fisher) and heated at 70°C for 10 minutes. The samples (100-150 μg protein) were loaded onto a 3 - 8% gradient Tris-acetate-gel or 4-20% SDS- Tris-glycine gel and the gel was run for 1 h at 150 V in running buffer. After that the gel was equilibrated for 10 min in 20% EtOH, transferred onto a nitrocellulose membrane and blotted with the iBlot 2 system (dry blot) for 10 minutes at 25 V. Membranes were blocked with 5% skim milk for 30 min at room temperature and incubated with primary antibody against SERCA2a (Badrilla A010-20 rabbit polyclonal, 1:1000) overnight at 4 °C with moderate shaking. Binding of the primary antibody was detected by Horseradish peroxidase (HRP)-conjugated secondary antibody (1 h, rt). The detection was done with the Western Lightning Plus ECL Kit (Perkin Elmer LAS GmbH, Germany, Rodgau) and the Amersham Imager 600.

Cytotoxicity

Cell viability was assessed using CytoTox-Glo™ Cytotoxicity Assay according to the manufacturing procedure (Promega GmbH). Briefly, HL-1 cardiac cells were cultivated for 24 h in black-walled, clear-bottom 96-well micro-plates (Corning) covered with fibronectin in DMEM, containing different concentrations of the tested compound (10,000 cells/well). Luminescence of a luminogenic peptide substrate was measured before and after addition of the lysis reagent (digitonin). The viable cell contribution was determined by a subtractive method. 0.1% DMSO treated cell were taken as a control.

Membrane permeability

Membrane permeability was assessed using PermeaPad® Plate according to the manufacturing procedure (innoME GmbH, Espelkamp, Germany). Briefly, 200 μl water was added into acceptor plate. 200 μl of 100 - 500 μM test compound in water was added directly to the well membranes of the donor plate. The donor plate contains a biomimetic membrane for simulating passive mass transfer through different barriers in the body. Then the donor plate was placed into the acceptor plate wells and incubated at room temperature for 24 hours in dark. To determine peak absorbance of test compounds, absorbance spectra of acceptor solutions and initial solutions for each test compound were read out. The Permeability Rate ($P_e$) was calculated using the formula:

$$P_e = C\times\text{-ln}(1\text{-}\frac{OD_A}{OD_E})\ \text{cm/s},$$

where $OD_A$ is the absorbance of acceptor solution, $OD_E$ is the absorbance of the standard solution; if the compound is able to permeabilize the membrane and fully reach equilibrium, 250 μM will be the final concentration of solution in the donor and acceptor wells. The coefficient C was calculated using the formula:

$$C = \frac{V_D \times V_A}{(V_D + V_A)\times Area \times time},$$

where $V_A$ - acceptor volume ($cm^3$), $V_D$ - donor volume ($cm^3$), membrane area 0.24 $cm^2$, time is 86,400 s.

2.1 Characterization of target compounds and RyR2 activity

New cyclopropanol compounds modulate the RyR2 channel activity. The inventors measured changes in $Ca^{2+}$ levels in the endoplasmic reticulum (ER) of HEK-293 cells with inducible expression of wild-type RyR2 (WT RyR2) [T. Murayama, N. Kurebayashi, M. Ishigami-Yuasa, S. Mori, Y. Suzuki, R. Akima, H. Ogawa, J. Suzuki, K. Kanemaru, H. Oyamada, Y. Kiuchi, M. Iino, H. Kagechika, T. Sakurai, Efficient High-Throughput Screening by Endoplasmic Reticulum Ca(2+) Measurement to Identify Inhibitors of Ryanodine Receptor Ca(2+)-Release Channels. Mol. Pharmacol. 2018, 94, 722 - 730]. The cells additionally stably express the $Ca^{2+}$ fluorescence indicator R-CEPIA1*er*, which is a genetically encoded sensor protein of $Ca^{2+}$ in the ER ($[Ca^{2+}]_{ER}$) [T. Murayama, N. Kurebayashi, Assays for Modulators of Ryanodine Receptor (RyR)/Ca(2+) Release Channel Activity for Drug Discovery for Skeletal Muscle and Heart Diseases. Curr. Protoc. Pharmacol. 2019, 87, e71].

The fluorescence ratio $F/F_0$ was calculated, where $F_0$ - is the average fluorescence for the first 90 s, $F$ - is the average fluorescence for the last 100 s, and normalized. The results are depicted in Figure 5 and Figure 6.

The addition of 25 μM dantrolene (Dant), S36 or 1 mM S107 resulted in an increase in R-CEPIA1*er* fluorescence (Figure 5). ARM210, which belongs to the second generation of Rycals, significantly increased $[Ca^{2+}]_{ER}$, proving its stabilizing activity in HEK-293 RyR2 R-CEP1*er* cells (Figure 5).

Compounds **13** and **14**, with the exception of **13d**, significantly increased $[Ca^{2+}]_{ER}$, in the RyR2 R-CEPIA1*er* assay (Figure 5). Compounds **13a-c** and **14** were tested in a concentration range from $10^{-11}$ to $10^{-4}$ M. At concentrations above 25 μM, compound **14a** demonstrated the most pronounced effect of all other compounds. Compounds **13b,c** and **14c** were less efficacious than **13a,** but showed lower values of half-maximal effective concentration (81.5 nM, 10.2 nM and 18.6 nM respectively) (Figure 6 and Table 1).

Compound **14a** (GM1869) mediated RyR2 $Ca^{2+}$ spark (a microscopic subcellular spontaneous form of intracellular $Ca^{2+}$ signal) activity in permeabilized, isolated adult mouse cardiomyocytes (CM, wild-type and $RyR2^{R2474S/+}$ mutant, Figure 7) and modulated the electrically-evoked $Ca^{2+}$ transients in human iPSC-CMs (Human Induced Pluripotent Stem Cell-Derived Cardiomyocytes, Figure 8).

2.2 SERCA2a activity in cardiac HL-1 cells

The SERCA2a activity of the compounds was confirmed in two orthogonal biochemical assays: caffeine-induced $Ca^{2+}$ release assay and nicotinamide adenine dinucleotide (NADH) assay on microsomal membrane vesicles from mouse heart ventricles (mouse SER) and HEK-293T cells (HEK-293 ER). The effect of caffeine-induced $Ca^{2+}$ release in HL-1 cells, immortalized mouse cardiomyocytes, treated with the cytosolic $Ca^{2+}$ indicator FLIPR Calcium 6 (Molecular Devices) was investigated.

When caffeine binds to the RyR channel, it interacts with specific amino acids of the protein, and this interaction causes conformational changes in the protein's structure [A. des Georges, O. B. Clarke, R. Zalk, Q. Yuan, K. J. Condon, R. A. Grassucci, W. A. Hendrickson, A. R. Marks, J. Frank, Structural Basis for Gating and Activation of RyR1. Cell 2016, 167, 145 - 157.e17]. This opens the RyR2 channel pore, enabling the mass release of calcium ions from the SER $Ca^{2+}$ store. The addition of 10 mM caffeine solution to HL-1 cells incubated for 2 h in solutions of the tested compounds (10 μM, 25 μM for **13d**) together with cytoplasmic $Ca^{2+}$ indicator showed a clear dependence of the magnitude of the caffeine effect from the concentration of tested compounds (Figure 9).

Compounds **13**, **14** showed improved SERCA2a activity in the micromolar concentration range, with **13d** being least efficacious and **14c** being most efficacious of all other compounds including CDN1163, known SERCA2 activator (Figure 9 and Figure 10).

Compound **13d** showed no saturation effect at concentrations up to 50 μM, which did not allow to calculate its $EC_{50}$ (Figure 10).

The $EC_{50}$ values for **13a-c** and **14a,c** were found 2.2 μM, 8.6 μM, 7.6 μM, 9.2 μM and 10.9 μM correspondingly (Table 1).

Compound **14b** displayed a bell-shaped dose response; it has shown a moderate to good stimulatory effect at 3 μM and 10 μM (up to 150% compared to the control) that decreased at higher doses, suggesting that the compound may exhibit inhibitory activity at higher concentrations (Figure 10).

S36 has shown no concentration dependent effect in caffeine-induced $Ca^{2+}$ release assay (Figure 11). This suggests that S36 does not increase SERCA2a activity. Addition of caffeine to HL-1 cells treated with different concentrations of S107 didn't show any effects on SR $Ca^{2+}$ content as well (Figure 11). Surprisingly, the second-generation Rycal ARM210 drug demonstrated a clear concentration-dependent effect in this assay (Figure 12). CDN1163 was used as a positive control.

2.3 SERCA2a activity in microsomal membrane vesicles

In an NADH assay, the change in NADH coenzyme fluorescence reflects the level of the consumption of ATP, which can be taken as an indicator of SERCA2 activity [G. B. Warren, P. A. Toon, N. J. Birdsall, A. G. Lee, J. C. Metcalfe, Reconstitution of a calcium pump using defined membrane components. Proc. Natl. Acad. Sci. U.S.A. 1974, 71, 622 - 626]. ATP hydrolysis by SERCA2 is required to pump calcium ions into the SER. A higher rate of ATP hydrolysis corresponds to enhanced SERCA2 ATPase activity that leads to a greater pumping of calcium ions into the SR.

The rate of NADH oxidation, which is coupled to ATP hydrolysis, was observed by measuring changes in NADH intrinsic fluorescence over time after ATP injection into microsomal membranes enriched in SER vesicles isolated from mouse heart ventricles (mouse SER) and ER vesicles from HEK-293T cells (HEK-293 ER). The presence of SERCA2 in mouse SER and HEK-293 ER of isolated microsomal vesicles was confirmed by Western blotting. The NADH fluorescence decrease was recorded on a multiwell plate reader TECAN Spark 20M ($\lambda_{ex}$ = 380 nm, $\lambda_{em}$ = 470 nm). Figure 13a shows a typical decrease in NADH fluorescence after the injection of 2 mM ATP using mouse heart SR vesicles pre-treated with compound **14a.** Consistent with the previous experiment on cardiac HL-1 cells, a strong decrease in NADH fluorescence reflecting an increase in SERCA2a activity in SER microsomes derived from mouse heart ventricles (n = 3 - 5) in solutions containing the tested compounds compared to 0.1 v/v% DMSO sample was observed (Figure 13). In microsomes from HEK-293 ER, the treatment with compounds **13a** and **14b** did not evoke significant increase in ATPase activity, while other compounds, **13c,d**, **14a-c** and ARM210, showed significant activation of SERCA2 ATPase (n = 3 - 5) (Figure 14). In cardiac SER vesicles from mouse heart all new cyclopropanol compounds improve the ATPase activity (Table 1, Figure 13). For compound **14a,** ATPase activity increased in a concentration-dependent manner with an $EC_{50}$ of 16 nM and 383 nM for HEK-293 ER and mouse SER, respectively (Table 1, Figure 13). In both experiments, CDN1163 was used as a positive control.

The SERCA2a activity of compound **14a** (GM1869) was proved by analysing the SERCA2a-mediated $Ca^{2+}$ wave decay in permeabilized mouse CMs, electrically-evoked $Ca^{2+}$ transients in intact CMs and in human iPSC-CMs (Figure 7,8).

2.4 Drug-related cell toxicity and membrane permeability

The cyclopropanol-substituted compounds showed no significant cell death at concentrations up to 50 μM in CytoTox-Glo™ Cytotoxicity Assay (Promega GmbH, Figure 15). The membrane permeability of the derivatives was determined using cell-free transport models (PermeaPad®, innoME GmbH), and permeability rates ($P_e$), which are a measure of how fast a compound can cross a membrane, were established (Table 1).

Albeit poor permeability shown in PAMPA assay, compounds S36, **13a** and **14a** acted on live RyR2-HEK-293 R-CEPIA1*er* cells, and the latter two had an effect on cardiac HL-1 cells in caffeine-induced $Ca^{2+}$ release assay.

The properties of the new compounds together with the properties of the known substances S36, S107 and ARM210 are given in Table 1. The values of calculated partition coefficients (log P) for cyclopropanol derivatives range from 2.2 to 4.4, which is in line with Lipinski's "rule of five" claiming that the calculated octanol-water partition coefficient should not exceed 5.

2.5 Discussion and conclusions

Cyclopropanols, except for **13d**, which is a 1,4-benzothiazepine derivative with the shortest $C_4H_8$ linker, showed dual RyR2/ SERCA2a activity in the above tests. 1,4-Benzothiazepine **13d**, in our initial tests, had no significant RyR2-stabilizing effect on HEK-293 RyR2 R-CEPIAer cells but demonstrated slight (HL-1 and HEK ER) and moderate (mouse SR) SERCA2 stimulating activity.

Although compound **13d** (k=4) did not meet the threshold on R-CEPIA1*er*-assay, the inventors expect that this compound will be active in cardiac cells and ryanodine receptor, in individual level therapy. This activity should further

increase when using a longer linker in formula (1) (wherein $R_5$ and optionally $R_6$ are selected from formulae (3), (6) and (9), which contain a linker with length "k").

The RyR2 tests clearly point out that derivatives of 3-[(4-methoxyphenyl)oxy]- and 3-[(4-methoxyphenylthio)] propane-1-amine, which are the mimetic analogues of 1,4-benzothiazepine, can activate RyR2 almost to the same extent as known Rycals. Compound **13a**, which is 1,4-benzothiazepine derivative with one cyclopropanol group and $C_4H_8NHC_4H_8$ linker, was most potent in the caffeine-induced $Ca^{2+}$ release assay in HL-1 cells. Although we could not detect SERCA2 activation by this compound (10 μM) on ER vesicles derived from HEK-293T cells, but we observed a significant effect on mouse heart microsomes (Figure 13).

Under certain experimental conditions, compounds **13b,c** and **14** demonstrated SERCA activities comparable or superior to that of known SERCA activator CDN1163. Compound **13c,** which is the 3-[(4-methoxyphenylthio)]propane-1-amine derivative with one cyclopropanol group, demonstrated nanomolar $EC_{50}$ in RyR2 assay. The 1,4-benzothiazepine analog **14a,** which contains two cyclopropanol groups and $C_4H_8NH(C_4H_8)_2$ linker, demonstrated RyR2 stabilizing activity in micromolar concentrations and a nanomolar $EC_{50}$ on HEK-293 microsomes and mouse SER in NADH assay. Of the other two compounds, 3-[(4-methoxyphenyl)oxy]- and 3-[(4-methoxyphenylthio)]propane-1-amine derivatives with the same $NH(C_4H_8)_2$ linker and two cyclopropanol groups (**14b** and **14c**), compound **14c** had a stronger activating effect on caffeine-induced $Ca^{2+}$ release at concentrations above 10 μM, whereas **14b** showed a slight increase in SERCA2a-stimulating activity up to 10 μM and a presumable inhibitory effect at higher concentrations, 25 and 50 μM (Figure 10).

The 1,4-benzothiazepine derivative ARM210, despite its known RyR2-modulating activity, was found to act as a SERCA2a activator.

The new cyclopropanol containing 1,4-benzothiazepines and their structural analogues act on RyR2 channels stabilizing the closed state while they activate SERCA2a, increasing SER $Ca^{2+}$ load. Relatively small changes in the structure of the new compounds lead to differences in their biochemical properties with respect to RyR2 and SERCA2a activity. Both proteins, RyR2 and SERCA2a, are the key players in cardiac intracellular $Ca^{2+}$ cycling e.g. the main source and the main sink, respectively, apart from the $Ca^{2+}$ binding protein Troponin C in the contractile myofilaments. Therefore, the ability to obtain drugs that can be flexibly adjusted to each individual $Ca^{2+}$ transporter defects (for example with stronger RyR2 stabilization and moderate SERCA2a activation, or vice versa) could be used as to achieve dual-acting tools ultimately for personalized treatment, improving the contractility of the failing heart muscle to prevent or slow heart failure and to prevent $Ca^{2+}$ triggered heart rhythm disorders on the individual level.

**Table 1:** Properties of 1,4-benzothiazepines with cyclopropanol groups and their structural analogues. S36, S107 and ARM210 are the known inhibitors of RyR-$Ca^{2+}$ leak.

| Compound | MW | Log $P^e$ | $P_e$ $10^{-6}$ cm/s | Viability$^b$, % | RyR2 ($EC_{50}$, nM) | SERCA2a$^c$ | | |
|---|---|---|---|---|---|---|---|---|
| | | | | | | HL-1 ($EC_{50}$, μM) | HEK ER ($EC_{50}$, nM) | mouse SR ($EC_{50}$, nM) |
| S36 | 267 | 0.9 ± 0.7 | 1.0 (low) | 94 | +++ | - | n.d. | n.d. |
| S107 | 209 | 0.8 ± 0.7 | 1.2 (medium ) | 98 | +++ | - | n.d. | n.d. |
| ARM21 0 | 329 | 3.8 ± 0.6 | 6.8 (high) | 90 | ++ | + (34.3; 13.1 - 157.1) | + | ++ |
| 13a | 378 | 3.3 ± 0.5 | 0.8 (low) | 84 | ++ (8170; 1221 - 48060) | + (2.2; 1.0-5.1) | - | + |
| 13b | 293 | 2.2 ± 0.3 | 4.0 (high) | 100 | +++ (81.5; 28.9 - 241.4) | ++ (8.6; 4.0 - 20.1) | - | + |
| 13c | 309 | 3.3 ± 0.5 | 6.9 (high) | 73 | ++ (10.2; 0.8-60.3) | ++ (7.6; 4.2-14.4) | + | +++ |
| 13d | 307 | 3.1 ± 0.5 | 3.6 (high) | 95 | - | + (> 1000) | + | ++ |
| 14a | 490 | 4.3 ± 0.6 | 0.6 (low) | 80 | +++ (2700; 1160 - 6570) | ++ (9.2; 4.1 - 22.8) | ++ (16; 6 - 39) | +++ (383; 26 - 985) |
| 14b | 405 | 3.0 ± 0.4 | 4.0 (high) | 100 | + (> 23000) | ++ (1.5; 0.6 - 4.4) | + | + |

(continued)

| Compound | MW | Log $P^e$ | $P_e$ $10^{-6}$ cm/s | Viability[b], % | RyR2 ($EC_{50}$, nM) | SERCA2a[c] | | |
|---|---|---|---|---|---|---|---|---|
| | | | | | | HL-1 ($EC_{50}$, μM) | HEK ER ($EC_{50}$, nM) | mouse SR ($EC_{50}$, nM) |
| 14c | 421 | 4.4 ± 0.5 | 3.6 (high) | 90 | + (18.6; 1.0-101.6) | +++ (10.9; 5.27 - 24.3) | + | + |

[a]Partition coefficients were evaluated using ACD/Labs 2021.1.1; CytoTox-Glo assay in 100 μM solutions after 24 h incubation; [c]"+++" a strong effect, comparable to or greater than that of a known compounds at a given concentration (25 μM in RyR2 R-CEPIA1*er* assay, 10 μM in caffeine assay and on microsomal membrane vesicles), "++" moderate effect, "+" observable effect,"-" no effect.

List of citations

1. A. R. Marks, Targeting ryanodine receptors to treat human diseases. J. Clin. Investig. 2023, 133, e162891.
2. D. M. Bers, Cardiac excitation-contraction coupling. Nature 2002, 415, 198 - 205.
3. S. O. Marx, A. R. Marks, Dysfunctional ryanodine receptors in the heart: new insights into complex cardiovascular diseases. J. Mol. Cell. Cardiol. 2013, 58, 225 - 231.
4. D. M. Bers, Cardiac sarcoplasmic reticulum calcium leak: basis and roles in cardiac dysfunction. Annu. Rev. Physiol. 2014, 76, 107-127.
5. H. Uchinoumi, Y. Yang, T. Oda, N. Li, K. M. Alsina, J. L. Puglisi, Y. Chen-Izu, R. L. Cornea, X. H. T. Wehrens, D. M. Bers, CaMKII-dependent phosphorylation of RyR2 promotes targetable pathological RyR2 conformational shift. J. Mol. Cell. Cardiol. 2016, 98, 62 - 72.
6. X. H. T. Wehrens, S. E. Lehnart, A. R. Marks, Ryanodine Receptor-Targeted Anti-Arrhythmic Therapy. Ann. N. Y. Acad. Sci. 2005, 1047, 366 - 375.
7. X. H. T. Wehrens, S. E. Lehnart, F. Huang, J. A. Vest, S. R. Reiken, P. J. Mohler, J. Sun, S. Guatimosim, L.-S. Song, N. Rosemblit, J. M. D'Armiento, C. Napolitano, M. Memmi, S. G. Priori, W. J. Lederer, A. R. Marks, FKBP12.6 Deficiency and Defective Calcium Release Channel (Ryanodine Receptor) Function Linked to Exercise-Induced Sudden Cardiac Death. Cell 2003, 113, 829 - 840.
8. J. Balderas-Villalobos, T. Molina-Muñoz, P. Mailloux-Salinas, G. Bravo, K. Carvajal, N. L. Gómez-Viquez, Oxidative stress in cardiomyocytes contributes to decreased SERCA2a activity in rats with metabolic syndrome. Am. J. Physiol. Heart Circ. 2013, 305, H1344 - H1353.
9. S. E. Lehnart, X. H. T. Wehrens, S. Reiken, S. Warrier, A. E. Belevych, R. D. Harvey, W. Richter, S. L. C. Jin, M. Conti, A. R. Marks, Phosphodiesterase 4D Deficiency in the Ryanodine-Receptor Complex Promotes Heart Failure and Arrhythmias. Cell 2005, 123, 25 - 35.
10. A. R. Marks, D. W. Landry, S. Deng, C. Z. Zhuang, S. E. Lehnart, Agents for preventing and treating disorders involving modulation of the RyR receptors. US7879840B2, February 1, 2011.
11. M. Jessup, B. Greenberg, D. Mancini, T. Cappola, D. F. Pauly, B. Jaski, A. Yaroshinsky, K. M. Zsebo, H. Dittrich, R. J. Hajjar, Calcium Upregulation by Percutaneous Administration of Gene Therapy in Cardiac Disease (CUPID). Circulation 2011, 124, 304 - 313.
12. M. Kaneko, H. Yamamoto, H. Sakai, Y. Kamada, T. Tanaka, S. Fujiwara, S. Yamamoto, H. Takahagi, H. Igawa, S. Kasai, M. Noda, M. Inui, T. Nishimoto, A pyridone derivative activates SERCA2a by attenuating the inhibitory effect of phospholamban. Eur. J. Pharmacol. 2017, 814, 1 - 8.
13. R. L. Cornea, S. J. Gruber, E. L. Lockamy, J. M. Muretta, D. Jin, J. Chen, R. Dahl, T. Bartfai, K. M. Zsebo, G. D. Gillispie, D. D. Thomas, High-Throughput FRET Assay Yields Allosteric SERCA Activators. J. Biomol. Screen. 2012, 18, 97 - 107.
14. A. Luraghi, M. Ferrandi, P. Barassi, M. Arici, S.-C. Hsu, E. Torre, C. Ronchi, A. Romerio, G.-J. Chang, P. Ferrari, G. Bianchi, A. Zaza, M. Rocchetti, F. Peri, Highly Selective SERCA2a Activators: Preclinical Development of a Congeneric Group of First-in-Class Drug Leads against Heart Failure. J. Med. Chem. 2022, 65, 7324 - 7333.
15. R. Nikolaienko, E. Bovo, S. L. Yuen, L. M. Treinen, K. Berg, C. C. Aldrich, D. D. Thomas, R. L. Cornea, A. V. Zima, New N-aryl-N-alkyl-thiophene-2-carboxamide compound enhances intracellular Ca2+ dynamics by increasing SERCA2a Ca2+ pumping. Biophysical Journal 2023, 122, 386 - 396.
16. R. Ueoka, M. Bortfeld-Miller, B. I. Morinaka, J. A. Vorholt, J. Piel, Toblerols: Cyclopropanol-Containing Polyketide

Modulators of Antibiosis in Methylobacteria. Angew. Chem. Int. Ed. 2018, 57, 977 - 981.
17. C. J. Suckling, The Cyclopropyl Group in Studies of Enzyme Mechanism and Inhibition. Angew. Chem. Int. Ed. 1988, 27, 537 - 552.
18. A. Esposito, P. P. Piras, D. Ramazzotti, M. Taddei, First Stereocontrolled Synthesis of (S)-Cleonin and Related Cyclopropyl-Substituted Amino Acids. Org. Lett. 2001, 3, 3273 - 3275.
19. X. Cai, F. He, J. Liang, B. Qu, W. Tao, J. Xu, L. Zhang, Y. Zhang, Ligand-drug conjugate of exatecan analogue, preparation method therefor and application thereof. WO2020063676A1, April 2, 2020.
20. O. G. Kulinkovich, The Chemistry of Cyclopropanols. Chem. Rev. 2003, 103, 2597 - 2632.
21. O. G. Kulinkovich, D. A. Astashko, V. I. Tyvorskii, N. A. Ilyina, Synthesis of $\alpha,\beta$-Epoxy Ketones from Alkyl- and Arylsubstituted Cyclopropanols. Synthesis 2001, 2001, 1453 - 1455.
22. G. Santulli, G. Pagano, C. Sardu, W. Xie, S. Reiken, S. L. D'Ascia, M. Cannone, N. Marziliano, B. Trimarco, T. A. Guise, A. Lacampagne, A. R. Marks, Calcium release channel RyR2 regulates insulin release and glucose homeostasis. J Clin Invest. 2015, 125, 1968 - 1978.
23. H. Dridi, Y. Liu, S. Reiken, X. Liu, E. K. Argyrousi, Q. Yuan, M. C. Miotto, L. Sittenfeld, A. Meddar, R. K. Soni, O. Arancio, A. Lacampagne, A. R. Marks, Heart failure-induced cognitive dysfunction is mediated by intracellular Ca2+ leak through ryanodine receptor type 2. Nat Neurosci. 2023, 26, 1365 - 1378.
24. R. T. Martuscello, M. L. Chen, S. Reiken, L. R. Sittenfeld, D. S. Ruff, C. L. Ni, C. C. Lin, M. K. Pan, E. D. Louis, A. R. Marks, S. H. Kuo, P. L. Faust, Defective cerebellar ryanodine receptor type 1 and endoplasmic reticulum calcium 'leak' in tremor pathophysiology. Acta Neuropathol. 2023, 146, 301 - 318.
25. A. M. Bellinger, S. Reiken, M. Dura, P. W. Murphy, S. X. Deng, D. W. Landry, D. Nieman, S. E. Lehnart, M. Samaru, A. LaCampagne, A. R. Marks, Remodeling of ryanodine receptor complex causes "leaky" channels: a molecular mechanism for decreased exercise capacity. Proc. Natl. Acad. Sci. U SA. 2008,105, 2198 - 2202.
26. S. E. Lehnart, M. Mongillo, A. Bellinger, N. Lindegger, B. X. Chen, W. Hsueh, S. Reiken, A. Wronska, L. J. Drew, C. W. Ward, W. J. Lederer, R. S. Kass, G. Morley, A. R. Marks, Leaky Ca2+ release channel/ryanodine receptor 2 causes seizures and sudden cardiac death in mice. J. Clin. Investig. 2008, 118, 2230 - 2245.
27. M. Yano, S. Kobayashi, M. Kohno, M. Doi, T. Tokuhisa, S. Okuda, M. Suetsugu, T. Hisaoka, M. Obayashi, T. Ohkusa, M. Kohno, M. Matsuzaki, FKBP12.6-mediated stabilization of calcium-release channel (ryanodine receptor) as a novel therapeutic strategy against heart failure. Circulation 2003,107, 477 - 484.
28. M. J. Wleklinski, P. J. Kannankeril, B. C. Knollmann, Molecular and tissue mechanisms of catecholaminergic polymorphic ventricular tachycardia. J. Physiol.-London 2020, 598, 2817 - 2834.
29. M. Ruiz-Meana, M. Minguet, D. Bou-Teen, E. Miro-Casas, C. Castans, J. Castellano, E. Bonzon-Kulichenko, A. Igual, R. Rodriguez-Lecoq, J. Vazquez, D. Garcia-Dorado, Ryanodine Receptor Glycation Favors Mitochondrial Damage in the Senescent Heart. Circulation 2019, 139, 949 - 964.
30. K. Kamei, N. Maeda, R. Ogino, M. Koyama, M. Nakajima, T. Tasuoka, T. Ohno, T. Inoue, New 5-HT1A receptor agonists possessing 1,4-Benzoxazepine scaffold exhibit highly potent anti-ischemic effects. Bioorg. Med. Chem. Lett. 2001, 11, 595-598.
31. D. Szalóki Vargáné, L. Tóth, B. Buglyó, A. Kiss-Szikszai, A. Mándi, P. Mátyus, S. Antus, Y. Chen, D. Li, L. Tao, et al. [1,5]-Hydride Shift-Cyclization versus C(sp2)-H Functionalization in the Knoevenagel-Cyclization Domino Reactions of 1,4- and 1,5-Benzoxazepines. Molecules 2020, 25, 1265.
32. A. K. Singh, V. Raj, A. Rai, A. K. Keshari, S. Saha Indole-fused benzooxazepines: a new structural class of anticancer agents. Future Sci OA. 2017;3(1):FSO168.
33. T. A. Popp, C. Tallant, C. Rogers, O. Fedorov, P. E. Brennan, S. Müller, S. Knapp, and F. Bracher. Development of Selective CBP/P300 Benzoxazepine Bromodomain Inhibitors. J. Med. Chem. 2016, 59, 8889 - 8912.
34. C. D. Smith, A. Wang, K. Vembaiyan, J. Zhang, C. Xie, Q. Zhou, G. Wu, S. R. Chen, T. G. Back, Novel carvedilol analogues that suppress store-overload-induced Ca2+ release. J. Med. Chem. 2013, 56, 8626 - 8655.
35. Z.-F. Shi, W.-Y. Chai, P. An, X.-P. Cao, Novel Synthesis of Amphiphilic Dendrons by the Double-Stage Convergent Method. Org. Lett. 2009, 11, 4394-4397.
36. D. B. Dess, J. C. Martin, Readily accessible 12-I-5 oxidant for the conversion of primary and secondary alcohols to aldehydes and ketones. J. Org. Chem. 1983, 48, 4155 - 4156.
37. G. Z. Elek, K. Koppel, D. M. Zubrytski, N. Konrad, I. Järving, M. Lopp, D. G. Kananovich, Divergent Access to Histone Deacetylase Inhibitory Cyclopeptides via a Late-Stage Cyclopropane Ring Cleavage Strategy. Short Synthesis of Chlamydocin. Org. Lett. 2019, 21, 8473-8478.
38. T. Murayama, N. Kurebayashi, M. Ishigami-Yuasa, S. Mori, Y. Suzuki, R. Akima, H. Ogawa, J. Suzuki, K. Kanemaru, H. Oyamada, Y. Kiuchi, M. Iino, H. Kagechika, T. Sakurai, Efficient High-Throughput Screening by Endoplasmic Reticulum Ca(2+) Measurement to Identify Inhibitors of Ryanodine Receptor Ca(2+)-Release Channels. Mol. Pharmacol. 2018, 94, 722 - 730.
39. T. Murayama, N. Kurebayashi, Assays for Modulators of Ryanodine Receptor (RyR)/Ca(2+) Release Channel Activity for Drug Discovery for Skeletal Muscle and Heart Diseases. Curr. Protoc. Pharmacol. 2019, 87, e71.

40. A. des Georges, O. B. Clarke, R. Zalk, Q. Yuan, K. J. Condon, R. A. Grassucci, W. A. Hendrickson, A. R. Marks, J. Frank, Structural Basis for Gating and Activation of RyR1. Cell 2016, 167, 145 - 157.e17.
41. G. B. Warren, P. A. Toon, N. J. Birdsall, A. G. Lee, J. C. Metcalfe, Reconstitution of a calcium pump using defined membrane components. Proc. Natl. Acad. Sci. U.S.A. 1974, 71, 622 - 626.
42. WO 2008/144483

## Claims

**1.** Compound represented by formula (1) or (2):

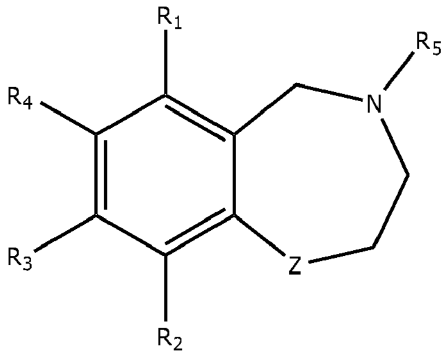

(1)

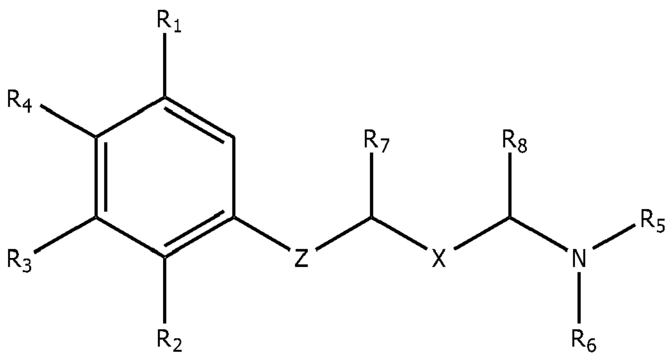

(2)

; wherein Z is O, or S;
$R_1$, $R_2$, $R_3$, and $R_4$ are independently selected from the group consisting of: H, OH, methoxy, ethoxy, methoxyalkyl, ethoxyalkyl, $CF_3$, and $CH_2CF_3$;
$R_5$ and optionally $R_6$ are independently selected from the group represented by any of formulae (3) to (11):

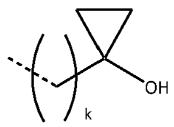 (3) 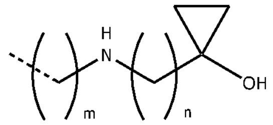 (4) 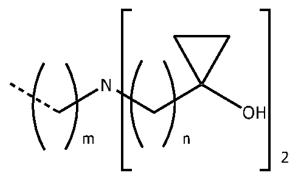 (5)

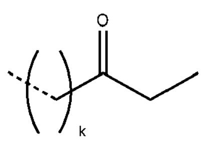 (6) 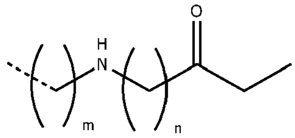 (7) 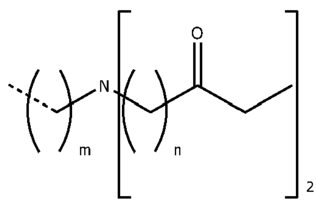 (8)

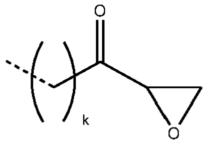 (9) 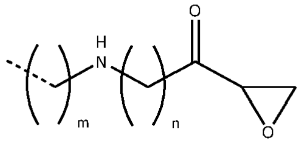 (10) 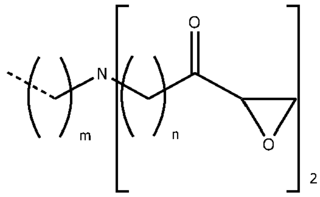 (11)

, wherein $R_6$ can be represented by H if it is not represented by any of formulae (3) to (11); wherein the dashed line represents the bond to the nitrogen atom of formula (1) or (2); and wherein

$$2 \leq k \leq 16;$$

$$2 \leq m \leq 8;$$

$$2 \leq n \leq 8;$$

and $R_7$ and $R_8$ are independently selected from the group consisting of: H, substituted or non-substituted $C_{1\text{-}8}$-alkyl, and $C_{1\text{-}12}$-alkylcyclopropanol;
wherein X represents a single bond or is -($CR_9R_{10}$)-, wherein $R_9$ and $R_{10}$ are independently selected from the group consisting of: H, substituted or non-substituted $C_{1\text{-}8}$-alkyl, and $C_{1\text{-}12}$-alkylcyclopropanol.

**2.** The compound according to claim 1, wherein Z is S.

**3.** The compound according to claim 1 or 2, wherein $R_1$, $R_2$, and $R_3$ are H.

**4.** The compound according to any of claims 1 to 3, wherein $R_4$ is methoxy.

**5.** Compound according to any of claims 1 to 4 for use as a medicament.

**6.** Compound according to any of claims 1 to 4 for use in the treatment of cardiac and skeletal muscle, brain and pancreas disorders, such as for the heart each cardiac arrhythmia including atrial fibrillation, heart failure with increased adrenergic stress, sudden cardiac death in the genetic RyR2 disorder stress-induced Catecholaminergic Polymorphic Ventricular Tachycardia patients, and age-related cardiac dysfunction by improving maintenance of resting cell $Ca^{2+}$ levels.

**7.** The compound for use according to claim 6, wherein improving maintenance of diastolic resting cell $Ca^{2+}$ levels comprises

a) preventing pathologically increased either acutely, preferably stress-induced acutely, or chronic $Ca^{2+}$ leak through inhibiting ryanodine receptor type 2 (RyR2) channel activity,
and/or
b) enhancing cardiac sarco-endoplasmic reticulum $Ca^{2+}$ load by stimulating $Ca^{2+}$ uptake via SER $Ca^{2+}$-ATPase (SERCA), preferably in cardiomyocytes the SER $Ca^{2+}$-ATPase 2a isoform SERCA2a.

**8.** A method of synthesizing

a compound represented by formula (1):

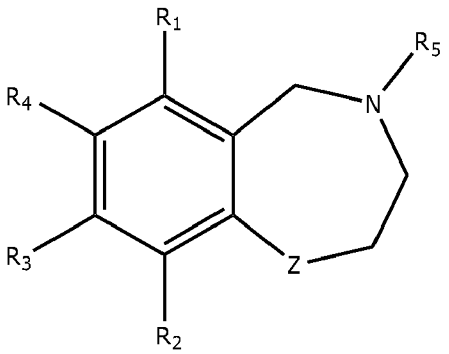

(1)

, or

a compound represented by formula (2):

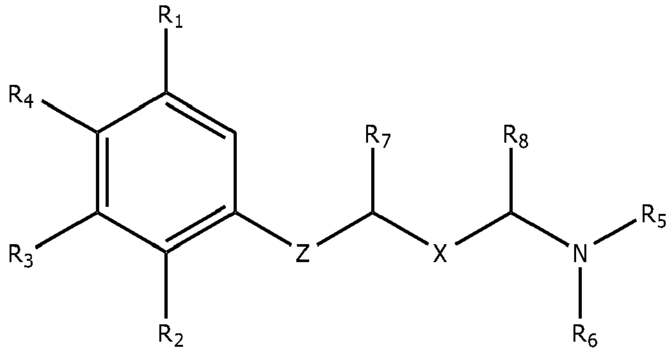

(2)

, wherein Z, X, $R_1$, $R_2$, $R_3$, $R_4$, $R_7$, and $R_8$ are as defined in claim 1,
wherein $R_5$ and optionally $R_6$ are independently selected from the group represented by any of formulae (3) to (5):

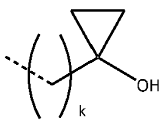

(3)

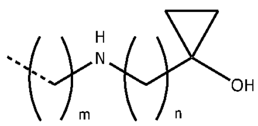

(4)

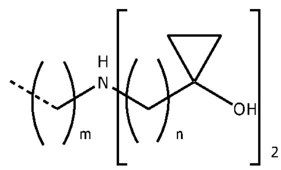

(5)

, wherein $R_6$ can be represented by H if it is not represented by any of formulae (3) to (5),
wherein k, m, n are as defined in claim 1;
wherein the method comprises the steps of:

a) providing

a compound represented by formula (Ia) to synthesize the compound represented by formula (1) wherein $R_5$ is represented by formula (3), (4), or (5):

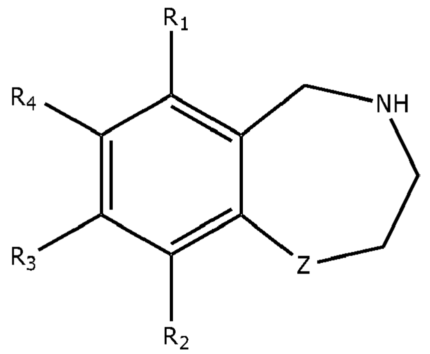

(Ia)

, or

a compound represented by formula (Ia') to synthesize the compound represented by formula (1) wherein $R_5$ is independently selected from the group represented by any of formulae (4) or (5):

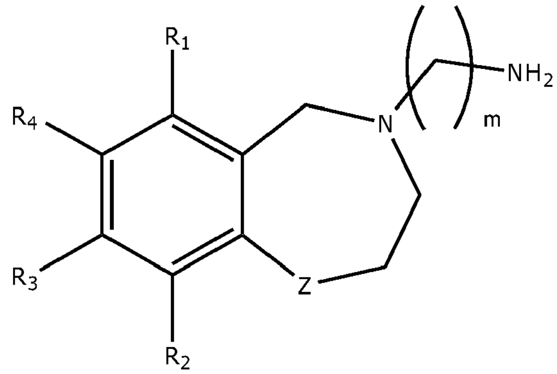

(Ia')

, or

a compound represented by formula (Ib) to synthesize the compound represented by formula (2):

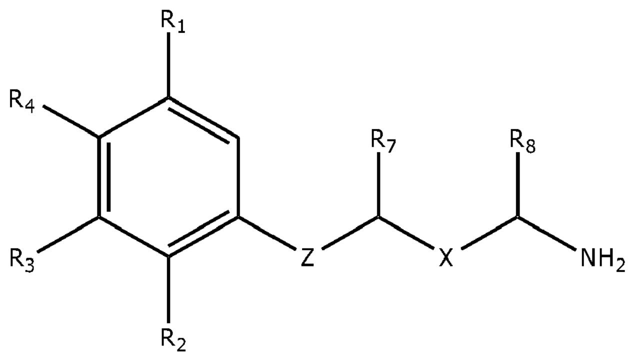

(Ib)

, wherein Z, X, $R_1$, $R_2$, $R_3$, $R_4$, $R_7$, and $R_8$ are as defined in claim 1,

b) reacting the compound provided by step a) using

b1) reductive alkylation with a suitable reducing agent and

a compound represented by formula (II), (III) or (IV) if the compound represented by formula (Ia) or if the compound represented by (Ib) is provided by step a):

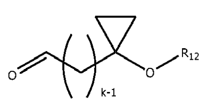 (II) 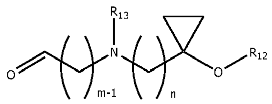 (III) 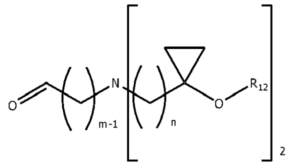 (IV)

, or

a compound represented by formula (II') if the compound represented by formula (Ia') is provided by step a):

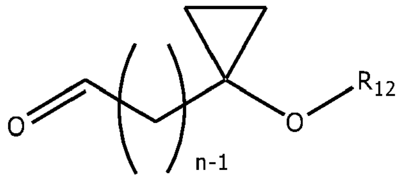 (II')

or

b2) direct alkylation with a suitable strong base and

a compound represented by formulae (V), (VI) or (VII) if the compound represented by formula (Ia) or if the compound represented by (Ib) is provided by step a):

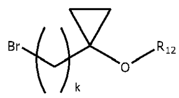 (V) 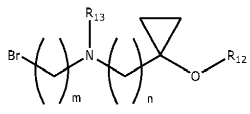 (VI) 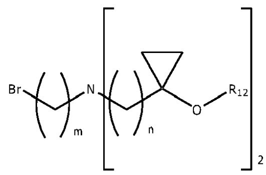 (VII)

, or

a compound represented by formula (V') if the compound represented by formula (Ia') is provided by step a):

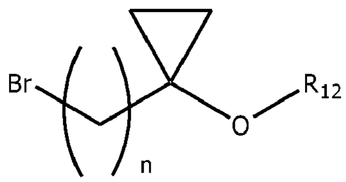 (V')

, wherein k, m, n are as defined in claim 1,
wherein $R_{12}$ is a silyl ether or allyl ether protecting group,
wherein $R_{13}$ is an amine protecting group, preferably selected from the group consisting of: tert-butyloxycarbonyl, 9-fluorenylmethyloxycarbonyl, allyloxycarbonyl;

c) reacting the reaction product of step b) in a deprotection reaction with a suitable deprotection agent to form the cyclopropanol groups represented by any of formulae (3) to (5).

**9.** The method of claim 8, wherein the reducing agent comprises borohydrides, preferably wherein the reducing agent comprises a borohydride selected from the group consisting of: sodium cyanoborohydride, triacetoxyborohydride, 2-picolynyl borane, and combinations thereof, more preferably wherein the reducing agent comprises sodium triacetoxyborohydride.

**10.** The method of claim 8 or 9, wherein the deprotection agent for

a) the silyl ether protecting group comprises a fluoride containing compound, preferably wherein the fluoride

containing compound is selected from the group consisting of: HF, KF, n-$Bu_4NF$, and combinations thereof; b) the allyl ether protecting group comprises a Pd (0) reagent in combination with a reducing agent.

**11.** The method according to of any of claims 8 to 10, wherein the silyl ether protecting group is selected from the group consisting of: trimethylsilyl, triethylsilyl, tert-butyldimethylsilyl, tert-butyldiphenylsilyl, triisopropylsilyl; preferably wherein the silyl ether protecting group is tert-butyldimethylsilyl.

**12.** A method of synthesizing

a compound represented by formula (1):

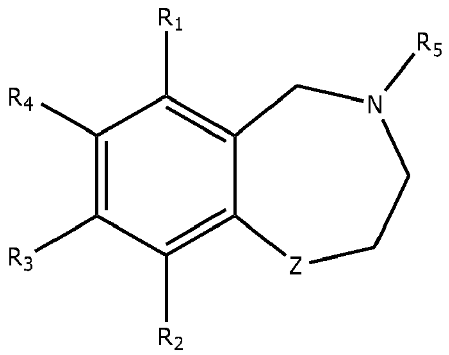

(1)

, or
a compound represented by formula (2):

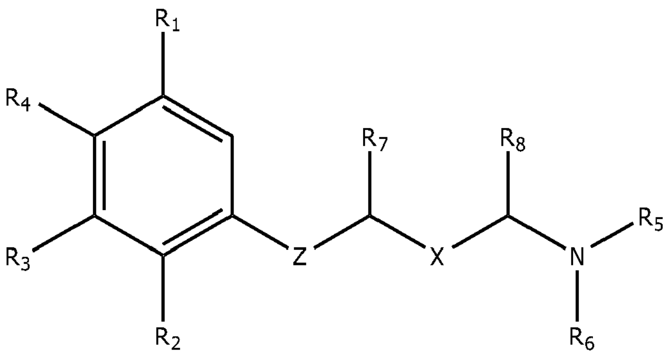

(2)

, wherein Z, X, $R_1$, $R_2$, $R_3$, $R_4$, $R_7$, and $R_8$ are as defined in claim 1,
wherein $R_5$ and optionally $R_6$ are independently selected from the group represented by any of formulae (6) to (8):

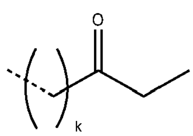 (6) 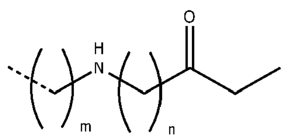 (7) 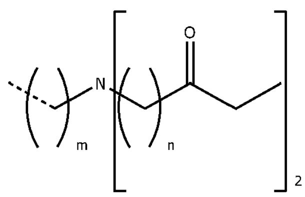 (8)

, wherein $R_6$ can be represented by H if it is not represented by any of formulae (6) to (8),
wherein k, m, and n are as defined in claim 1;
wherein the method comprises the steps of:

a) providing

a compound represented by formula (1),

or
a compound represented by formula (2),
each prepared by the method according to any of claims 8 to 11;

b) reacting the compound provided in step a) under acidic conditions in a rearrangement reaction to form the groups represented any of formulae (6) to (8).

**13.** The method of claim 12, wherein step b) is performed at a pH < 2.

**14.** A method of synthesizing

a compound represented by formula (1):

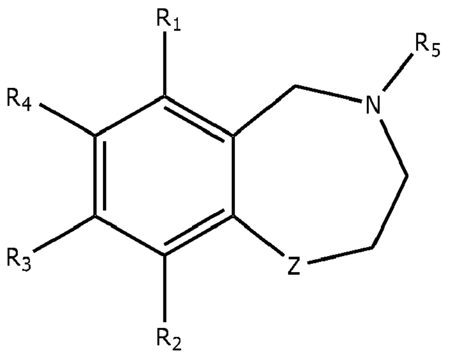

(1)

, or
a compound represented by formula (2):

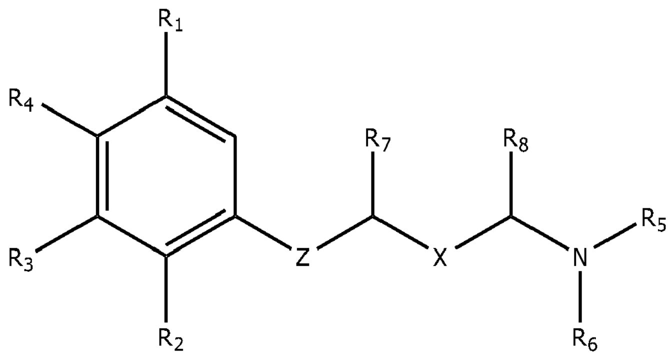

(2)

, wherein Z, X, $R_1$, $R_2$, $R_3$, $R_4$, $R_7$, and $R_8$ are as defined in claim 1,
wherein $R_5$ and optionally $R_6$ are independently selected from the group represented by any of formulae (9) to (11):

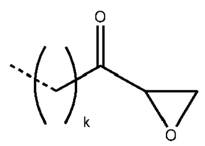

(9)

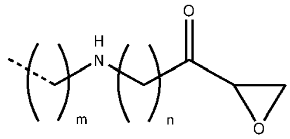

(10)

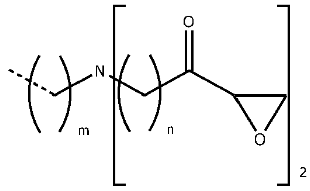

(11)

, wherein $R_6$ can be represented by H if it is not represented by any of formulae (9) to (11),
wherein k, m, n are as defined in claim 1;
wherein the method comprises the steps of:

a) providing

a compound represented by formula (1),
or
a compound represented by formula (2),
each prepared by the method according to any of claims 8 to 11;

b) reacting the compound provided by step a) with a compound selected from the group consisting of Mn(acac)$_3$, Mn(II) abietate, and combinations thereof in an oxygen-containing atmosphere, followed by an addition of a base to form the groups represented by any of formulae (9), (10) or (11).

**15.** The method of claim 14, wherein the base is selected from the group consisting of: 1,8-diazabicyclo[5.4.0]undec-7-ene, KOH, and combinations thereof, preferably wherein the base is 1,8-diazabicyclo[5.4.0]undec-7-ene.

## Fig. 1

A

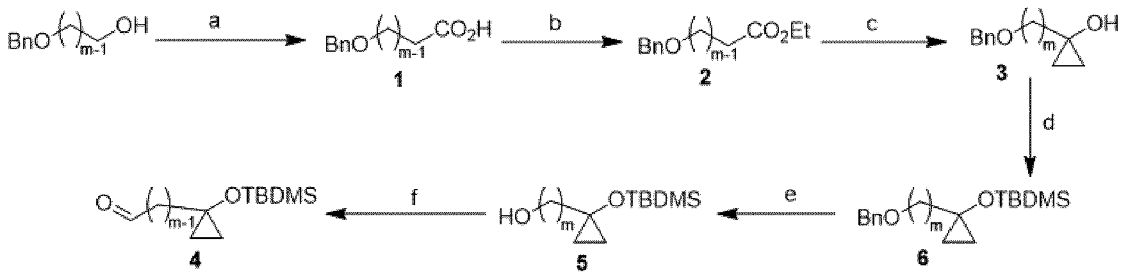


B

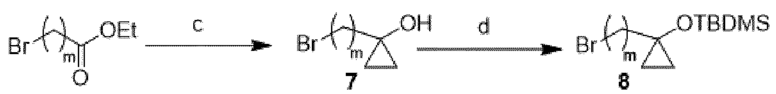


C

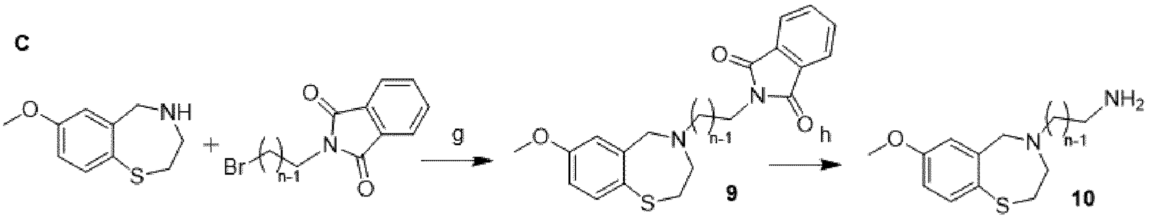


D

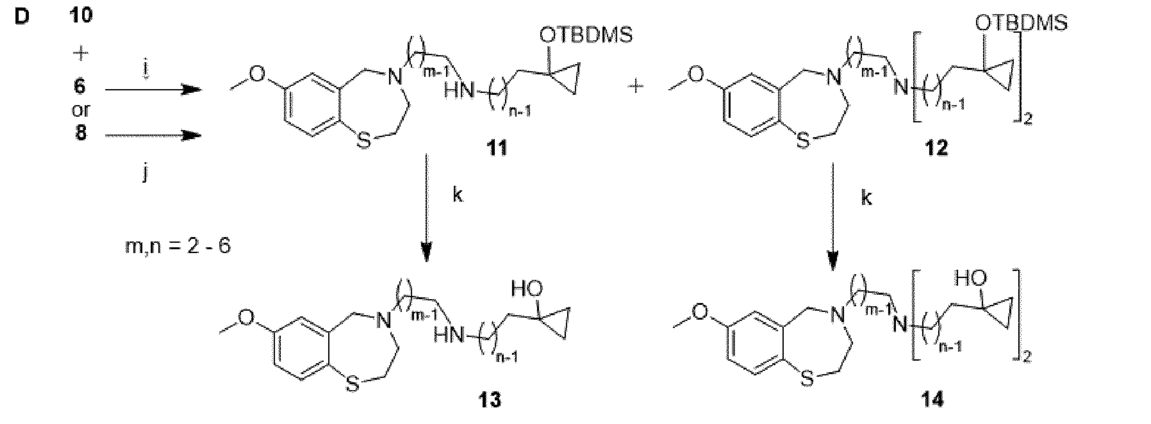

## Fig. 2

**E**

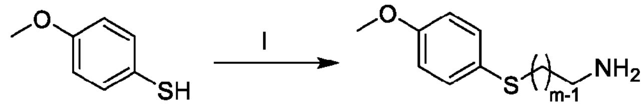


**15**

**F**

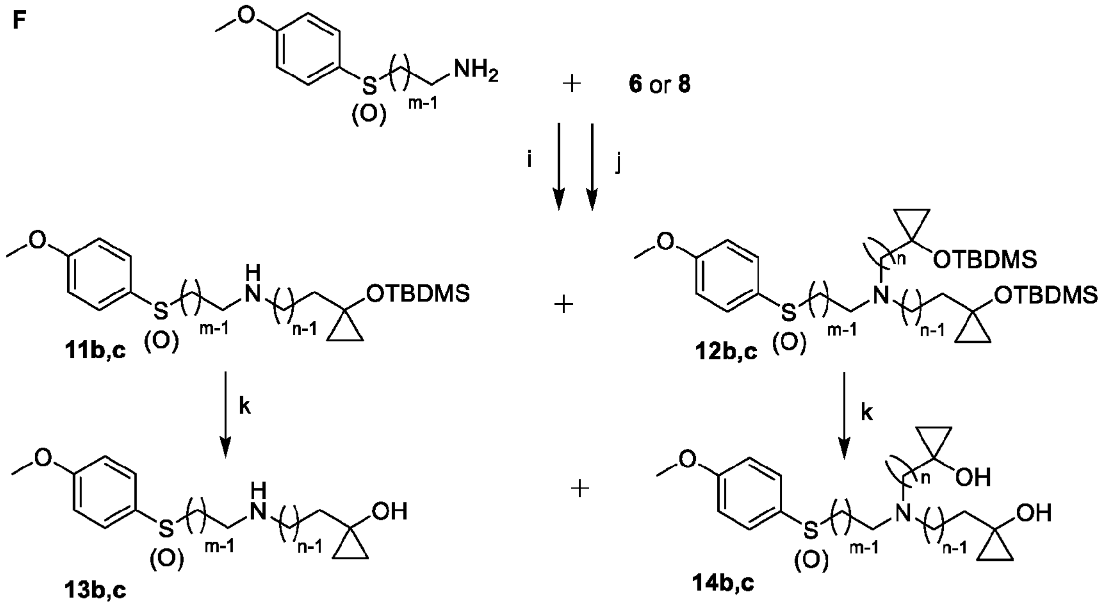


**11b,c** **12b,c** **13b,c** **14b,c**

## Fig. 3

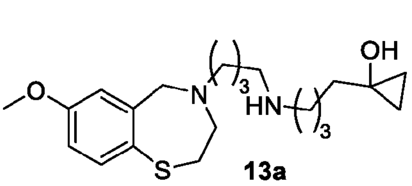

**13a**

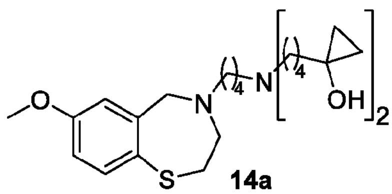

**14a**

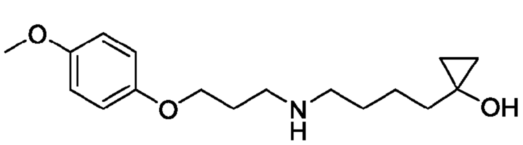

**13b**

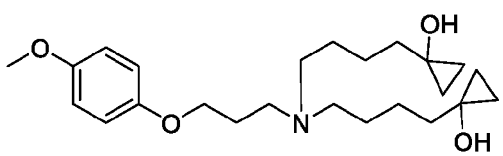

**14b**

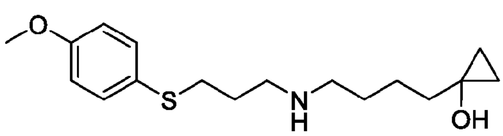

**13c**

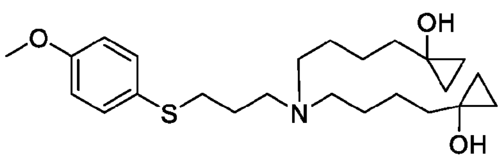

**14c**

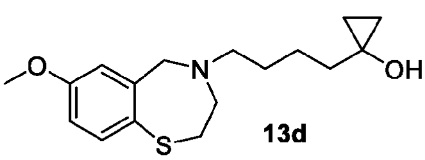

**13d**

**Fig. 4**

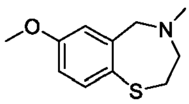

S107

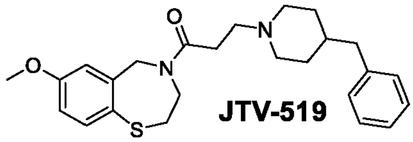

JTV-519

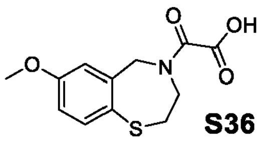

S36

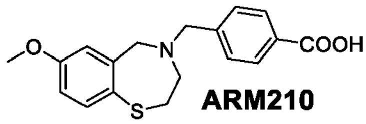

ARM210

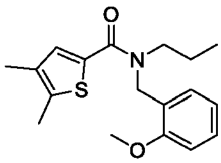

**Fig. 5**

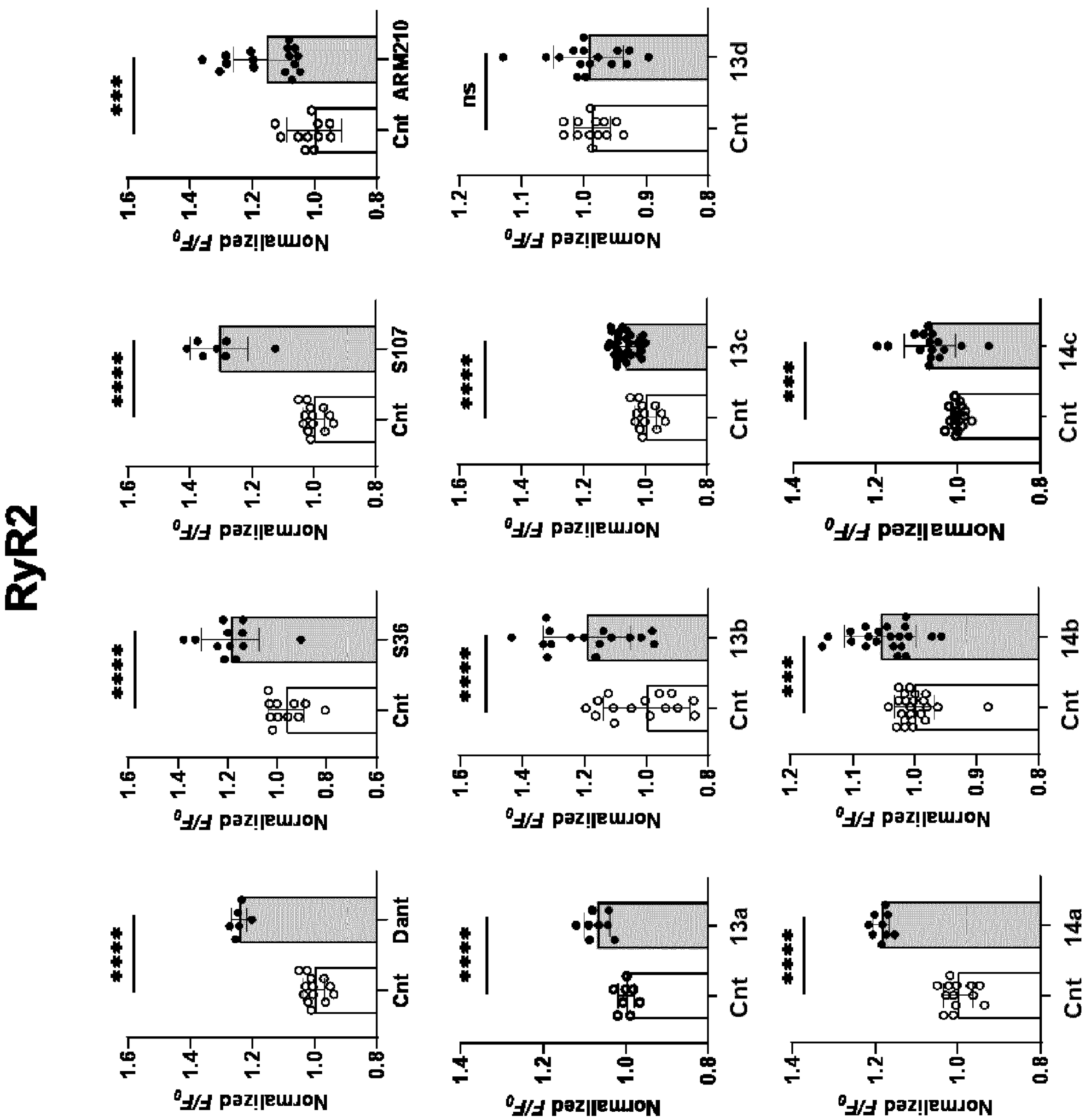
RyR2
Normalized F/F0
Cnt
Dant
S36
S107
ARM210
13a
13b
13c
13d
14a
14b
14c
****
***
ns

**Fig. 6**

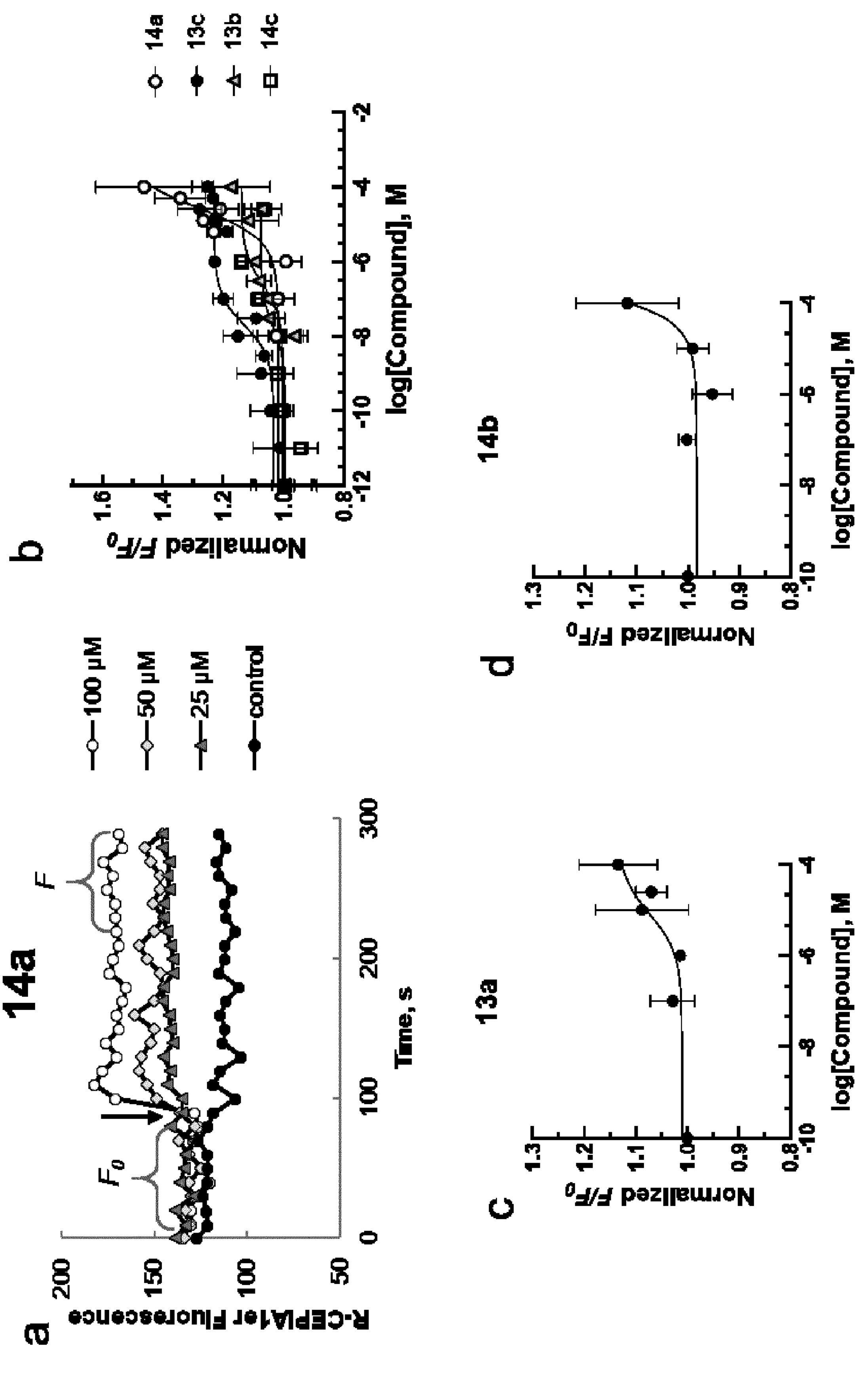
a
14a
R-CEPIA1er Fluorescence
Time, s
F
F0
100 µM
50 µM
25 µM
control
b
Normalized F/F0
log[Compound], M
14a
13c
13b
14c
c
13a
Normalized F/F0
log[Compound], M
d
14b
Normalized F/F0
log[Compound], M

**Fig. 7**

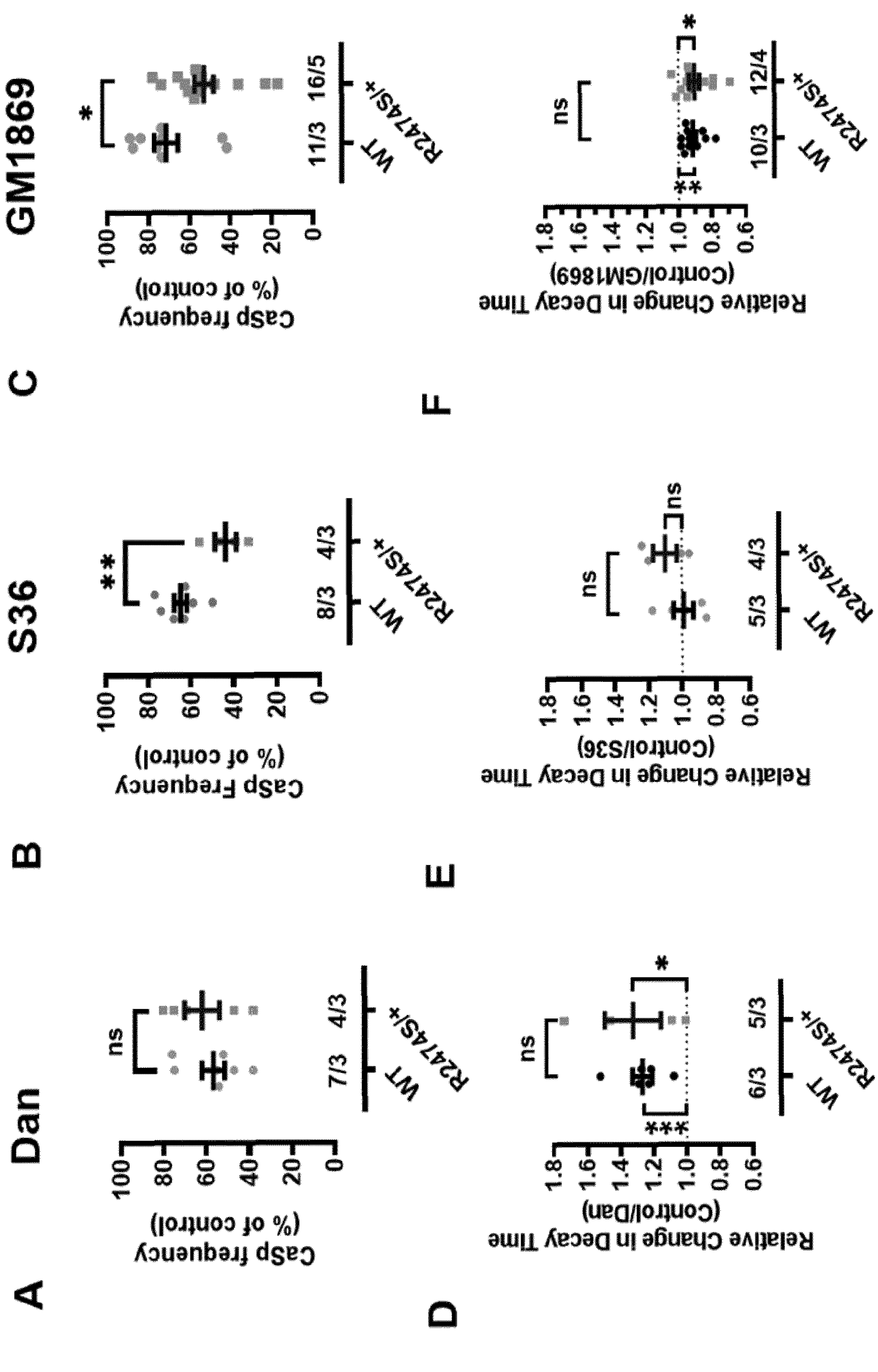
A
Dan
B
S36
C
GM1869
CaSp frequency (% of control)
CaSp Frequency (% of control)
100
80
60
40
20
0
ns
**
*
WT
R2474S/+
7/3
4/3
8/3
11/3
16/5
D
E
F
Relative Change in Decay Time (Control/Dan)
Relative Change in Decay Time (Control/S36)
Relative Change in Decay Time (Control/GM1869)
1.8
1.6
1.4
1.2
1.0
0.8
0.6
***
6/3
5/3
5/3
4/3
10/3
12/4

Fig. 8

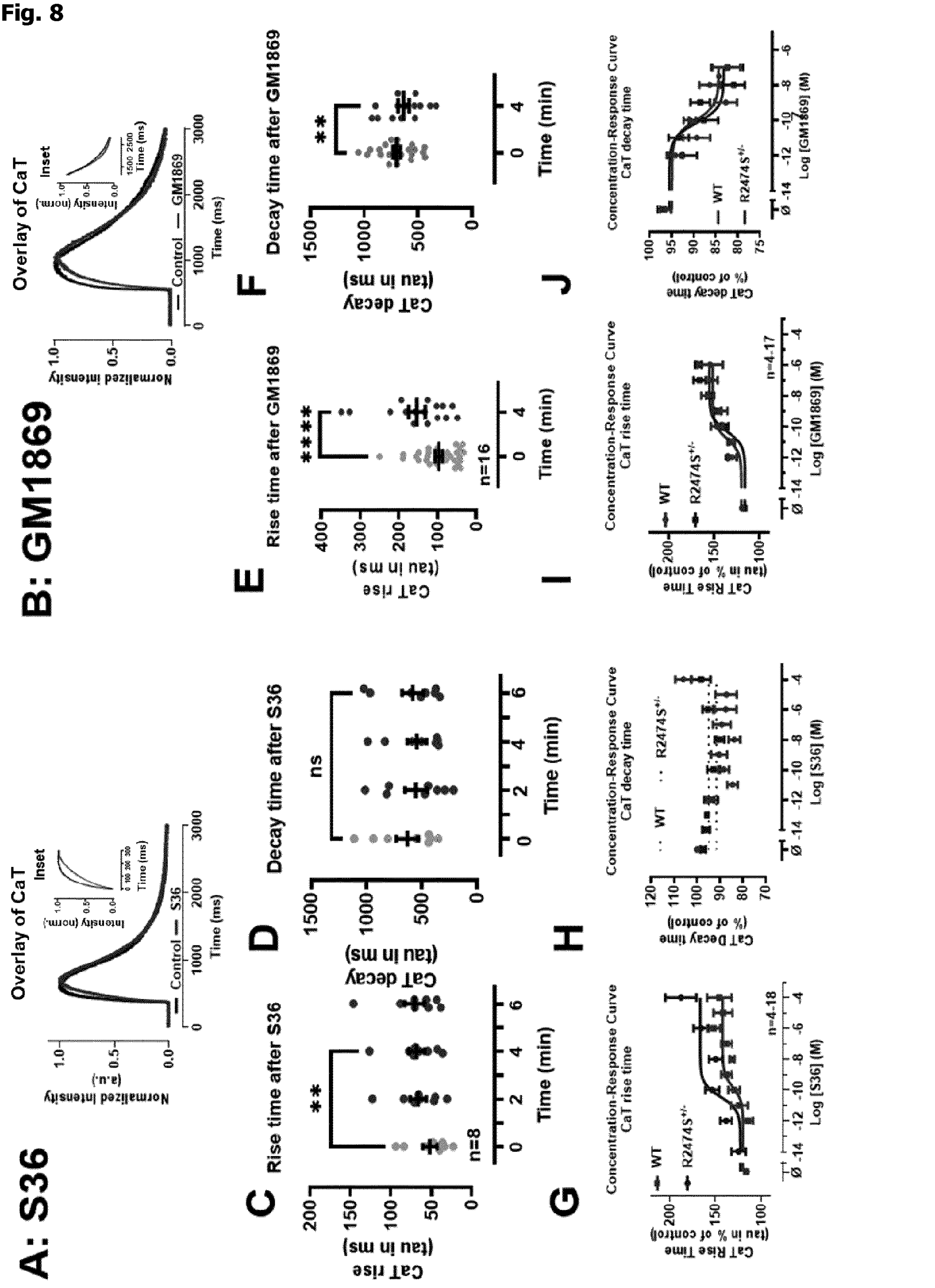
A: S36
B: GM1869
Overlay of CaT
Inset
Control
S36
GM1869
Time (ms)
Normalized intensity (a.u.)
Intensity (norm.)
C
Rise time after S36
D
Decay time after S36
E
Rise time after GM1869
F
Decay time after GM1869
CaT rise (tau in ms)
CaT decay (tau in ms)
Time (min)
n=8
n=16
ns
G
H
I
J
Concentration-Response Curve
CaT rise time
CaT decay time
WT
R2474S+/-
CaT Rise Time (tau in % of control)
CaT Decay time (% of control)
Log [S36] (M)
Log [GM1869] (M)
n=4-18
n=4-17

**Fig. 9**

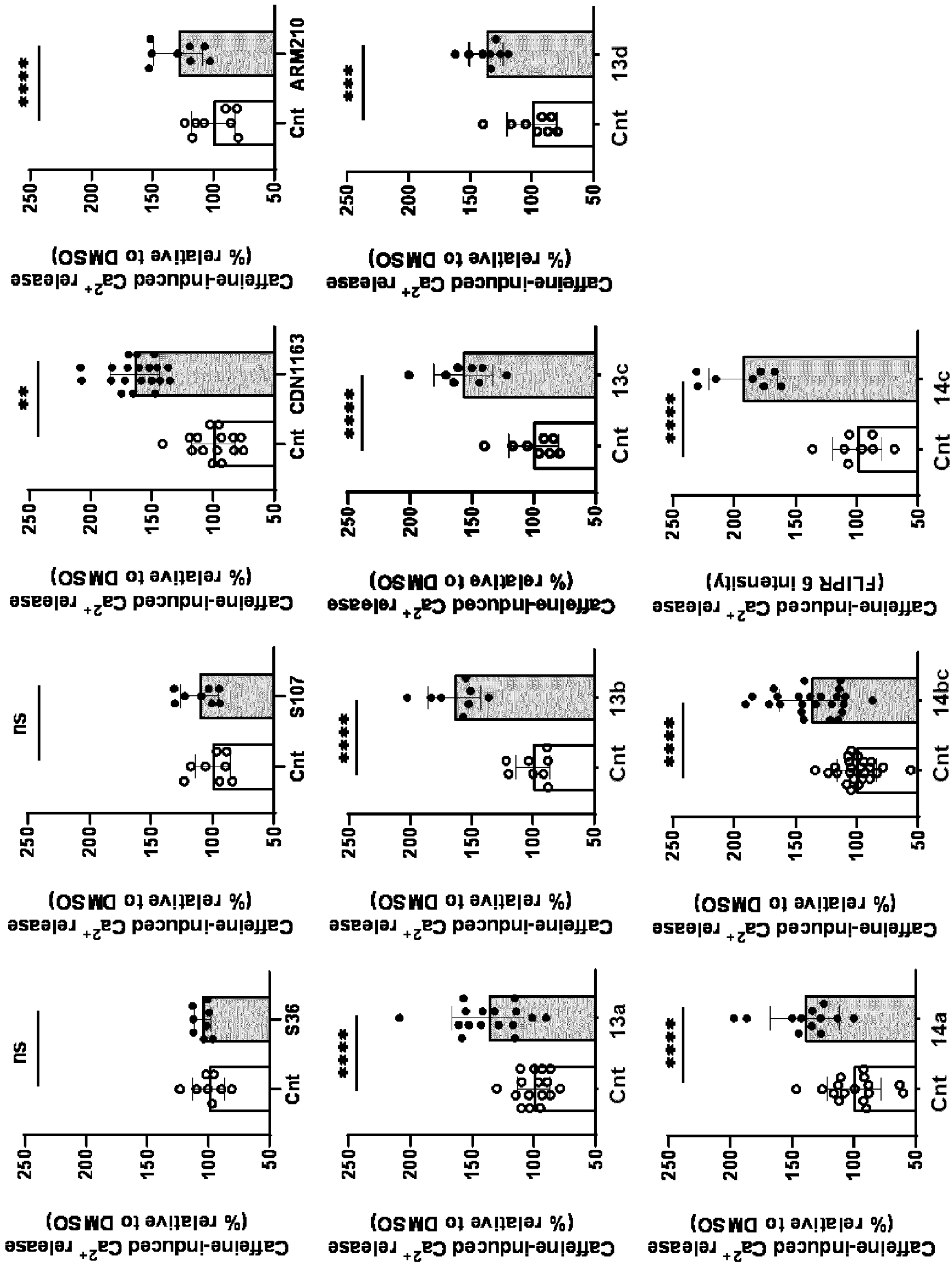
Caffeine-induced Ca2+ release (% relative to DMSO)
Caffeine-induced Ca2+ release (FLIPR 6 intensity)
250
200
150
100
50
Cnt
S36
S107
CDN1163
ARM210
13a
13b
13c
13d
14a
14bc
14c
ns
**
***
****

Fig. 10

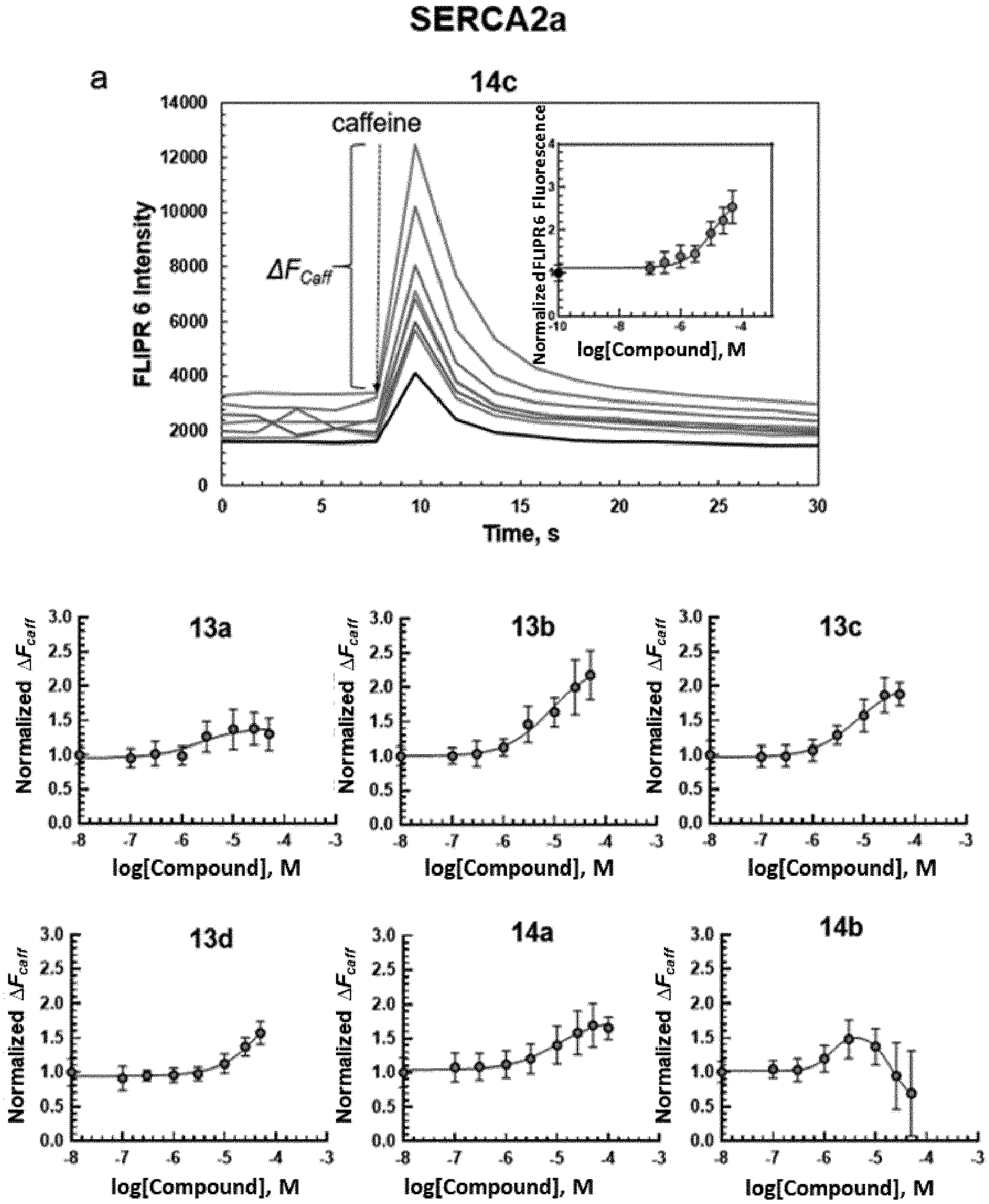
SERCA2a
a
14c
caffeine
ΔF_Caff
FLIPR 6 Intensity
Time, s
Normalized FLIPR 6 Fluorescence
log[Compound], M
b
13a
13b
13c
13d
14a
14b
Normalized ΔF_caff
log[Compound], M

Fig. 11

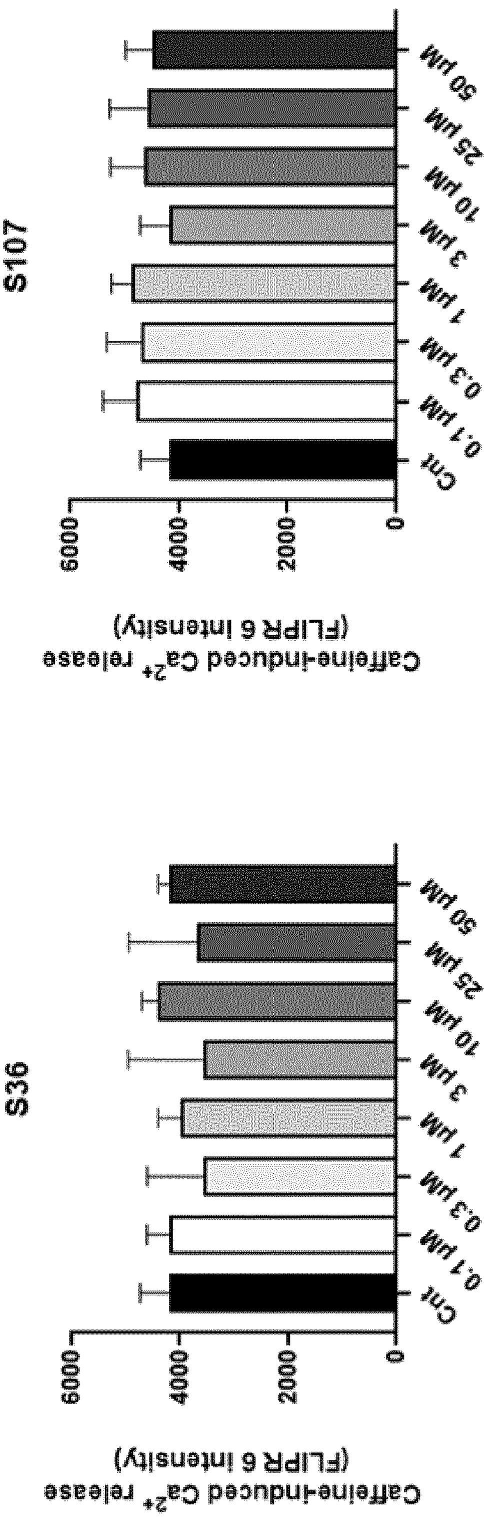
S107
S36
Caffeine-induced Ca2+ release
(FLIPR 6 intensity)
6000
4000
2000
0
Cnt
0.1 uM
0.3 uM
1 uM
3 uM
10 uM
25 uM
50 uM

**Fig. 12**

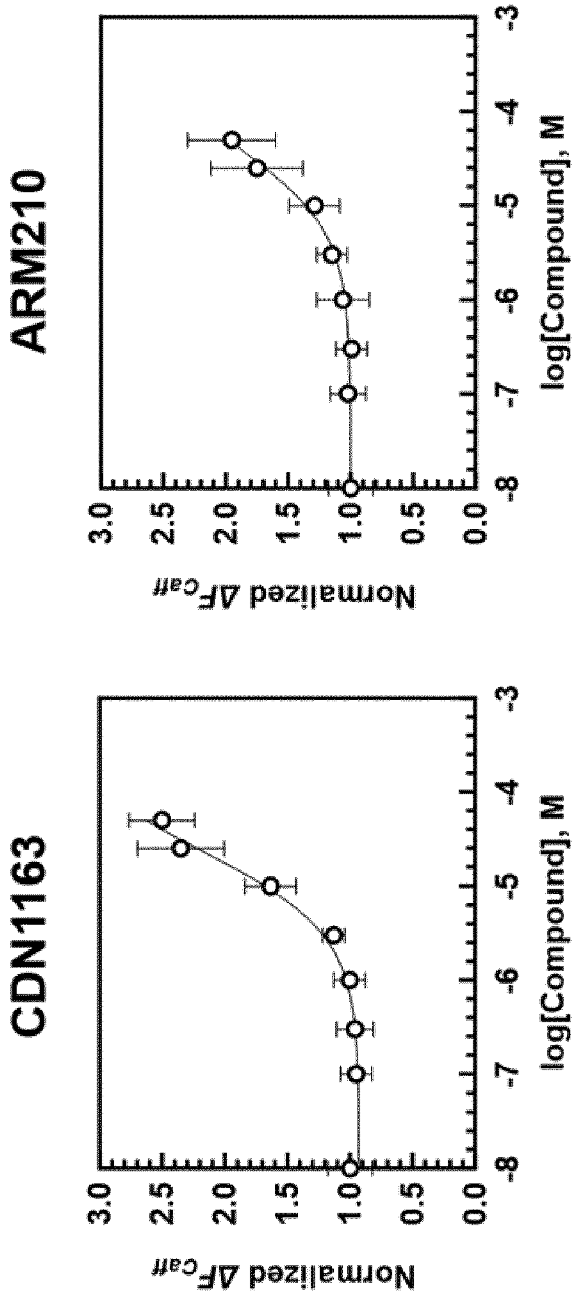
ARM210
CDN1163
Normalized ΔF_Caff
log[Compound], M
3.0
2.5
2.0
1.5
1.0
0.5
0.0
-8
-7
-6
-5
-4
-3

**Fig. 13**

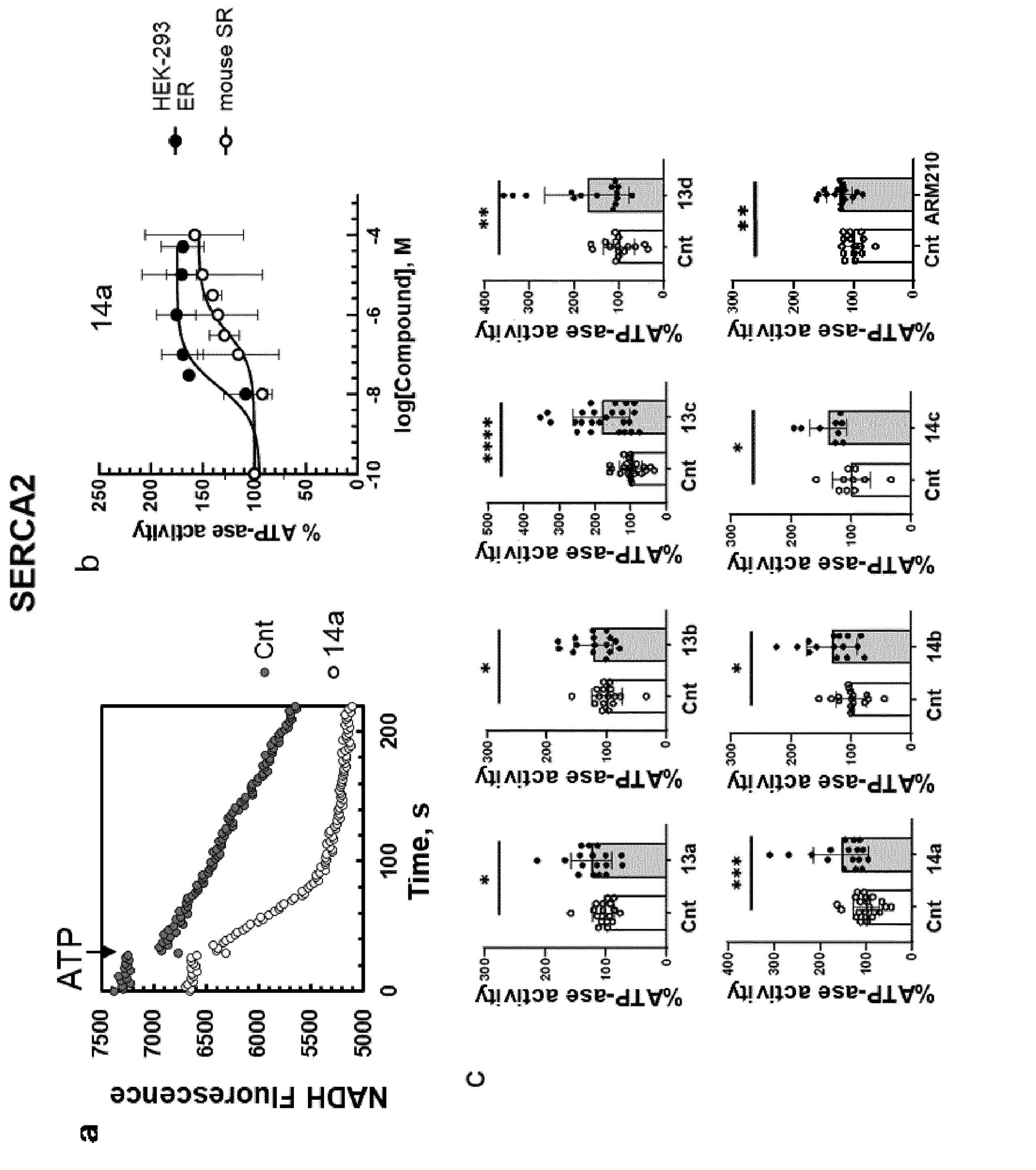
SERCA2
a
NADH Fluorescence
Time, s
ATP
Cnt
14a
b
14a
% ATP-ase activity
log[Compound], M
HEK-293 ER
mouse SR
c
%ATP-ase activity
Cnt
13a
13b
13c
13d
14a
14b
14c
ARM210

**Fig. 14**

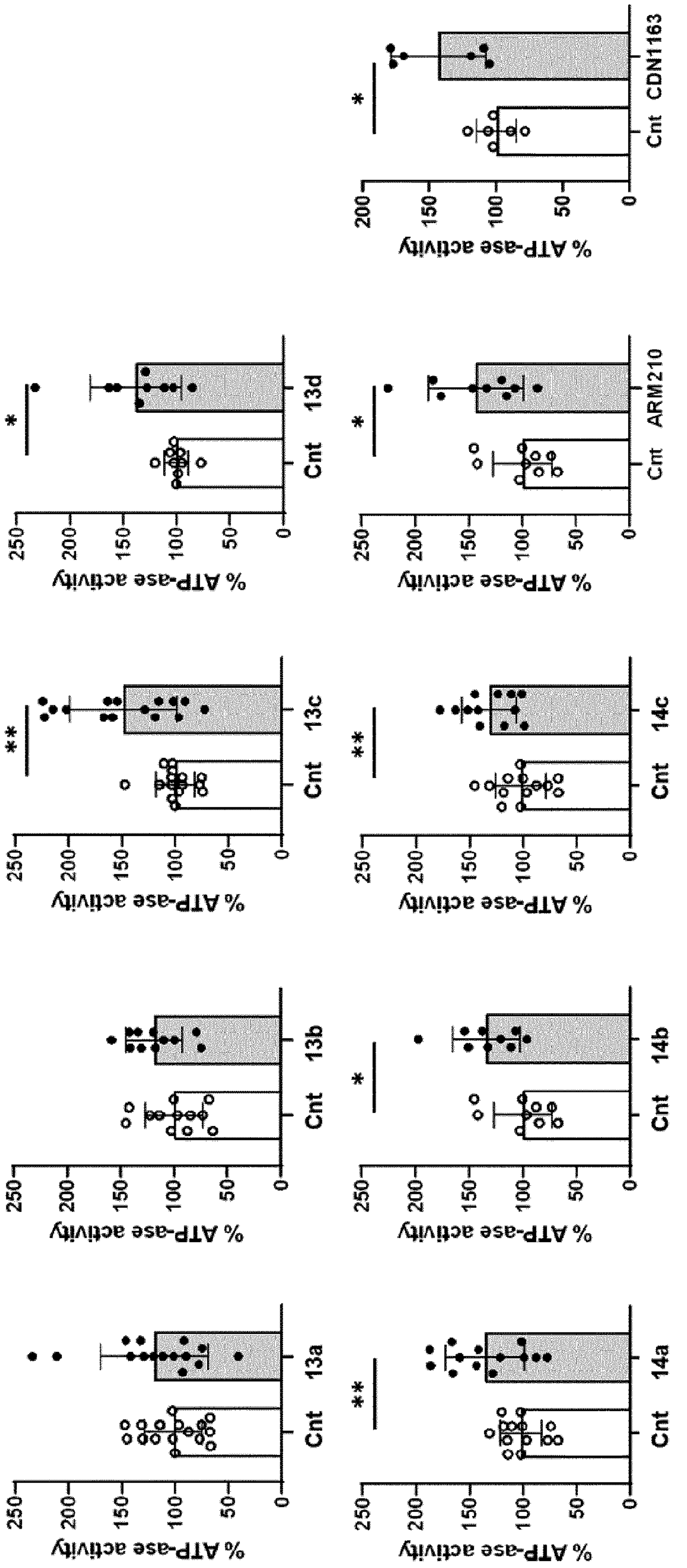
% ATP-ase activity
Cnt
13a
13b
13c
13d
14a
14b
14c
ARM210
CDN1163
*
**

**Fig. 15**

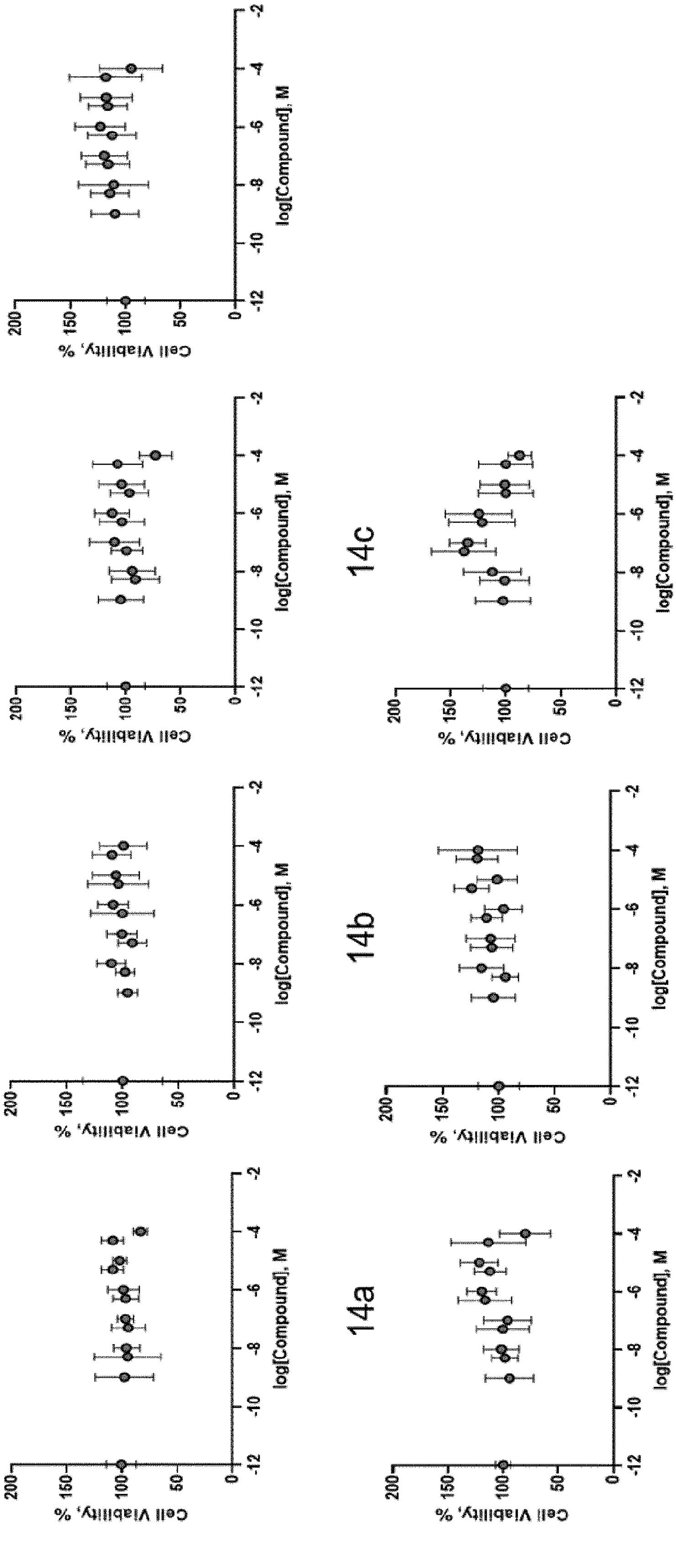

Europäisches Patentamt
European Patent Office
Office européen des brevets

# EUROPEAN SEARCH REPORT

Application Number
EP 23 20 8053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] CHEMICAL ABSTRACTS SERVICE, COLUMBUS, OHIO, US; 12 September 2016 (2016-09-12), XP002811291, retrieved from STNext Database accession no. 1991712-84-5 * abstract * | 1,3 | INV. A61P1/18 A61P9/04 A61P9/06 A61P25/00 C07C213/08 C07C217/20 C07C319/20 C07C323/25 C07D281/10 |
| A | WO 2022/108945 A1 (ARMGO PHARMA INC [US]) 27 May 2022 (2022-05-27) * claims; examples * | 1-15 | |
| A | WO 2016/109596 A1 (MYOTHERIX INC [US]) 7 July 2016 (2016-07-07) * the whole document * | 1-15 | |
| A | EP 2 653 466 A1 (SERVIER LAB [FR]; ARMGO PHARMA INC [US]) 23 October 2013 (2013-10-23) * the whole document * | 1-15 | TECHNICAL FIELDS SEARCHED (IPC) |
| A | WO 2007/024717 A2 (UNIV COLUMBIA [US]; MARKS ANDREW ROBERT [US] ET AL.) 1 March 2007 (2007-03-01) * the whole document * | 1-15 | C07D C07C |

-/--

The present search report has been drawn up for all claims

1

| Place of search | Date of completion of the search | Examiner |
|---|---|---|
| Munich | 28 March 2024 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office / Europäisches Patentamt / Office européen des brevets

# EUROPEAN SEARCH REPORT

Application Number
EP 23 20 8053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MITRONOVA GYUZEL Y. ET AL: "1,4-Benzothiazepines with Cyclopropanol Groups and Their Structural Analogues Exhibit Both RyR2-Stabilizing and SERCA2a-Stimulating Activities", JOURNAL OF MEDICINAL CHEMISTRY, vol. 66, no. 23, 22 November 2023 (2023-11-22), pages 15761-15775, XP093146211, US ISSN: 0022-2623, DOI: 10.1021/acs.jmedchem.3c01235 Retrieved from the Internet: URL:https://pubs.acs.org/doi/pdf/10.1021/acs.jmedchem.3c01235> ----- | | |

TECHNICAL FIELDS SEARCHED (IPC)

The present search report has been drawn up for all claims

| Place of search | Date of completion of the search | Examiner |
|---|---|---|
| Munich | 28 March 2024 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 20 8053

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2024

| Patent document cited in search report | Publication date | Patent family member(s) | Publication date |
| --- | --- | --- | --- |
| WO 2022108945 A1 | 27-05-2022 | AU 2021381942 A1 | 29-06-2023 |
| | | BR 112023009303 A2 | 06-02-2024 |
| | | CA 3177397 A1 | 27-05-2022 |
| | | CN 116710425 A | 05-09-2023 |
| | | EP 4247778 A1 | 27-09-2023 |
| | | IL 302866 A | 01-07-2023 |
| | | JP 2023549583 A | 27-11-2023 |
| | | KR 20230129394 A | 08-09-2023 |
| | | US 2023399324 A1 | 14-12-2023 |
| | | WO 2022108945 A1 | 27-05-2022 |
| WO 2016109596 A1 | 07-07-2016 | AU 2015374155 A1 | 20-07-2017 |
| | | CA 2971869 A1 | 07-07-2016 |
| | | CN 107454842 A | 08-12-2017 |
| | | EP 3240547 A1 | 08-11-2017 |
| | | JP 2018500382 A | 11-01-2018 |
| | | KR 20170102504 A | 11-09-2017 |
| | | US 2016207893 A1 | 21-07-2016 |
| | | WO 2016109596 A1 | 07-07-2016 |
| EP 2653466 A1 | 23-10-2013 | AP 3591 A | 15-02-2016 |
| | | AR 090712 A1 | 03-12-2014 |
| | | AU 2013248313 A1 | 30-10-2014 |
| | | BR 112014025670 B1 | 30-06-2020 |
| | | CA 2870599 A1 | 24-10-2013 |
| | | CL 2014002756 A1 | 12-06-2015 |
| | | CN 104350045 A | 11-02-2015 |
| | | CO 7091184 A2 | 21-10-2014 |
| | | CR 20140476 A | 02-12-2014 |
| | | CU 20140119 A7 | 30-03-2015 |
| | | CY 1115826 T1 | 25-01-2017 |
| | | DK 2653466 T3 | 02-02-2015 |
| | | DO P2014000230 A | 15-12-2014 |
| | | EA 201401141 A1 | 30-04-2015 |
| | | EC SP14027564 A | 31-08-2015 |
| | | EP 2653466 A1 | 23-10-2013 |
| | | EP 2708535 A1 | 19-03-2014 |
| | | ES 2529890 T3 | 25-02-2015 |
| | | GT 201400222 A | 28-09-2018 |
| | | HK 1192537 A1 | 22-08-2014 |
| | | HK 1207073 A1 | 22-01-2016 |
| | | HR P20141113 T1 | 02-01-2015 |
| | | HU E024284 T2 | 28-01-2016 |
| | | IL 235043 A | 31-10-2016 |
| | | JO 3070 B1 | 15-03-2017 |
| | | JP 5965542 B2 | 10-08-2016 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 23 20 8053

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2024

| Patent document cited in search report | Publication date | Patent family member(s) | Publication date |
|---|---|---|---|
| | | JP 2015514736 A | 21-05-2015 |
| | | KR 20150003347 A | 08-01-2015 |
| | | MA 37525 A1 | 31-05-2016 |
| | | MD 20140119 A2 | 30-04-2015 |
| | | ME 01969 B | 20-05-2015 |
| | | MX 350890 B | 25-09-2017 |
| | | MY 167783 A | 25-09-2018 |
| | | NI 201400123 A | 05-03-2015 |
| | | NZ 701122 A | 29-07-2016 |
| | | PE 20142193 A1 | 31-12-2014 |
| | | PH 12014502256 A1 | 09-02-2015 |
| | | PL 2653466 T3 | 30-04-2015 |
| | | PT 2653466 E | 03-12-2014 |
| | | RU 2014145939 A | 10-06-2016 |
| | | SG 11201406404Y A | 27-11-2014 |
| | | SI 2653466 T1 | 31-03-2015 |
| | | TN 2014000424 A1 | 30-03-2016 |
| | | TW 201345900 A | 16-11-2013 |
| | | UA 113759 C2 | 10-03-2017 |
| | | US 2013281512 A1 | 24-10-2013 |
| | | US 2014088171 A1 | 27-03-2014 |
| | | US 2014378437 A1 | 25-12-2014 |
| | | UY 34742 A | 29-11-2013 |
| | | WO 2013156505 A1 | 24-10-2013 |
| | | ZA 201407499 B | 23-12-2015 |
| WO 2007024717 A2 | 01-03-2007 | AP 3091 A | 31-01-2015 |
| | | AR 057776 A1 | 19-12-2007 |
| | | AU 2006283534 A1 | 01-03-2007 |
| | | BR PI0615097 A2 | 14-07-2009 |
| | | CA 2620183 A1 | 01-03-2007 |
| | | CN 101500576 A | 05-08-2009 |
| | | CN 102558093 A | 11-07-2012 |
| | | CR 9812 A | 14-09-2009 |
| | | CY 1113974 T1 | 27-07-2016 |
| | | CY 1113975 T1 | 27-07-2016 |
| | | CY 1114091 T1 | 27-07-2016 |
| | | DK 1928850 T3 | 23-09-2013 |
| | | DK 2177224 T3 | 13-05-2013 |
| | | DK 2311464 T3 | 13-05-2013 |
| | | EA 200800665 A1 | 29-08-2008 |
| | | EC SP088306 A | 30-07-2008 |
| | | EP 1928850 A2 | 11-06-2008 |
| | | EP 2177224 A2 | 21-04-2010 |
| | | EP 2311464 A1 | 20-04-2011 |
| | | EP 2764867 A1 | 13-08-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 20 8053

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2024

| Patent document cited in search report | Publication date | Patent family member(s) | Publication date |
|---|---|---|---|
| | | ES 2405765 T3 | 03-06-2013 |
| | | ES 2405780 T3 | 03-06-2013 |
| | | ES 2421159 T3 | 29-08-2013 |
| | | GE P20105134 B | 27-12-2010 |
| | | HK 1119705 A1 | 13-03-2009 |
| | | HK 1137150 A1 | 23-07-2010 |
| | | HK 1153664 A1 | 05-04-2012 |
| | | HN 2008000299 A | 11-07-2011 |
| | | HR P20130353 T1 | 30-06-2013 |
| | | HR P20130360 T1 | 30-06-2013 |
| | | HR P20130669 T1 | 31-08-2013 |
| | | IL 189675 A | 31-12-2013 |
| | | JP 5342877 B2 | 13-11-2013 |
| | | JP 2009506034 A | 12-02-2009 |
| | | KR 20080037741 A | 30-04-2008 |
| | | KR 20120025626 A | 15-03-2012 |
| | | MA 29785 B1 | 01-09-2008 |
| | | MY 144622 A | 14-10-2011 |
| | | NZ 566822 A | 29-04-2011 |
| | | PL 1928850 T3 | 29-11-2013 |
| | | PL 2177224 T3 | 31-07-2013 |
| | | PL 2311464 T3 | 31-07-2013 |
| | | PT 1928850 E | 23-08-2013 |
| | | PT 2177224 E | 15-05-2013 |
| | | PT 2311464 E | 15-05-2013 |
| | | RS 52780 B | 31-10-2013 |
| | | RS 52781 B | 31-10-2013 |
| | | RS 52852 B | 31-12-2013 |
| | | SI 1928850 T1 | 30-09-2013 |
| | | SI 2177224 T1 | 28-06-2013 |
| | | SI 2311464 T1 | 28-06-2013 |
| | | SV 2008002828 A | 20-07-2010 |
| | | TN SN08078 A1 | 14-07-2009 |
| | | TW 200800930 A | 01-01-2008 |
| | | UA 93388 C2 | 10-02-2011 |
| | | US 2007049572 A1 | 01-03-2007 |
| | | WO 2007024717 A2 | 01-03-2007 |
| | | ZA 200802338 B | 28-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008144483 A
- WO 2020063676 A1, X. Cai, F. He, J. Liang, B. Qu, W. Tao, J. Xu, L. Zhang, Y. Zhang,
- US 7879840 B2, A. R. Marks, D. W. Landry, S. Deng, C. Z. Zhuang, S. E. Lehnart


### Non-patent literature cited in the description


- **A.R. MARKS**. Targeting ryanodine receptors to treat human diseases. *J Clin Invest*, 2023, vol. 133, e162891
- **D. M. BERS**. Cardiac excitation-contraction coupling. *Nature*, 2002, vol. 415, 198-205
- **S. O. MARX** ; **A. R. MARKS**. Dysfunctional ryanodine receptors in the heart: new insights into complex cardiovascular diseases. *J. Mol. Cell. Cardiol.*, 2013, vol. 58, 225-231
- **S. O. MARX** ; **A. R. MARKS**. Dysfunctional ryanodine receptors in the heart: new insights into complex cardiovascular diseases.. *J. Mol. Cell. Cardiol.*, 2013, vol. 58, 225-231
- **D. M. BERS**. Cardiac sarcoplasmic reticulum calcium leak: basis and roles in cardiac dysfunction. *Annu. Rev. Physiol.*, 2014, vol. 76, 107-127
- **D. M. BERS**. Cardiac sarcoplasmic reticulum calcium leak: basis and roles in cardiac dysfunction. *Annu. Rev. Physiol*, 2014, vol. 76, 107-127
- **H. UCHINOUMI** ; **Y. YANG** ; **T. ODA** ; **N. LI** ; **K. M. ALSINA** ; **J. L. PUGLISI** ; **Y. CHEN-IZU** ; **R. L. CORNEA** ; **X. H. T. WEHRENS** ; **D. M. BERS**. CaMKII-dependent phosphorylation of RyR2 promotes targetable pathological RyR2 conformational shift. *J. Mol. Cell. Cardiol.*, 2016, vol. 98, 62-72
- **X. H. T. WEHRENS** ; **S. E. LEHNART** ; **A. R. MARKS**. Ryanodine Receptor-Targeted Anti-Arrhythmic Therapy. *Ann. N. Y. Acad. Sci.*, 2005, vol. 1047, 366-375
- **J. BALDERAS-VILLALOBOS** ; **T. MOLINA-MUÑOZ** ; **P. MAILLOUX-SALINAS** ; **G. BRAVO** ; **K. CARVAJAL** ; **N. L. GÓMEZ-VIQUEZ**. Oxidative stress in cardiomyocytes contributes to decreased SERCA2a activity in rats with metabolic syndrome. *Am. J. Physiol. Heart Circ.*, 2013, vol. 305, H1344-H1353
- **S. E. LEHNART** ; **X. H. T. WEHRENS** ; **S. REIKEN** ; **S. WARRIER** ; **A. E. BELEVYCH** ; **R. D. HARVEY** ; **W. RICHTER** ; **S. L. C. JIN** ; **M. CONTI** ; **A. R. MARKS**. Phosphodiesterase 4D Deficiency in the Ryanodine-Receptor Complex Promotes Heart Failure and Arrhythmias. *Cell*, 2005, vol. 123, 25-35
- **W. J. PARK** ; **J. G. OH**. SERCA2a: a prime target for modulation of cardiac contractility during heart failure. *BMB Rep*, 2013, vol. 46, 237-243
- **M. JESSUP** ; **B. GREENBERG** ; **D. MANCINI** ; **T. CAPPOLA** ; **D. F. PAULY** ; **B. JASKI** ; **A. YAROSHINSKY** ; **K. M. ZSEBO** ; **H. DITTRICH** ; **R. J. HAJJAR**. Calcium Upregulation by Percutaneous Administration of Gene Therapy in Cardiac Disease (CUPID). *Circulation*, 2011, vol. 124, 304-313
- **M. KANEKO** ; **H. YAMAMOTO** ; **H. SAKAI** ; **Y. KAMADA** ; **T. TANAKA** ; **S. FUJIWARA** ; **S. YAMAMOTO** ; **H. TAKAHAGI** ; **H. IGAWA** ; **S. KASAI**. A pyridone derivative activates SERCA2a by attenuating the inhibitory effect of phospholamban. *Eur. J. Pharmacol.*, 2017, vol. 814, 1-8
- **R. L. CORNEA** ; **S. J. GRUBER** ; **E. L. LOCKAMY** ; **J. M. MURETTA** ; **D. JIN** ; **J. CHEN** ; **R. DAHL** ; **T. BARTFAI** ; **K. M. ZSEBO** ; **G. D. GILLISPIE**. High-Throughput FRET Assay Yields Allosteric SERCA Activators. *J. Biomol. Screen*, 2012, vol. 18, 97-107
- **A. LURAGHI** ; **M. FERRANDI** ; **P. BARASSI** ; **M. ARICI** ; **S.-C. HSU** ; **E. TORRE** ; **C. RONCHI** ; **A. ROMERIO** ; **G.-J. CHANG** ; **P. FERRARI**. Highly Selective SERCA2a Activators: Preclinical Development of a Congeneric Group of First-in-Class Drug Leads against Heart Failure. *J. Med. Chem*, 2022, vol. 65, 7324-7333

- **R. NIKOLAIENKO** ; **E. BOVO** ; **S. L. YUEN** ; **L. M. TREINEN** ; **K. BERG** ; **C. C. ALDRICH** ; **D. D. THOMAS** ; **R. L. CORNEA** ; **A. V. ZIMA**. New N-aryl-N-alkyl-thiophene-2-carboxamide compound enhances intracellular Ca2+ dynamics by increasing SERCA2a Ca2+ pumping. *Biophysical Journal*, 2023, vol. 122, 386-396
- **R. UEOKA** ; **M. BORTFELD-MILLER** ; **B. I. MORINAKA** ; **J. A. VORHOLT** ; **J. PIEL**. Toblerols: Cyclopropanol-Containing Polyketide Modulators of Antibiosis in Methylobacteria. *Angew. Chem. Int. Ed.*, 2018, vol. 57, 977-981
- **C. J. SUCKLING**. The Cyclopropyl Group in Studies of Enzyme Mechanism and Inhibition. *Angew. Chem. Int. Ed.*, 1988, vol. 27, 537-552
- **A. ESPOSITO** ; **P. P. PIRAS** ; **D. RAMAZZOTTI** ; **M. TADDEI**. First Stereocontrolled Synthesis of (S)-Cleonin and Related Cyclopropyl-Substituted Amino Acids. *Org. Lett.*, 2001, vol. 3, 3273-3275
- **O. G. KULINKOVICH**. The Chemistry of Cyclopropanols. *Chem. Rev.*, 2003, vol. 103, 2597-2632
- **O. G. KULINKOVICH** ; **D. A. ASTASHKO** ; **V. I. TYVORSKII** ; **N. A. ILYINA**. Synthesis of $\alpha$,$\beta$-Epoxy Ketones from Alkyl- and Arylsubstituted Cyclopropanols. *Synthesis*, 2001, vol. 2001, 1453-1455
- **G. SANTULLI** ; **G. PAGANO** ; **C. SARDU** ; **W. XIE** ; **S. REIKEN** ; **S. L. D'ASCIA** ; **M. CANNONE** ; **N. MARZILIANO** ; **B. TRIMARCO** ; **T. A. GUISE**. Calcium release channel RyR2 regulates insulin release and glucose homeostasis. *J Clin Invest.*, 2015, vol. 125, 1968-1978
- **H. DRIDI** ; **Y. LIU** ; **S. REIKEN** ; **X. LIU** ; **E. K. ARGYROUSI** ; **Q. YUAN** ; **M. C. MIOTTO** ; **L. SITTENFELD** ; **A. MEDDAR** ; **R. K. SONI**. Heart failure-induced cognitive dysfunction is mediated by intracellular Ca2+ leak through ryanodine receptor type 2. *Nat Neurosci.*, 2023, vol. 26, 1365-1378
- **R. T. MARTUSCELLO** ; **M. L. CHEN** ; **S. REIKEN** ; **L. R. SITTENFELD** ; **D. S. RUFF** ; **C. L. NI** ; **C. C. LIN** ; **M. K. PAN** ; **E. D. LOUIS** ; **A. R. MARKS**. Defective cerebellar ryanodine receptor type 1 and endoplasmic reticulum calcium 'leak' in tremor pathophysiology. *Acta Neuropathol.*, 2023, vol. 146, 301-318
- **A. R. MARKS**. Targeting ryanodine receptors to treat human diseases. *J. Clin. Invest*, 2023, vol. 133, e162891
- **A. M. BELLINGER** ; **S. REIKEN** ; **M. DURA** ; **P. W. MURPHY** ; **S. X. DENG** ; **D. W. LANDRY** ; **D. NIEMAN** ; **S. E. LEHNART** ; **M. SAMARU** ; **A. LACAMPAGNE**. Remodeling of ryanodine receptor complex causes "leaky" channels: a molecular mechanism for decreased exercise capacity. *Proc. Natl. Acad. Sci. USA*, 2008, vol. 105, 2198-2202
- **A. R. MARKS**. Targeting ryanodine receptors to treat human diseases. *J. Clin. Invest.*, 2023, vol. 133, e162891
- **S. E. LEHNART** ; **M. MONGILLO** ; **A. BELLINGER** ; **N. LINDEGGER** ; **B. X. CHEN** ; **W. HSUEH** ; **S. REIKEN** ; **A. WRONSKA** ; **L. J. DREW** ; **C. W. WARD**. Leaky Ca2+ release channel/ryanodine receptor 2 causes seizures and sudden cardiac death in mice. *J. Clin. Investig.*, 2008, vol. 118, 2230-2245
- **G. SANTULLI** ; **G. PAGANO** ; **C. SARDU** ; **W. XIE** ; **S. REIKEN** ; **S. L. D'ASCIA** ; **M. CANNONE** ; **N. MARZILIANO** ; **B. TRIMARCO** ; **T. A. GUISE**. Calcium release channel RyR2 regulates insulin release and glucose homeostasis. *J Clin Invest*, 2015, vol. 125, 1968-1978
- **S. E. LEHNART** ; **M. MONGILLO** ; **A. BELLINGER** ; **N. LINDEGGER** ; **B. X. CHEN** ; **W. HSUEH** ; **S. REIKEN** ; **A. WRONSKA** ; **L. J. DREW** ; **C. W. WARD**. Leaky Ca2+ release channel/ryanodine receptor 2 causes seizures and sudden cardiac death in mice. *J. Clin. Investig*, 2008, vol. 118, 2230-2245
- **M. YANO** ; **S. KOBAYASHI** ; **M. KOHNO** ; **M. DOI** ; **T. TOKUHISA** ; **S. OKUDA** ; **M. SUETSUGU** ; **T. HISAOKA** ; **M. OBAYASHI** ; **T. OHKUSA**. FKBP12.6-mediated stabilization of calcium-release channel (ryanodine receptor) as a novel therapeutic strategy against heart failure. *Circulation*, 2003, vol. 107, 477-484
- **M. J. WLEKLINSKI** ; **P. J. KANNANKERIL** ; **B. C. KNOLLMANN**. Molecular and tissue mechanisms of catecholaminergic polymorphic ventricular tachycardia. *J. Physiol.-London*, 2020, vol. 598, 2817-2834
- **M. RUIZ-MEANA** ; **M. MINGUET** ; **D. BOU-TEEN** ; **E. MIRO-CASAS** ; **C. CASTANS** ; **J. CASTELLANO** ; **E. BONZON-KULICHENKO** ; **A. IGUAL** ; **R. RODRIGUEZ-LECOQ** ; **J. VAZQUEZ**. Ryanodine Receptor Glycation Favors Mitochondrial Damage in the Senescent Heart. *Circulation*, 2019, vol. 139, 949-964
- **K. KAMEI** ; **N. MAEDA** ; **R. OGINO** ; **M. KOYAMA** ; **M. NAKAJIMA** ; **T. TASUOKA** ; **T. OHNO** ; **T. INOUE**. New 5-HT1A receptor agonists possessing 1,4-Benzoxazepine scaffold exhibit highly potent anti-ischemic effects. *Bioorg. Med. Chem. Lett.*, 2001, vol. 11, 595-598

- **D. SZALÓKI VARGÁNÉ** ; **L. TÓTH** ; **B. BUGLYÓ** ; **A. KISS-SZIKSZAI** ; **A. MÁNDI** ; **P. MÁTYUS** ; **S. ANTUS** ; **Y. CHEN** ; **D. LI** ; **L. TAO et al.** 1,5]-Hydride Shift-Cyclization versus C(sp2)-H Functionalization in the Knoevenagel-Cyclization Domino Reactions of 1,4- and 1,5-Benzoxazepines. *Molecules*, 2020, vol. 25, 1265
- **A. K. SINGH** ; **V. RAJ** ; **A. RAI** ; **A. K. KESHARI** ; **S. SAHA**. Indole-fused benzooxazepines: a new structural class of anticancer agents. *Future Sci OA*, 2017, vol. 3 (1), FSO168
- **T. A. POPP** ; **C. TALLANT** ; **C. ROGERS** ; **O. FEDOROV** ; **P. E. BRENNAN** ; **S. MÜLLER** ; **S. KNAPP** ; **F. BRACHER**. Development of Selective CBP/P300 Benzoxazepine Bromodomain Inhibitors. *J. Med. Chem.*, 2016, vol. 59, 8889-8912
- **P. A. GORSKI** ; **A. LEE** ; **P. LEE** ; **J. G. OH** ; **P. VANGHELUWE** ; **K. ISHIKAWA** ; **R. HAJJAR** ; **C. KHO**. Identification and Characterization of p300-Mediated Lysine Residues in Cardiac SERCA2a.. *Int. J. Mol. Sci.*, 2023, vol. 24, 3502
- **O. G. KULINKOVICH**. The Chemistry of Cyclopropanols.. *Chem. Rev*, 2003, vol. 103, 2597-2632
- **O. G. KULINKOVICH**. The Chemistry of Cyclopropanols. *Chem. Rev*, 2003, vol. 103, 2597-2632
- **C. D. SMITH** ; **A. WANG** ; **K. VEMBAIYAN** ; **J. ZHANG** ; **C. XIE** ; **Q. ZHOU** ; **G. WU** ; **S. R. CHEN** ; **T. G. BACK**. Novel carvedilol analogues that suppress store-overload-induced Ca2+ release. *J. Med. Chem.*, 2013, vol. 56, 8626-8655
- **Z.-F. SHI** ; **W.-Y. CHAI** ; **P. AN** ; **X.-P. CAO**. Novel Synthesis of Amphiphilic Dendrons by the Double-Stage Convergent Method. *Org. Lett.*, 2009, vol. 11, 4394-4397
- **D. B. DESS** ; **J. C. MARTIN**. Readily accessible 12-I-5 oxidant for the conversion of primary and secondary alcohols to aldehydes and ketones. *J. Org. Chem.*, 1983, vol. 48, 4155-4156
- **G. Z. ELEK** ; **K. KOPPEL** ; **D. M. ZUBRYTSKI** ; **N. KONRAD** ; **I. JÄRVING** ; **M. LOPP** ; **D. G. KANANOVICH**. Divergent Access to Histone Deacetylase Inhibitory Cyclopeptides via a Late-Stage Cyclopropane Ring Cleavage Strategy. Short Synthesis of Chlamydocin. *Org. Lett*, 2019, vol. 21, 8473-8478
- **T. MURAYAMA** ; **N. KUREBAYASHI**. Assays for Modulators of Ryanodine Receptor (RyR)/Ca(2+) Release Channel Activity for Drug Discovery for Skeletal Muscle and Heart Diseases. *Curr. Protoc. Pharmacol.*, 2019, vol. 87, e71
- **T. MURAYAMA** ; **N. KUREBAYASHI** ; **M. ISHIGAMI-YUASA** ; **S. MORI** ; **Y. SUZUKI** ; **R. AKIMA** ; **H. OGAWA** ; **J. SUZUKI** ; **K. KANEMARU** ; **H. OYAMADA**. Efficient High-Throughput Screening by Endoplasmic Reticulum Ca(2+) Measurement to Identify Inhibitors of Ryanodine Receptor Ca(2+)-Release Channels. *Mol. Pharmacol.*, 2018, vol. 94, 722-730
- **A. DES GEORGES** ; **O. B. CLARKE** ; **R. ZALK** ; **Q. YUAN** ; **K. J. CONDON** ; **R. A. GRASSUCCI** ; **W. A. HENDRICKSON** ; **A. R. MARKS** ; **J. FRANK**. Structural Basis for Gating and Activation of RyR1. *Cell*, 2016, vol. 167, 145-157e17
- **G. B. WARREN** ; **P. A. TOON** ; **N. J. BIRDSALL** ; **A. G. LEE** ; **J. C. METCALFE**. Reconstitution of a calcium pump using defined membrane components. *Proc. Natl. Acad. Sci. U.S.A.*, 1974, vol. 71, 622-626
- **A. R. MARKS**. Targeting ryanodine receptors to treat human diseases. *J. Clin. Investig*, 2023, vol. 133, e162891
- **S. O. MARX** ; **A. R. MARKS**. Dysfunctional ryanodine receptors in the heart: new insights into complex cardiovascular diseases. *J. Mol. Cell. Cardiol*, 2013, vol. 58, 225-231
- **X. H. T. WEHRENS** ; **S. E. LEHNART** ; **F. HUANG** ; **J. A. VEST** ; **S. R. REIKEN** ; **P. J. MOHLER** ; **J. SUN** ; **S. GUATIMOSIM** ; **L.-S. SONG** ; **N. ROSEMBLIT**. FKBP12.6 Deficiency and Defective Calcium Release Channel (Ryanodine Receptor) Function Linked to Exercise-Induced Sudden Cardiac Death. *Cell*, 2003, vol. 113, 829-840
- **J. BALDERAS-VILLALOBOS** ; **T. MOLINA-MUÑOZ** ; **P. MAILLOUX-SALINAS** ; **G. BRAVO** ; **K. CARVAJAL** ; **N. L. GÓMEZ-VIQUEZ**. Oxidative stress in cardiomyocytes contributes to decreased SERCA2a activity in rats with metabolic syndrome.. *Am. J. Physiol. Heart Circ*, 2013, vol. 305, H1344-H1353
- **R. L. CORNEA** ; **S. J. GRUBER** ; **E. L. LOCKAMY** ; **J. M. MURETTA** ; **D. JIN** ; **J. CHEN** ; **R. DAHL** ; **T. BARTFAI** ; **K. M. ZSEBO** ; **G. D. GILLISPIE**. High-Throughput FRET Assay Yields Allosteric SERCA Activators.. *J. Biomol. Screen*, 2012, vol. 18, 97-107
- **A. LURAGHI** ; **M. FERRANDI** ; **P. BARASSI** ; **M. ARICI** ; **S.-C. HSU** ; **E. TORRE** ; **C. RONCHI** ; **A. ROMERIO** ; **G.-J. CHANG** ; **P. FERRARI**. Highly Selective SERCA2a Activators: Preclinical Development of a Congeneric Group of First-in-Class Drug Leads against Heart Failure. *J. Med. Chem.*, 2022, vol. 65, 7324-7333
- **R. UEOKA** ; **M. BORTFELD-MILLER** ; **B. I. MORINAKA** ; **J. A. VORHOLT** ; **J. PIEL**. Toblerols: Cyclopropanol-Containing Polyketide Modulators of Antibiosis in Methylobacteria. *Angew. Chem. Int. Ed*, 2018, vol. 57, 977-981

- **O. G. KULINKOVICH** ; **D. A. ASTASHKO** ; **V. I. TYVORSKII** ; **N. A. ILYINA**. Synthesis of α,β-Epoxy Ketones from Alkyl- and Arylsubstituted Cyclopropanols.. *Synthesis*, 2001, vol. 2001, 1453-1455
- **H. DRIDI** ; **Y. LIU** ; **S. REIKEN** ; **X. LIU** ; **E. K. ARGYROUSI** ; **Q. YUAN** ; **M. C. MIOTTO** ; **L. SITTENFELD** ; **A. MEDDAR** ; **R. K. SONI**. Heart failure-induced cognitive dysfunction is mediated by intracellular Ca2+ leak through ryanodine receptor type 2. *Nat Neurosci*, 2023, vol. 26, 1365-1378
- **A. M. BELLINGER** ; **S. REIKEN** ; **M. DURA** ; **P. W. MURPHY** ; **S. X. DENG** ; **D. W. LANDRY** ; **D. NIEMAN** ; **S. E. LEHNART** ; **M. SAMARU** ; **A. LACAMPAGNE**. Remodeling of ryanodine receptor complex causes "leaky" channels: a molecular mechanism for decreased exercise capacity. *Proc. Natl. Acad. Sci. U SA*, 2008, vol. 105, 2198-2202
- **K. KAMEI** ; **N. MAEDA** ; **R. OGINO** ; **M. KOYAMA** ; **M. NAKAJIMA** ; **T. TASUOKA** ; **T. OHNO** ; **T. INOUE**. New 5-HT1A receptor agonists possessing 1,4-Benzoxazepine scaffold exhibit highly potent anti-ischemic effects. *Bioorg. Med. Chem. Lett*, 2001, vol. 11, 595-598
- **A. K. SINGH** ; **V. RAJ** ; **A. RAI** ; **A. K. KESHARI**. S. Saha Indole-fused benzooxazepines: a new structural class of anticancer agents. *Future Sci OA*, 2017, vol. 3 (1), FSO168
- **T. A. POPP** ; **C. TALLANT** ; **C. ROGERS** ; **O. FEDOROV** ; **P. E. BRENNAN** ; **S. MÜLLER** ; **S. KNAPP** ; **F. BRACHER**. Development of Selective CBP/P300 Benzoxazepine Bromodomain Inhibitors. *J. Med. Chem*, 2016, vol. 59, 8889-8912
- **D. B. DESS** ; **J. C. MARTIN**. Readily accessible 12-I-5 oxidant for the conversion of primary and secondary alcohols to aldehydes and ketones. *J. Org. Chem*, 1983, vol. 48, 4155-4156
- **G. Z. ELEK** ; **K. KOPPEL** ; **D. M. ZUBRYTSKI** ; **N. KONRAD** ; **I. JÄRVING** ; **M. LOPP** ; **D. G. KANANOVICH**. Divergent Access to Histone Deacetylase Inhibitory Cyclopeptides via a Late-Stage Cyclopropane Ring Cleavage Strategy. Short Synthesis of Chlamydocin. *Org. Lett.*, 2019, vol. 21, 8473-8478
- **T. MURAYAMA** ; **N. KUREBAYASHI**. Assays for Modulators of Ryanodine Receptor (RyR)/Ca(2+) Release Channel Activity for Drug Discovery for Skeletal Muscle and Heart Diseases.. *Curr. Protoc. Pharmacol.*, 2019, vol. 87, e71
- **A. DES GEORGES** ; **O. B. CLARKE** ; **R. ZALK** ; **Q. YUAN** ; **K. J. CONDON** ; **R. A. GRASSUCCI** ; **W. A. HENDRICKSON** ; **A. R. MARKS** ; **J. FRANK**. Structural Basis for Gating and Activation of RyR1. *Cell*, 2016, vol. 167, 145-157, e17
- **G. B. WARREN** ; **P. A. TOON** ; **N. J. BIRDSALL** ; **A. G. LEE** ; **J. C. METCALFE**. Reconstitution of a calcium pump using defined membrane components. *Proc. Natl. Acad. Sci. U.S.A*, 1974, vol. 71, 622-626